# EUROPEAN PATENT APPLICATION

(11) **EP 4 831 205 A1**
(43) Date of publication of application: **28.10.2026**
(21) Application number: 25736222.8
(22) Date of filing: 03.01.2025
(51) Int. Cl.: C07K 16/28, A61K 39/395, A61P 35/00

(54) **GHR AGONISTIC ANTIBODY AND USE THEREOF**

(30) Priority: 05.01.2024 CN 202410020660
(71) Applicant: Changchun Genescience Pharmaceutical Co., Ltd., Changchun, Jilin 130012 (CN)
(72) Inventor: KANG, Lishan, Changchun, Jilin 130012 (CN); HAO, Qian, Changchun, Jilin 130012 (CN); JIN, Houcong, Changchun, Jilin 130012 (CN); XU, Henan, Changchun, Jilin 130012 (CN); LIU, Ning, Changchun, Jilin 130012 (CN); SHEN, Hongxia, Changchun, Jilin 130012 (CN); GU, Xiaoyu, Changchun, Jilin 130012 (CN); CHE, Chong, Changchun, Jilin 130012 (CN); LIU, Dan, Changchun, Jilin 130012 (CN); LIAO, Xuemei, Changchun, Jilin 130012 (CN); WANG, Tao, Changchun, Jilin 130012 (CN); WANG, Siqin, Changchun, Jilin 130012 (CN); JIN, Lei, Changchun, Jilin 130012 (CN)
(74) Representative: E. Blum & Co. AG
(86) International application number: PCT/CN2025/070359
(87) International publication number: WO 2025/146121

(57) **Abstract**

Provided are an agonistic single-domain antibody specifically binding to a growth hormone receptor or an antigen-binding fragment thereof, and a polypeptide construct containing the single-domain antibody or the antigen-binding fragment thereof. Further provided is a use of the single-domain antibody or the antigen-binding fragment thereof and the polypeptide construct in prevention and/or treatment of diseases.

## Description

The present application is based on and claims priority to CN Application No. 202410020660.X filed on January 05, 2024, the entire disclosure of which is incorporated herein by reference.

### TECHNICAL FIELD

The present invention relates to the field of biomedicine, and more specifically, to an agonistic single-domain antibody specifically binding to a growth hormone receptor or an antigen-binding fragment thereof, and a polypeptide construct containing the single-domain antibody or antigen-binding fragment thereof, and related uses.

### BACKGROUND

Human growth hormone (hGH) is a protein hormone secreted by the acidophilic cells of the anterior pituitary gland. It is composed of 191 amino acids and its main physiological function is to promote metabolism and growth and development. hGH acts on cartilage tissue mediated by somatomedins or insulin-like growth factors, increases bone length, thereby promoting linear growth in the human body. In recent years, more functions of hGH have been discovered, including promoting the growth of skeletal muscle and cardiomyocytes, promoting protein synthesis, regulating immune system function, and enhancing immune defense capabilities. The large-scale clinical application of hGH has expanded from the initial prevention and treatment of pediatric short stature caused by growth hormone deficiency and adult growth hormone deficiency, to fields such as burns, acute pancreatitis, and anti-aging in the elderly, and its clinical indications are still continuously expanding.

Currently marketed and investigational GHR (GH receptor) agonists are all daily or weekly formulations, and all are GH or GH modified products. However, for patients with point mutations in the GHR receptor or those with GH neutralizing antibodies, GH replacement therapy may not be ideal. In addition, due to receptor-mediated clearance, GH and related modified products require higher doses to achieve longer dosing intervals, which can lead to excessively high levels of stimulated IGF-1 and raise safety concerns.

### SUMMARY OF THE INVENTION

Through extensive research, the inventors of the present application have obtained an agonistic single-domain antibody that can specifically bind to GHR and a polypeptide construct containing it. The single-domain antibody or polypeptide construct can mimic the in vitro and in vivo functions of GH, that is, activate the downstream signaling pathway of GHR to achieve effects such as promoting animal weight gain and bone growth.

Therefore, in a first aspect, the present application provides a single-domain antibody specifically binding to a growth hormone receptor (GHR) or an antigen-binding fragment thereof.

In some embodiments, the single-domain antibody or antigen-binding fragment thereof comprises:

CDR1 or a variant thereof, CDR2 or a variant thereof, and CDR3 or a variant thereof contained in a heavy chain variable region (VHH) as set forth in any one of SEQ ID NO: 61, 67-75, 90-92;

wherein the variant has one or several amino acid substitutions, deletions or insertions (e.g., 1, 2 or 3 amino acid substitutions, deletions or insertions) compared with the sequence from which it is derived.

In some embodiments, the substitutions are conservative substitutions.

In some embodiments, the single-domain antibody or antigen-binding fragment thereof comprises the 3 CDRs contained in the heavy chain variable region (VHH) as set forth in any one of SEQ ID NO: 61, 67-75, 90-92.

In some embodiments, the CDRs are defined by the Kabat, IMGT or Chothia numbering system.

In some embodiments, the single-domain antibody or antigen-binding fragment thereof comprises:
(a) (i) CDR1 having a sequence as set forth in SEQ ID NO: 7, or a sequence having one or several amino acid substitutions, deletions or insertions (e.g., 1, 2 or 3 amino acid substitutions, deletions or insertions) compared with the sequence as set forth in SEQ ID NO: 7;
   (ii) CDR2 having a sequence as set forth in any one of SEQ ID NO: 22-24, or a sequence having one or several amino acid substitutions, deletions or insertions (e.g., 1, 2 or 3 amino acid substitutions, deletions or insertions) compared with the sequence as set forth in any one of SEQ ID NO: 22-24; and
   (iii) CDR3 having a sequence as set forth in SEQ ID NO: 48, or a sequence having one or several amino acid substitutions, deletions or insertions (e.g., 1, 2 or 3 amino acid substitutions, deletions or insertions) compared with the sequence as set forth in SEQ ID NO: 48;
   wherein the CDRs are defined by the Kabat numbering system;
(b) (i) CDR1 having a sequence as set forth in SEQ ID NO: 9, or a sequence having one or several amino acid substitutions, deletions or insertions (e.g., 1, 2 or 3 amino acid substitutions, deletions or insertions) compared with the sequence as set forth in SEQ ID NO: 9;
   (ii) CDR2 having a sequence as set forth in SEQ ID NO: 27 or 28, or a sequence having one or several amino acid substitutions, deletions or insertions (e.g., 1, 2 or 3 amino acid substitutions, deletions or insertions) compared with the sequence as set forth in SEQ ID NO: 27 or 28; and
   (iii) CDR3 having a sequence as set forth in SEQ ID NO: 49, or a sequence having one or several amino acid substitutions, deletions or insertions (e.g., 1, 2 or 3 amino acid substitutions, deletions or insertions) compared with the sequence as set forth in SEQ ID NO: 49;
   wherein the CDRs are defined by the IMGT numbering system;
      or,
(c) (i) CDR1 having a sequence as set forth in SEQ ID NO: 8, or a sequence having one or several amino acid substitutions, deletions or insertions (e.g., 1, 2 or 3 amino acid substitutions, deletions or insertions) compared with the sequence as set forth in SEQ ID NO: 8;
   (ii) CDR2 having a sequence as set forth in SEQ ID NO: 25 or 26, or a sequence having one or several amino acid substitutions, deletions or insertions (e.g., 1, 2 or 3 amino acid substitutions, deletions or insertions) compared with the sequence as set forth in SEQ ID NO: 25 or 26; and
   (iii) CDR3 having a sequence as set forth in SEQ ID NO: 48, or a sequence having one or several amino acid substitutions, deletions or insertions (e.g., 1, 2 or 3 amino acid substitutions, deletions or insertions) compared with the sequence as set forth in SEQ ID NO: 48;
   wherein the CDRs are defined by the Chothia numbering system.

In some embodiments, the substitutions are conservative substitutions.

In some embodiments, the single-domain antibody or antigen-binding fragment thereof comprises:
(a) CDR1 as set forth in SEQ ID NO: 7, CDR2 as set forth in any one of SEQ ID NO: 22-24, and CDR3 as set forth in SEQ ID NO: 48; wherein the CDRs are defined by the Kabat numbering system;
(b) CDR1 as set forth in SEQ ID NO: 9, CDR2 as set forth in SEQ ID NO: 27 or 28, and CDR3 as set forth in SEQ ID NO: 49; wherein the CDRs are defined by the IMGT numbering system;
   or,
(c) CDR1 as set forth in SEQ ID NO: 8, CDR2 as set forth in SEQ ID NO: 25 or 26, and CDR3 as set forth in SEQ ID NO: 48; wherein the CDRs are defined by the Chothia numbering system.

In some embodiments, the single-domain antibody or antigen-binding fragment thereof comprises CDR1 as set forth in SEQ ID NO: 7, CDR2 as set forth in SEQ ID NO: 22, and CDR3 as set forth in SEQ ID NO: 48; wherein the CDRs are defined by the Kabat numbering system.

In some embodiments, the single-domain antibody or antigen-binding fragment thereof comprises CDR1 as set forth in SEQ ID NO: 7, CDR2 as set forth in SEQ ID NO: 23, and CDR3 as set forth in SEQ ID NO: 48; wherein the CDRs are defined by the Kabat numbering system.

In some embodiments, the single-domain antibody or antigen-binding fragment thereof comprises CDR1 as set forth in SEQ ID NO: 7, CDR2 as set forth in SEQ ID NO: 24, and CDR3 as set forth in SEQ ID NO: 48; wherein the CDRs are defined by the Kabat numbering system.

In some embodiments, the single-domain antibody or antigen-binding fragment thereof comprises CDR1 as set forth in SEQ ID NO: 9, CDR2 as set forth in SEQ ID NO: 27, and CDR3 as set forth in SEQ ID NO: 49; wherein the CDRs are defined by the IMGT numbering system.

In some embodiments, the single-domain antibody or antigen-binding fragment thereof comprises CDR1 as set forth in SEQ ID NO: 9, CDR2 as set forth in SEQ ID NO: 28, and CDR3 as set forth in SEQ ID NO: 49; wherein the CDRs are defined by the IMGT numbering system.

In some embodiments, the single-domain antibody or antigen-binding fragment thereof comprises CDR1 as set forth in SEQ ID NO: 8, CDR2 as set forth in SEQ ID NO: 25, and CDR3 as set forth in SEQ ID NO: 48; wherein the CDRs are defined by the Chothia numbering system.

In some embodiments, the single-domain antibody or antigen-binding fragment thereof comprises CDR1 as set forth in SEQ ID NO: 8, CDR2 as set forth in SEQ ID NO: 26, and CDR3 as set forth in SEQ ID NO: 48; wherein the CDRs are defined by the Chothia numbering system.

In some embodiments, the single-domain antibody or antigen-binding fragment thereof comprises an amino acid sequence selected from the group consisting of:
(i) a sequence as set forth in SEQ ID NO: 61;
(ii) a sequence having one or several amino acid substitutions, deletions or insertions (e.g., 1, 2, 3, 4 or 5 amino acid substitutions, deletions or insertions) compared with the sequence as set forth in SEQ ID NO: 61; or
(iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as set forth in SEQ ID NO: 61.

In some embodiments, the substitutions are conservative substitutions.

In some embodiments, the single-domain antibody or antigen-binding fragment thereof is humanized.

In some embodiments, the single-domain antibody or antigen-binding fragment thereof comprises the heavy chain framework regions of a human immunoglobulin.

In some embodiments, the single-domain antibody or antigen-binding fragment thereof comprises the heavy chain framework regions in an amino acid sequence encoded by a human heavy chain germline antibody gene.

In some embodiments, the single-domain antibody or antigen-binding fragment thereof comprises the heavy chain framework regions in an amino acid sequence encoded by a human IGHV gene, wherein the human IGHV gene is selected from IGHV3-23, IGHV3-30, IGHV3-34, IGHV3-7 and IGHV3-74. In some embodiments, the heavy chain framework regions are defined by the IMGT, Kabat or Chothia numbering system. In some embodiments, the heavy chain framework regions are defined by the IMGT numbering system.

In some embodiments, the heavy chain framework regions optionally comprise one or more (e.g., 1, 2, 3, 4, 5, 6, 7, 8, 9 or 10) back mutations from human residues to camelid residues.

In some embodiments, the single-domain antibody or antigen-binding fragment thereof comprises:
(a) FR1 as set forth in SEQ ID NO: 2 or 6, FR2 as set forth in any one of SEQ ID NO: 10-13, FR3 as set forth in any one of SEQ ID NO: 30-33, and/or FR4 as set forth in SEQ ID NO: 51; wherein the FRs are defined by the Kabat numbering system;
(b) FR1 as set forth in SEQ ID NO: 4 or 5, FR2 as set forth in any one of SEQ ID NO: 18-21, FR3 as set forth in any one of SEQ ID NO: 42-47, and/or FR4 as set forth in SEQ ID NO: 51; wherein the FRs are defined by the IMGT numbering system;
   or,
(c) FR1 as set forth in SEQ ID NO: 4 or 5, FR2 as set forth in any one of SEQ ID NO: 14-17, FR3 as set forth in any one of SEQ ID NO: 35-40, and/or FR4 as set forth in SEQ ID NO: 51; wherein the FRs are defined by the Chothia numbering system.

In some embodiments, the single-domain antibody or antigen-binding fragment thereof comprises:
(i) a sequence as set forth in any one of SEQ ID NO: 67-75 and 90-92;
(ii) a sequence having one or several amino acid substitutions, deletions or insertions (e.g., 1, 2, 3, 4 or 5 amino acid substitutions, deletions or insertions) compared with the sequence as set forth in any one of SEQ ID NO: 67-75 and 90-92; or
(iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as set forth in any one of SEQ ID NO: 67-75 and 90-92.

In some embodiments, the substitutions are conservative substitutions.

In some embodiments, the single-domain antibody is a GHR agonistic single-domain antibody.

In some embodiments, the single-domain antibody or antigen-binding fragment thereof possesses cross-reactivity with human GHR and murine (e.g., rat) GHR.

In some embodiments, the GHR is human GHR or murine (e.g., rat) GHR.

In a second aspect, the present application provides a polypeptide construct, comprising the single-domain antibody or antigen-binding fragment thereof of the first aspect, an Fc peptide derived from an immunoglobulin, and an optional hinge peptide.

In some embodiments, the single-domain antibody or antigen-binding fragment thereof is covalently linked to the Fc peptide and the optional hinge peptide (e.g., forming a covalent linkage via covalent bonds including peptide bonds, isopeptide bonds and/or disulfide bonds).

In some embodiments, the polypeptide construct comprises the single-domain antibody or antigen-binding fragment thereof of the first aspect, the Fc peptide and the hinge peptide, and the single-domain antibody or antigen-binding fragment thereof is covalently linked to the Fc peptide and the hinge peptide through covalent bonds including peptide bonds.

In some embodiments, the polypeptide construct comprises a first peptide chain, wherein the first peptide chain comprises: a first single-domain antibody or antigen-binding fragment thereof, a first hinge peptide, and a first Fc peptide; wherein the first single-domain antibody or antigen-binding fragment thereof is selected from the single-domain antibodies or antigen-binding fragments thereof of the first aspect. In some embodiments, the first peptide chain comprises, sequentially from the N-terminus to the C-terminus: the first single-domain antibody or antigen-binding fragment thereof, the first hinge peptide, and the first Fc peptide.

In some embodiments, the polypeptide construct further comprises a second peptide chain, the second peptide chain comprising a second hinge peptide and a second Fc peptide. In some embodiments, the second peptide chain further comprises a second single-domain antibody or antigen-binding fragment thereof. In some embodiments, the second single-domain antibody is selected from single-domain antibodies specifically binding to GHR. In some embodiments, the second single-domain antibody or antigen-binding fragment thereof is selected from the single-domain antibodies or antigen-binding fragments thereof of the first aspect.

In some embodiments, the second peptide chain comprises, sequentially from the N-terminus to the C-terminus: the second single-domain antibody or antigen-binding fragment thereof, the second hinge peptide, and the second Fc peptide.

In some embodiments, the polypeptide construct is a heavy chain antibody.

In some embodiments, no additional amino acid residues are present between the first single-domain antibody or antigen-binding fragment thereof and the first hinge peptide, and/or no additional amino acid residues are present between the second single-domain antibody or antigen-binding fragment thereof and the second hinge peptide.

In some embodiments, the first hinge peptide and the first Fc peptide are optionally linked with or without a peptide linker, and/or the second hinge peptide and the second Fc peptide are optionally linked with or without a peptide linker.

In some embodiments, no additional amino acid residues are present between the first hinge peptide and the first Fc peptide, and/or no additional amino acid residues are present between the second hinge peptide and the second Fc peptide.

In some embodiments, the polypeptide construct has one or more features selected from the group consisting of:
(i) the first single-domain antibody or antigen-binding fragment thereof is the same as or different from the second single-domain antibody or antigen-binding fragment thereof;
(ii) the first hinge peptide and the second hinge peptide are derived from the same or different immunoglobulins;
(iii) the first Fc peptide and the second Fc peptide are derived from the same or different immunoglobulins;
(iv) the first hinge peptide and the second hinge peptide are the same or different;
(v) the first Fc peptide and the second Fc peptide are the same or different;
(vi) the first peptide chain and the second peptide chain are the same or different;
(vii) the first peptide chain and the second peptide chain are linked via disulfide bonds.

In some embodiments, the hinge peptide (e.g., the first hinge peptide and/or the second hinge peptide) is a hinge peptide derived from an immunoglobulin (e.g., IgG1, IgG2, IgG3 or IgG4).

In some embodiments, the hinge peptide (e.g., the first hinge peptide and/or the second hinge peptide) is a hinge peptide derived from a human immunoglobulin (e.g., IgG1, IgG2, IgG3 or IgG4).

Those skilled in the art will readily understand that the hinge peptides derived from immunoglobulins (e.g., human immunoglobulins) include both hinge peptides directly derived from immunoglobulins (e.g., human immunoglobulins) and hinge peptides obtained by modifying or engineering hinge peptides derived from immunoglobulins (e.g., human immunoglobulins) (e.g., artificially designed hinge peptides).

In some embodiments, the hinge peptide (e.g., the first hinge peptide and/or the second hinge peptide) is selected from the hinge peptides of wild-type human immunoglobulins IgG1, IgG2 and IgG4, and artificially designed hinge peptides.

In some embodiments, the artificially designed hinge peptide has enhanced FcRn binding activity compared with the hinge peptides of wild-type human immunoglobulins IgG1, IgG2 and IgG4 (e.g., the hinge peptide of wild-type human immunoglobulin IgG4).

In some embodiments, the artificially designed hinge peptide has a structure represented by Formula I from the N-terminus to the C-terminus:

Z-CPPCP (Formula I)

wherein Z is absent or is a short peptide consisting of 1, 2, 3, 4, 5, 6, 7, 8, 9 or 10 amino acid residues.

In some embodiments, Z is linked to the short peptide CPPCP via a peptide bond.

In some embodiments, Z does not comprise a cysteine residue.

In some embodiments, the hinge peptide (e.g., the first hinge peptide and/or the second hinge peptide) is selected from the hinge peptide of wild-type human immunoglobulin IgG1 and artificially designed hinge peptides.

In some embodiments, the artificially designed hinge peptide has a structure represented by Formula II:

X₁X₂X₃X₄X₅X₆X₇X₈X₉X₁₀CPPCP (Formula II);

wherein X₁ is absent or selected from (i) amino acid residue E and (ii) an amino acid residue (such as D) that is a conservative substitution relative to (i);
X₂ is absent or selected from (i) amino acid residue P and (ii) an amino acid residue (such as A, V, L, I, M) that is a conservative substitution relative to (i);
X₃ is absent or selected from (i) amino acid residue K and (ii) an amino acid residue (such as R, H) that is a conservative substitution relative to (i);
X₄ is absent or selected from (i) amino acid residue S and (ii) an amino acid residue (such as N, Q, G, T, C, Y, W) that is a conservative substitution relative to (i);
X₅ is absent or selected from (i) amino acid residue S and (ii) an amino acid residue (such as N, Q, G, T, C, Y, W) that is a conservative substitution relative to (i);
X₆ is absent or selected from (i) amino acid residue D and (ii) an amino acid residue (such as E) that is a conservative substitution relative to (i);
X₇ is absent or selected from (i) amino acid residue K and (ii) an amino acid residue (such as R, H) that is a conservative substitution relative to (i);
X₈ is absent or selected from (i) amino acid residue T and (ii) an amino acid residue (such as N, Q, G, S, C, Y, W) that is a conservative substitution relative to (i);
X₉ is absent or selected from (i) amino acid residue H and (ii) an amino acid residue (such as R, K) that is a conservative substitution relative to (i);
X₁₀ is absent or selected from (i) amino acid residue T and (ii) an amino acid residue (such as N, Q, G, S, C, Y, W) that is a conservative substitution relative to (i).

In some embodiments, the artificially designed hinge peptide has one or more features selected from the group consisting of: (1) X₁ is absent or is the amino acid residue E, (2) X₂ is absent or is the amino acid residue P, (3) X₃ is absent or is the amino acid residue K, (4) X₄ is absent or is the amino acid residue S, (5) X₅ is absent or is the amino acid residue S, (6) X₆ is absent or is the amino acid residue D, (7) X₇ is absent or is the amino acid residue K, (8) X₈ is absent or is the amino acid residue T, (9) X₉ is absent or is the amino acid residue H, (10) X₁₀ is absent or is the amino acid residue T.

In some embodiments, X₁ is absent or is the amino acid residue E, X₂ is absent or is the amino acid residue P, X₃ is absent or is the amino acid residue K, X₄ is absent or is the amino acid residue S, X₅ is absent or is the amino acid residue S, X₆ is absent or is the amino acid residue D, X₇ is absent or is the amino acid residue K, X₈ is absent or is the amino acid residue T, X₉ is absent or is the amino acid residue H, and X₁₀ is absent or is the amino acid residue T.

In some embodiments, any 1, 2, 3, 4, 5, 6, 7, 8, 9, or 10 amino acid residues of X₁ to X₁₀ are absent.

In some embodiments, X₁ is absent, X₁ to X₂ are absent, X₁ to X₃ are absent, X₁ to X₄ are absent, X₁ to X₅ are absent, X₁ to X₆ are absent, X₁ to X₇ are absent, X₁ to X₈ are absent, X₁ to X₉ are absent, or X₁ to X₁₀ are absent.

In some embodiments, X₁ to X₅ are absent, X₁ to X₆ are absent, X₁ to X₇ are absent, X₁ to X₈ are absent, X₁ to X₉ are absent, or X₁ to X₁₀ are absent. In some embodiments, X₁ to X₇ are absent, or X₁ to X₈ are absent.

In some embodiments, the hinge peptide (e.g., the first hinge peptide and/or the second hinge peptide) is selected from the hinge peptide of wild-type human immunoglobulin IgG2 and artificially designed hinge peptides.

In some embodiments, the artificially designed hinge peptide has a structure represented by Formula III:

XᵢXᵢᵢXᵢᵢᵢXᵢᵥXᵥXᵥᵢXᵥᵢᵢCPPCP (Formula III);

wherein Xᵢ is absent or selected from (i) amino acid residue E and (ii) an amino acid residue (such as D) that is a conservative substitution relative to (i);
Xᵢᵢ is absent or selected from (i) amino acid residue R and (ii) an amino acid residue (such as K or H) that is a conservative substitution relative to (i);
Xᵢᵢᵢ is absent or selected from (i) amino acid residue K and (ii) an amino acid residue (such as R or H) that is a conservative substitution relative to (i);
Xᵢᵥ is absent or selected from non-cysteine residues; preferably, Xᵢᵥ is absent or selected from (i) amino acid residue S and (ii) an amino acid residue (e.g., N, Q, G, T, C, Y, W) that is a conservative substitution relative to (i);
Xᵥ is absent or selected from non-cysteine residues; preferably, Xᵥ is absent or selected from (i) amino acid residue S and (ii) an amino acid residue (e.g., N, Q, G, T, C, Y, W) that is a conservative substitution relative to (i);
Xᵥᵢ is absent or selected from (i) amino acid residue V and (ii) an amino acid residue (e.g., A, P, L, I, M) that is a conservative substitution relative to (i);
Xᵥᵢᵢ is absent or selected from (i) amino acid residue E and (ii) an amino acid residue (e.g., D) that is a conservative substitution relative to (i);

In some embodiments, the artificially designed hinge peptide has one or more features selected from the group consisting of: (1) Xᵢ is absent or is the amino acid residue E, (2) Xᵢᵢ is absent or is the amino acid residue R, (3) Xᵢᵢᵢ is absent or is the amino acid residue K, (4) Xᵢᵥ is absent or is the amino acid residue S, (5) Xᵥ is absent or is the amino acid residue S, (6) Xᵥᵢ is absent or is the amino acid residue V, (7) Xᵥᵢᵢ is absent or is the amino acid residue E.

In some embodiments, Xᵢ is absent or is the amino acid residue E, Xᵢᵢ is absent or is the amino acid residue R, Xᵢᵢᵢ is absent or is the amino acid residue K, Xᵢᵥ is absent or is the amino acid residue S, Xᵥ is absent or is the amino acid residue S, Xᵥᵢ is absent or is the amino acid residue V, and Xᵥᵢᵢ is absent or is the amino acid residue E.

In some embodiments, any 1, 2, 3, 4, 5, 6, or 7 amino acid residues of Xᵢ to Xᵥᵢᵢ are absent. In some embodiments, Xᵢᵥ to Xᵥᵢᵢ are absent.

In some embodiments, the hinge peptide (e.g., the first hinge peptide and/or the second hinge peptide) has an amino acid sequence as set forth in any one of SEQ ID NO: 62-64 and 80-89.

In some embodiments, the Fc peptide (e.g., the first Fc peptide and/or the second Fc peptide) is an Fc peptide derived from an immunoglobulin (e.g., IgG1, IgG2, IgG3 or IgG4).

In some embodiments, the Fc peptide (e.g., the first Fc peptide and/or the second Fc peptide) is an Fc peptide derived from a human immunoglobulin (e.g., IgG1, IgG2, IgG3 or IgG4).

It is readily understood by those skilled in the art that the Fc peptide derived from an immunoglobulin (e.g., human immunoglobulin) includes both Fc peptides directly derived from an immunoglobulin (e.g., human immunoglobulin) and Fc peptides obtained by modifying or engineering Fc peptides derived from an immunoglobulin (e.g., human immunoglobulin) (e.g., Fc peptide variants).

In some embodiments, the Fc peptide (e.g., the first Fc peptide and/or the second Fc peptide) is an Fc peptide derived from human immunoglobulin IgG1 or IgG4.

In some embodiments, the Fc peptide (e.g., the first Fc peptide and/or the second Fc peptide) is an Fc peptide derived from human immunoglobulin IgG1.

In some embodiments, the Fc peptide (e.g., the first Fc peptide and/or the second Fc peptide) is selected from the Fc peptide of wild-type human immunoglobulin IgG1 and variants thereof; wherein compared with the Fc peptide of wild-type human immunoglobulin IgG1, the Fc peptide variant has reduced Fc effector functions (e.g., reduced ADCC, CDC and/or ADCP activities) and/or an extended half-life.

In some embodiments, compared with the Fc peptide of wild-type human immunoglobulin IgG1, the Fc peptide variant has reduced Fc³R binding activity, reduced serum complement molecule (C1q) binding activity, and/or enhanced FcRn binding activity.

In some embodiments, compared with the Fc peptide of wild-type human immunoglobulin IgG1, the Fc peptide variant comprises mutations selected from L234A, L235A, M252Y, S254T, T256E, and any combination thereof (e.g., selected from mutations: (i) L234A and L235A, (ii) M252Y, S254T and T256E, and (iii): a combination of (i) and (ii)).

In some embodiments, compared with the Fc peptide of wild-type human immunoglobulin IgG1, the Fc peptide variant comprises mutations L234A and L235A. In some embodiments, the Fc peptide variant further comprises mutations M252Y, S254T and T256E.

In some embodiments, the mutation site of the mutation is defined by the EU numbering system.

In some embodiments, the Fc peptide (e.g., the first Fc peptide and/or the second Fc peptide) comprises a sequence as set forth in any one of SEQ ID NO: 52, 65, 76, 93.

In some embodiments, the Fc peptide (e.g., the first Fc peptide and/or the second Fc peptide) is an Fc peptide derived from human immunoglobulin IgG4.

In some embodiments, the Fc peptide (e.g., the first Fc peptide and/or the second Fc peptide) is selected from the Fc peptide of wild-type human immunoglobulin IgG4 and variants thereof; wherein compared with the Fc peptide of wild-type human immunoglobulin IgG4, the Fc peptide variant has reduced Fc effector functions (e.g., reduced ADCC, CDC and/or ADCP activities) and/or an extended half-life.

In some embodiments, compared with the Fc peptide of wild-type human immunoglobulin IgG4, the Fc peptide variant has reduced Fc³R binding activity, reduced serum complement molecule (C1q) binding activity, and/or enhanced FcRn binding activity.

In some embodiments, compared with the Fc peptide of wild-type human immunoglobulin IgG4, the Fc peptide variant comprises mutation L235E.

In some embodiments, the mutation site of the mutation is defined by the EU numbering system.

In some embodiments, the Fc peptide (e.g., the first Fc peptide and/or the second Fc peptide) comprises a sequence as set forth in SEQ ID NO: 79 or 99.

In some embodiments, the polypeptide construct comprises an amino acid sequence as set forth in any one of SEQ ID NO: 94-97 and 103-126.

In some embodiments, the polypeptide construct comprises the first peptide chain and optionally the second peptide chain; wherein the first peptide chain comprises a sequence as set forth in any one of SEQ ID NO: 94-97 and 103-126, and/or the second peptide chain comprises a sequence as set forth in any one of SEQ ID NO: 94-97 and 103-126.

In some embodiments, the polypeptide construct comprises the first peptide chain and optionally the second peptide chain; wherein:
(1) the first peptide chain and the second peptide chain each independently comprise a sequence as set forth in SEQ ID NO: 94;
(2) the first peptide chain and the second peptide chain each independently comprise a sequence as set forth in SEQ ID NO: 95;
(3) the first peptide chain and the second peptide chain each independently comprise a sequence as set forth in SEQ ID NO: 96;
(4) the first peptide chain and the second peptide chain each independently comprise a sequence as set forth in SEQ ID NO: 97;
(5) the first peptide chain and the second peptide chain each independently comprise a sequence as set forth in SEQ ID NO: 103;
(6) the first peptide chain and the second peptide chain each independently comprise a sequence as set forth in SEQ ID NO: 104;
(7) the first peptide chain and the second peptide chain each independently comprise a sequence as set forth in SEQ ID NO: 105;
(8) the first peptide chain and the second peptide chain each independently comprise a sequence as set forth in SEQ ID NO: 106;
(9) the first peptide chain and the second peptide chain each independently comprise a sequence as set forth in SEQ ID NO: 107;
(10) the first peptide chain and the second peptide chain each independently comprise a sequence as set forth in SEQ ID NO: 108;
(11) the first peptide chain and the second peptide chain each independently comprise a sequence as set forth in SEQ ID NO: 109;
(12) the first peptide chain and the second peptide chain each independently comprise a sequence as set forth in SEQ ID NO: 110;
(13) the first peptide chain and the second peptide chain each independently comprise a sequence as set forth in SEQ ID NO: 111;
(14) the first peptide chain and the second peptide chain each independently comprise a sequence as set forth in SEQ ID NO: 112;
(15) the first peptide chain and the second peptide chain each independently comprise a sequence as set forth in SEQ ID NO: 113;
(16) the first peptide chain and the second peptide chain each independently comprise a sequence as set forth in SEQ ID NO: 114;
(17) the first peptide chain and the second peptide chain each independently comprise a sequence as set forth in SEQ ID NO: 115;
(18) the first peptide chain and the second peptide chain each independently comprise a sequence as set forth in SEQ ID NO: 116;
(19) the first peptide chain and the second peptide chain each independently comprise a sequence as set forth in SEQ ID NO: 117;
(20) the first peptide chain and the second peptide chain each independently comprise a sequence as set forth in SEQ ID NO: 118;
(21) the first peptide chain and the second peptide chain each independently comprise a sequence as set forth in SEQ ID NO: 119;
(22) the first peptide chain and the second peptide chain each independently comprise a sequence as set forth in SEQ ID NO: 120;
(23) the first peptide chain and the second peptide chain each independently comprise a sequence as set forth in SEQ ID NO: 121;
(24) the first peptide chain and the second peptide chain each independently comprise a sequence as set forth in SEQ ID NO: 122;
(25) the first peptide chain and the second peptide chain each independently comprise a sequence as set forth in SEQ ID NO: 123;
(26) the first peptide chain and the second peptide chain each independently comprise a sequence as set forth in SEQ ID NO: 124;
(27) the first peptide chain and the second peptide chain each independently comprise a sequence as set forth in SEQ ID NO: 125;
(28) the first peptide chain and the second peptide chain each independently comprise a sequence as set forth in SEQ ID NO: 126.

In some embodiments, the polypeptide construct is a GHR agonistic construct (e.g., the peptide construct can serve as a GHR agonist).

In some embodiments, the polypeptide construct possesses cross-binding activity to human GHR and murine (e.g., rat) GHR.

In some embodiments, the GHR is human GHR or murine (e.g., rat) GHR.

In a third aspect, the present application provides a polypeptide construct, comprising a single-domain antibody or an antigen-binding fragment thereof, a hinge peptide, and an Fc peptide derived from an immunoglobulin;

wherein the hinge peptide has a structure represented by Formula I from the N-terminus to the C-terminus:

Z-CPPCP (Formula I)

wherein Z is absent, or is a short peptide consisting of 1, 2, 3, 4, 5, 6, 7, 8, 9 or 10 non-cysteine amino acid residues.

In some embodiments, Z is linked to the short peptide CPPCP via a peptide bond.

In some embodiments, the single-domain antibody or antigen-binding fragment thereof is covalently linked to the Fc peptide and the hinge peptide (e.g., forming a covalent linkage via covalent bonds including peptide bonds, isopeptide bonds and/or disulfide bonds).

In some embodiments, the single-domain antibody or antigen-binding fragment thereof is covalently linked to the Fc peptide and the hinge peptide via covalent bonds including peptide bonds.

In some embodiments, the polypeptide construct comprises a first peptide chain, the first peptide chain comprising: a first single-domain antibody or an antigen-binding fragment thereof, a first hinge peptide, and a first Fc peptide. In some embodiments, the first hinge peptide has the same meaning as the hinge peptide described above in this aspect (the third aspect). In some embodiments, the first peptide chain comprises, sequentially from the N-terminus to the C-terminus: the first single-domain antibody or antigen-binding fragment thereof, the first hinge peptide, and the first Fc peptide.

In some embodiments, the polypeptide construct further comprises a second peptide chain, the second peptide chain comprising a second hinge peptide and a second Fc peptide. In some embodiments, the second peptide chain further comprises a second single-domain antibody or antigen-binding fragment thereof.

In some embodiments, the second peptide chain comprises, sequentially from the N-terminus to the C-terminus: the second single-domain antibody or antigen-binding fragment thereof, the second hinge peptide, and the second Fc peptide.

In some embodiments, the polypeptide construct is a heavy chain antibody.

In some embodiments, no additional amino acid residues are present between the first single-domain antibody or antigen-binding fragment thereof and the first hinge peptide, and/or no additional amino acid residues are present between the second single-domain antibody or antigen-binding fragment thereof and the second hinge peptide.

In some embodiments, the first hinge peptide and the first Fc peptide are optionally linked with or without a peptide linker, and/or the second hinge peptide and the second Fc peptide are optionally linked with or without a peptide linker.

In some embodiments, no additional amino acid residues are present between the first hinge peptide and the first Fc peptide, and/or no additional amino acid residues are present between the second hinge peptide and the second Fc peptide.

In some embodiments, the polypeptide construct has one or more features selected from the group consisting of:
(i) the first single-domain antibody or antigen-binding fragment thereof is the same as or different from the second single-domain antibody or antigen-binding fragment thereof;
(ii) the first hinge peptide and the second hinge peptide are derived from the same or different immunoglobulins;
(iii) the first Fc peptide and the second Fc peptide are derived from the same or different immunoglobulins;
(iv)the first hinge peptide and the second hinge peptide are the same or different;
(v)the first Fc peptide and the second Fc peptide are the same or different;
(vi)the first peptide chain and the second peptide chain are the same or different;
(vii)the first peptide chain and the second peptide chain are linked via disulfide bonds.

In some embodiments, the first single-domain antibody is selected from single-domain antibodies specifically binding to a growth hormone receptor (GHR).

In some embodiments, the first single-domain antibody is selected from GHR agonistic antibodies.

In some embodiments, the first single-domain antibody or antigen-binding fragment thereof is selected from the single-domain antibodies or antigen-binding fragments thereof of the first aspect.

In some embodiments, the second single-domain antibody is selected from single-domain antibodies specifically binding to a growth hormone receptor (GHR).

In some embodiments, the second single-domain antibody is selected from GHR agonistic antibodies.

In some embodiments, the second single-domain antibody or antigen-binding fragment thereof is selected from the single-domain antibodies or antigen-binding fragments thereof of the first aspect.

In some embodiments, the hinge peptide (e.g., the first hinge peptide and/or the second hinge peptide) has a structure as shown in Formula II:

X₁X₂X₃X₄X₅X₆X₇X₈X₉X₁₀CPPCP (Formula II);

wherein X₁ is absent or selected from (i) amino acid residue E and (ii) an amino acid residue (such as D) that is a conservative substitution relative to (i);
X₂ is absent or selected from (i) amino acid residue P and (ii) an amino acid residue (such as A, V, L, I, M) that is a conservative substitution relative to (i);
X₃ is absent or selected from (i) amino acid residue K and (ii) an amino acid residue (such as R, H) that is a conservative substitution relative to (i);
X₄ is absent or selected from (i) amino acid residue S and (ii) an amino acid residue (such as N, Q, G, T, C, Y, W) that is a conservative substitution relative to (i);
X₅ is absent or selected from (i) amino acid residue S and (ii) an amino acid residue (such as N, Q, G, T, C, Y, W) that is a conservative substitution relative to (i);
X₆ is absent or selected from (i) amino acid residue D and (ii) an amino acid residue (such as E) that is a conservative substitution relative to (i);
X₇ is absent or selected from (i) amino acid residue K and (ii) an amino acid residue (such as R, H) that is a conservative substitution relative to (i);
X₈ is absent or selected from (i) amino acid residue T and (ii) an amino acid residue (such as N, Q, G, S, C, Y, W) that is a conservative substitution relative to (i);
X₉ is absent or selected from (i) amino acid residue H and (ii) an amino acid residue (such as R, K) that is a conservative substitution relative to (i);
X₁₀ is absent or selected from (i) amino acid residue T and (ii) an amino acid residue (such as N, Q, G, S, C, Y, W) that is a conservative substitution relative to (i).

In some embodiments, the hinge peptide has one or more features selected from the group consisting of: (1) X₁ is absent or is the amino acid residue E, (2) X₂ is absent or is the amino acid residue P, (3) X₃ is absent or is the amino acid residue K, (4) X₄ is absent or is the amino acid residue S, (5) X₅ is absent or is the amino acid residue S, (6) X₆ is absent or is the amino acid residue D, (7) X₇ is absent or is the amino acid residue K, (8) X₈ is absent or is the amino acid residue T, (9) X₉ is absent or is the amino acid residue H, (10) X₁₀ is absent or is the amino acid residue T.

In some embodiments, X₁ is absent or is the amino acid residue E, X₂ is absent or is the amino acid residue P, X₃ is absent or is the amino acid residue K, X₄ is absent or is the amino acid residue S, X₅ is absent or is the amino acid residue S, X₆ is absent or is the amino acid residue D, X₇ is absent or is the amino acid residue K, X₈ is absent or is the amino acid residue T, X₉ is absent or is the amino acid residue H, and X₁₀ is absent or is the amino acid residue T.

In some embodiments, any 1, 2, 3, 4, 5, 6, 7, 8, 9, or 10 amino acid residues of X₁ to X₁₀ are absent.

In some embodiments, X₁ is absent, X₁ to X₂ are absent, X₁ to X₃ are absent, X₁ to X₄ are absent, X₁ to X₅ are absent, X₁ to X₆ are absent, X₁ to X₇ are absent, X₁ to X₈ are absent, X₁ to X₉ are absent, or X₁ to X₁₀ are absent.

In some embodiments, X₁ to X₅ are absent, X₁ to X₆ are absent, X₁ to X₇ are absent, X₁ to X₈ are absent, X₁ to X₉ are absent, or X₁ to X₁₀ are absent. In some embodiments, X₁ to X₇ are absent, or X₁ to X₈ are absent.

In some embodiments, none of X₁ to X₁₀ comprises a cysteine residue.

In some embodiments, the hinge peptide (e.g., the first hinge peptide and/or the second hinge peptide) has a structure as shown in Formula III:

XᵢXᵢᵢXᵢᵢᵢXᵢᵥXᵥXᵥᵢXᵥᵢᵢCPPCP (Formula III);

wherein Xᵢ is absent or selected from (i) amino acid residue E and (ii) an amino acid residue (such as D) that is a conservative substitution relative to (i);
Xᵢᵢ is absent or selected from (i) amino acid residue R and (ii) an amino acid residue (such as K or H) that is a conservative substitution relative to (i);
Xᵢᵢᵢ is absent or selected from (i) amino acid residue K and (ii) an amino acid residue (such as R or H) that is a conservative substitution relative to (i);
Xᵢᵥ is absent or selected from non-cysteine residues; preferably, Xᵢᵥ is absent or selected from (i) amino acid residue S and (ii) an amino acid residue (e.g., N, Q, G, T, C, Y, W) that is a conservative substitution relative to (i);
Xᵥ is absent or selected from non-cysteine residues; preferably, Xᵥ is absent or selected from (i) amino acid residue S and (ii) an amino acid residue (e.g., N, Q, G, T, C, Y, W) that is a conservative substitution relative to (i);
Xᵥᵢ is absent or selected from (i) amino acid residue V and (ii) an amino acid residue (e.g., A, P, L, I, M) that is a conservative substitution relative to (i);
Xᵥᵢᵢ is absent or selected from (i) amino acid residue E and (ii) an amino acid residue (e.g., D) that is a conservative substitution relative to (i);

In some embodiments, the hinge peptide has one or more features selected from the group consisting of: (1) Xᵢ is absent or is the amino acid residue E, (2) Xᵢᵢ is absent or is the amino acid residue R, (3) Xᵢᵢᵢ is absent or is the amino acid residue K, (4) Xᵢᵥ is absent or is the amino acid residue S, (5) Xᵥ is absent or is the amino acid residue S, (6) Xᵥᵢ is absent or is the amino acid residue V, (7) Xᵥᵢᵢ is absent or is the amino acid residue E.

In some embodiments, Xᵢ is absent or is the amino acid residue E, Xᵢᵢ is absent or is the amino acid residue R, Xᵢᵢᵢ is absent or is the amino acid residue K, Xᵢᵥ is absent or is the amino acid residue S, Xᵥ is absent or is the amino acid residue S, Xᵥᵢ is absent or is the amino acid residue V, and Xᵥᵢᵢ is absent or is the amino acid residue E.

In some embodiments, any 1, 2, 3, 4, 5, 6, or 7 amino acid residues of Xᵢ to Xᵥᵢᵢ are absent. In some embodiments, Xᵢᵥ to Xᵥᵢᵢ are absent.

In some embodiments, none of Xᵢ to Xᵥᵢᵢ comprises a cysteine residue.

In some embodiments, the hinge peptide (e.g., the first hinge peptide and/or the second hinge peptide) has an amino acid sequence as set forth in any one of SEQ ID NO: 80-89.

In some embodiments, the Fc peptide (e.g., the first Fc peptide and/or the second Fc peptide) is an Fc peptide derived from an immunoglobulin (e.g., IgG1, IgG2, IgG3 or IgG4).

In some embodiments, the Fc peptide (e.g., the first Fc peptide and/or the second Fc peptide) is an Fc peptide derived from a human immunoglobulin (e.g., IgG1, IgG2, IgG3 or IgG4).

It is readily understood by those skilled in the art that the Fc peptide derived from an immunoglobulin (e.g., human immunoglobulin) includes both Fc peptides directly derived from an immunoglobulin (e.g., human immunoglobulin) and Fc peptides obtained by modifying or engineering Fc peptides derived from an immunoglobulin (e.g., human immunoglobulin) (e.g., Fc peptide variants).

In some embodiments, the Fc peptide (e.g., the first Fc peptide and/or the second Fc peptide) is an Fc peptide derived from human immunoglobulin IgG1 or IgG4.

In some embodiments, the Fc peptide of the immunoglobulin (e.g., the first Fc peptide and/or the second Fc peptide) is an Fc peptide derived from human immunoglobulin IgG1.

In some embodiments, the Fc peptide (e.g., the first Fc peptide and/or the second Fc peptide) is selected from the Fc peptide of wild-type human immunoglobulin IgG1 and variants thereof; wherein compared with the Fc peptide of wild-type human immunoglobulin IgG1, the Fc peptide variant has reduced Fc effector functions (e.g., reduced ADCC, CDC and/or ADCP activities) and/or an extended half-life.

In some embodiments, compared with the Fc peptide of wild-type human immunoglobulin IgG1, the Fc peptide variant has reduced Fc³R binding activity, reduced serum complement molecule (C1q) binding activity, and/or enhanced FcRn binding activity.

In some embodiments, compared with the Fc peptide of wild-type human immunoglobulin IgG1, the Fc peptide variant comprises mutations selected from L234A, L235A, M252Y, S254T, T256E, and any combination thereof (e.g., selected from mutations (i): L234A and L235A, (ii) M252Y, S254T and T256E, and (iii): a combination of (i) and (ii)).

In some embodiments, compared with the Fc peptide of wild-type human immunoglobulin IgG1, the Fc peptide variant comprises mutations L234A and L235A. In some embodiments, the Fc peptide variant further comprises mutations M252Y, S254T and T256E.

In some embodiments, the mutation site of the mutation is defined by the EU numbering system.

In some embodiments, the Fc peptide (e.g., the first Fc peptide and/or the second Fc peptide) comprises a sequence as set forth in any one of SEQ ID NO: 52, 65, 76, 93.

In some embodiments, the Fc peptide (e.g., the first Fc peptide and/or the second Fc peptide) is an Fc peptide derived from human immunoglobulin IgG4.

In some embodiments, the Fc peptide (e.g., the first Fc peptide and/or the second Fc peptide) is selected from the Fc peptide of wild-type human immunoglobulin IgG4 and variants thereof; wherein compared with the Fc peptide of wild-type human immunoglobulin IgG4, the Fc peptide variant has reduced Fc effector functions (e.g., reduced ADCC, CDC and/or ADCP activities) and/or an extended half-life.

In some embodiments, compared with the Fc peptide of wild-type human immunoglobulin IgG4, the Fc peptide variant has reduced Fc³R binding activity, reduced serum complement molecule (C1q) binding activity, and/or enhanced FcRn binding activity.

In some embodiments, compared with the Fc peptide of wild-type human immunoglobulin IgG4, the Fc peptide variant comprises mutation L235E.

In some embodiments, the mutation site of the mutation is defined by the EU numbering system.

In some embodiments, the Fc peptide (e.g., the first Fc peptide and/or the second Fc peptide) comprises a sequence as set forth in SEQ ID NO: 79 or 99.

In some embodiments, the polypeptide construct comprises an amino acid sequence as set forth in any one of SEQ ID NO: 94-97 and 113-126.

In some embodiments, the polypeptide construct comprises the first peptide chain and optionally the second peptide chain; wherein the first peptide chain comprises a sequence as set forth in any one of SEQ ID NO: 94-97 and 113-126, and/or the second peptide chain comprises a sequence as set forth in any one of SEQ ID NO: 94-97 and 113-126.

In some embodiments, the polypeptide construct comprises the first peptide chain and optionally the second peptide chain; wherein:
(1) the first peptide chain and the second peptide chain each independently comprise a sequence as set forth in SEQ ID NO: 94;
(2) the first peptide chain and the second peptide chain each independently comprise a sequence as set forth in SEQ ID NO: 95;
(3) the first peptide chain and the second peptide chain each independently comprise a sequence as set forth in SEQ ID NO: 96;
(4) the first peptide chain and the second peptide chain each independently comprise a sequence as set forth in SEQ ID NO: 97;
(5) the first peptide chain and the second peptide chain each independently comprise a sequence as set forth in SEQ ID NO: 113;
(6) the first peptide chain and the second peptide chain each independently comprise a sequence as set forth in SEQ ID NO: 114;
(7) the first peptide chain and the second peptide chain each independently comprise a sequence as set forth in SEQ ID NO: 115;
(8) the first peptide chain and the second peptide chain each independently comprise a sequence as set forth in SEQ ID NO: 116;
(9) the first peptide chain and the second peptide chain each independently comprise a sequence as set forth in SEQ ID NO: 117;
(10) the first peptide chain and the second peptide chain each independently comprise a sequence as set forth in SEQ ID NO: 118;
(11) the first peptide chain and the second peptide chain each independently comprise a sequence as set forth in SEQ ID NO: 119;
(12) the first peptide chain and the second peptide chain each independently comprise a sequence as set forth in SEQ ID NO: 120;
(13) the first peptide chain and the second peptide chain each independently comprise a sequence as set forth in SEQ ID NO: 121;
(14) the first peptide chain and the second peptide chain each independently comprise a sequence as set forth in SEQ ID NO: 122;
(15) the first peptide chain and the second peptide chain each independently comprise a sequence as set forth in SEQ ID NO: 123;
(16) the first peptide chain and the second peptide chain each independently comprise a sequence as set forth in SEQ ID NO: 124;
(17) the first peptide chain and the second peptide chain each independently comprise a sequence as set forth in SEQ ID NO: 125;
(18) the first peptide chain and the second peptide chain each independently comprise a sequence as set forth in SEQ ID NO: 126.

In some embodiments, the polypeptide construct is a GHR agonistic construct (e.g., the peptide construct can serve as a GHR agonist).

In some embodiments, the polypeptide construct possesses cross-binding activity to human GHR and murine (e.g., rat) GHR.

In some embodiments, the GHR is human GHR or murine (e.g., rat) GHR.

In a fourth aspect, the present application provides an isolated nucleic acid molecule encoding the single-domain antibody or antigen-binding fragment thereof of the first aspect, or the polypeptide construct of the second or third aspect.

In some embodiments, the isolated nucleic acid molecule encodes the single-domain antibody or antigen-binding fragment thereof of the first aspect.

In some embodiments, the isolated nucleic acid molecule encodes the polypeptide construct of the second or third aspect.

In some embodiments, the polypeptide construct comprises a first peptide chain and a second peptide chain, and the isolated nucleic acid molecule comprises a first nucleotide sequence encoding the first peptide chain and a second nucleotide sequence encoding the second peptide chain, wherein the first nucleotide sequence and the second nucleotide sequence are present on the same or different isolated nucleic acid molecules. When the first nucleotide sequence and the second nucleotide sequence are present on different isolated nucleic acid molecules, the isolated nucleic acid molecule of the present application comprises a first nucleic acid molecule comprising the first nucleotide sequence and a second nucleic acid molecule comprising the second nucleotide sequence.

In a fifth aspect, the present application provides a vector comprising the isolated nucleic acid molecule of the fourth aspect. In some embodiments, the vector is a cloning vector or an expression vector.

In some embodiments, the vector comprises a nucleotide sequence encoding the single-domain antibody or antigen-binding fragment thereof of the first aspect.

In some embodiments, the vector comprises a nucleotide sequence encoding the polypeptide construct of the second or third aspect.

In some embodiments, the polypeptide construct comprises a first peptide chain and a second peptide chain, and the vector comprises a first nucleotide sequence encoding the first peptide chain and a second nucleotide sequence encoding the second peptide chain, wherein the first nucleotide sequence and the second nucleotide sequence are present on the same or different vectors. When the first nucleotide sequence and the second nucleotide sequence are present on different vectors, the vector of the present application comprises a first vector comprising the first nucleotide sequence and a second vector comprising the second nucleotide sequence.

In a sixth aspect, the present application provides a host cell comprising the isolated nucleic acid molecule of the fourth aspect or the vector of the fifth aspect.

Such host cells include, but are not limited to, prokaryotic cells such as bacterial cells (e.g., Escherichia coli cells), and eukaryotic cells such as fungal cells (e.g., yeast cells), insect cells, plant cells, and animal cells (e.g., mammalian cells, such as mouse cells and human cells). In some embodiments, the host cell is a microorganism.

The single-domain antibody or antigen-binding fragment thereof, or polypeptide construct of the present invention may be prepared by various methods known in the art, for example, by genetic engineering recombinant techniques. For example, DNA molecules encoding the single-domain antibody or antigen-binding fragment thereof, or polypeptide construct of the present invention may be obtained by chemical synthesis or PCR amplification. The obtained DNA molecules are inserted into an expression vector which is then used for transfecting host cells. Then, the transfected host cells are cultured under specific conditions to express the single-domain antibody, antigen-binding fragment thereof, or polypeptide construct of the present invention. The antigen-binding fragments of the present invention may be obtained by hydrolysis of intact antibody molecules.

In a seventh aspect, the present application provides a method for preparing the single-domain antibody or antigen-binding fragment thereof of the first aspect, or the polypeptide construct of the second or third aspect, comprising culturing the host cell of the sixth aspect under conditions permitting expression of the single-domain antibody or antigen-binding fragment thereof or the polypeptide construct, and recovering the single-domain antibody or antigen-binding fragment thereof or the polypeptide construct from the cultured host cell culture.

In an eighth aspect, the present application provides a bispecific or multispecific molecule comprising the single-domain antibody or antigen-binding fragment thereof of the first aspect, or the polypeptide construct of the second or third aspect.

In some embodiments, the bispecific or multispecific molecule specifically binds to GHR, and additionally specifically binds to one or more other targets.

In some embodiments, the bispecific or multispecific molecule further comprises at least one molecule with a second binding specificity for a second target (e.g., a second antibody or an antigen-binding fragment thereof).

In a ninth aspect, the present application provides an immunoconjugate comprising the single-domain antibody or antigen-binding fragment thereof of the first aspect, or the polypeptide construct of the second or third aspect, and a therapeutic agent linked to the single-domain antibody or antigen-binding fragment thereof or the polypeptide construct.

In some embodiments, the immunoconjugate is an antibody-drug conjugate (ADC).

In a tenth aspect, the present application provides a pharmaceutical composition comprising the single-domain antibody or antigen-binding fragment thereof of the first aspect, the polypeptide construct of the second or third aspect, the isolated nucleic acid molecule of the fourth aspect, the vector of the fifth aspect, the host cell of the sixth aspect, the bispecific or multispecific molecule of the eighth aspect, or the immunoconjugate of the ninth aspect, and a pharmaceutically acceptable carrier and/or excipient.

In some embodiments, the pharmaceutical composition further comprises an additional pharmaceutically active agent.

In some embodiments, the additional pharmaceutically active agent is selected from drugs for the treatment of diseases associated with GH, IGF-1 and/or GHRH deficiency.

In an eleventh aspect, the present application provides the use of the single-domain antibody or antigen-binding fragment thereof of the first aspect, the polypeptide construct of the second or third aspect, the isolated nucleic acid molecule of the fourth aspect, the vector of the fifth aspect, the host cell of the sixth aspect, the bispecific or multispecific molecule of the eighth aspect, the immunoconjugate of the ninth aspect, or the pharmaceutical composition of the tenth aspect in the manufacture of a drug for the prevention and/or treatment of a disease associated with GHR signaling in a subject.

In some embodiments, the prevention and/or treatment of the disease associated with GHR signaling would benefit from agonism of GHR signaling.

In some embodiments, the disease associated with GHR signaling is selected from diseases associated with deficiency of GH (growth hormone), IGF-1 (insulin-like growth factor-1) and/or GHRH (growth hormone-releasing hormone).

In some embodiments, the disease associated with GHR signaling is selected from: growth hormone deficiency (GHD) (e.g., growth hormone deficiency caused by hypothalamic-pituitary disorders), chronic kidney disease (CKD), Turner syndrome, Prader-Willi syndrome, small for gestational age infant (SGA), idiopathic short stature (ISS), SHOX gene deficiency, Noonan syndrome, pediatric short stature (e.g., pediatric short stature caused by achondroplasia (ACH), etc.), severe burns, adult growth hormone deficiency, adult short bowel syndrome, HIV wasting syndrome, aging, reproductive disorders (e.g., reproductive disorders associated with the growth hormone-growth hormone receptor signaling pathway, such as reproductive disorders resulting from gonadal dysgenesis due to growth disorders caused by Turner syndrome).

In some embodiments, the subject is a mammal, such as a human or a murine.

In some embodiments, the single-domain antibody or antigen-binding fragment thereof, polypeptide construct, isolated nucleic acid molecule, vector, host cell or pharmaceutical composition is used alone or administered in combination with an additional pharmaceutically active agent (e.g., administered simultaneously or sequentially).

In some embodiments, the additional pharmaceutically active agent is selected from drugs for the treatment of diseases associated with GH, IGF-1 and/or GHRH deficiency.

In a twelfth aspect, the present application provides a method for preventing and/or treating a disease associated with GHR signaling in a subject, comprising: administering to a subject in need thereof an effective amount of the single-domain antibody or antigen-binding fragment thereof of the first aspect, the polypeptide construct of the second or third aspect, the isolated nucleic acid molecule of the fourth aspect, the vector of the fifth aspect, the host cell of the sixth aspect, the bispecific or multispecific molecule of the eighth aspect, the immunoconjugate of the ninth aspect, or the pharmaceutical composition of the tenth aspect.

In some embodiments, the prevention and/or treatment of the disease associated with GHR signaling would benefit from agonism of GHR signaling.

In some embodiments, the disease associated with GHR signaling is selected from diseases associated with deficiency of GH (growth hormone), IGF-1 (insulin-like growth factor-1) and/or GHRH (growth hormone-releasing hormone).

In some embodiments, the disease associated with GHR signaling is selected from: growth hormone deficiency (GHD) (e.g., growth hormone deficiency caused by hypothalamic-pituitary disorders), chronic kidney disease (CKD), Turner syndrome, Prader-Willi syndrome, small for gestational age infant (SGA), idiopathic short stature (ISS), SHOX gene deficiency, Noonan syndrome, pediatric short stature (e.g., pediatric short stature caused by achondroplasia (ACH), etc.), severe burns, adult growth hormone deficiency, adult short bowel syndrome, HIV wasting syndrome, aging, reproductive disorders (e.g., reproductive disorders associated with the growth hormone-growth hormone receptor signaling pathway, such as reproductive disorders resulting from gonadal dysgenesis due to growth disorders caused by Turner syndrome).

In some embodiments, the subject is a mammal, such as a human or a murine.

In some embodiments, the single-domain antibody or antigen-binding fragment thereof, polypeptide construct, isolated nucleic acid molecule, vector, host cell or pharmaceutical composition is used alone or administered in combination with an additional pharmaceutically active agent (e.g., administered simultaneously or sequentially).

In some embodiments, the additional pharmaceutically active agent is selected from drugs for the treatment of diseases associated with GH, IGF-1 and/or GHRH deficiency.

The single-domain antibody or antigen-binding fragment thereof, or polypeptide construct or pharmaceutical composition of the present application can be formulated into any dosage form known in the medical arts, for example, tablets, pills, suspensions, emulsions, solutions, gels, capsules, powders, granules, elixirs, lozenges, suppositories, injections (including injection solutions, sterile powders for injection, and concentrated solutions for injection), inhalants, sprays, etc. The preferred dosage form depends on the intended mode of administration and therapeutic use. The single-domain antibody or antigen-binding fragment thereof, polypeptide construct, or pharmaceutical composition of the present invention shall be sterile and stable under the conditions of manufacture and storage. A preferred dosage form is an injection. Such injections may be sterile injection solutions. For example, a sterile injectable solution may be prepared by incorporating the required dose of the single-domain antibody or antigen-binding fragment thereof, polypeptide construct, or pharmaceutical composition of the present invention in a suitable solvent, and optionally, simultaneously incorporating other desired ingredients (including but not limited to pH adjusters, surfactants, adjuvants, ionic strength enhancers, diluents, reagents for maintaining osmotic pressure, reagents for delaying absorption, preservatives, stabilizers, or any combination thereof), followed by sterile filtration. Additionally, the sterile injectable solutions can be prepared as sterile lyophilized powders (e.g., by vacuum drying or freeze drying) for ease of storage and use. Such sterile lyophilized powders can be dispersed in suitable carriers before use, such as water for injection (WFI), bacteriostatic water for injection (BWFI), a sodium chloride solution (e.g., 0.9% NaCl), a glucose solution (e.g., 5% glucose), a solution comprising a surfactant (e.g., 0.01% polysorbate 20), a pH buffer (e.g., phosphate buffer), a Ringer's solution, and any combination thereof.

The single-domain antibody or antigen-binding fragment thereof, or polypeptide construct or pharmaceutical composition of the present application can be administered by any suitable method known in the art, including but not limited to, oral, buccal, sublingual, ocular, topical, parenteral, rectal, intrathecal, intracytoplasmic reticulum, inguinal, intravesical, topical (e.g., powders, ointments or drops), or nasal routes. However, for many therapeutic uses, the preferred route/mode of administration is parenteral administration (e.g., intravenous or bolus injection, subcutaneous injection, intraperitoneal injection, and intramuscular injection). It will be appreciated by technicians that the route and/or mode of administration will vary depending upon the intended purpose. In some embodiments, the single-domain antibody or antigen-binding fragment thereof, polypeptide construct, or pharmaceutical composition of the present invention is administered by intravenous injection or bolus.

In a thirteenth aspect, the present application further provides a conjugate comprising the single-domain antibody or antigen-binding fragment thereof of the first aspect, or the polypeptide construct of the second or third aspect, and a detectable label linked to the single-domain antibody or antigen-binding fragment thereof or the polypeptide construct.

In some embodiments, the detectable label is selected from an enzyme (e.g., horseradish peroxidase or alkaline phosphatase), a chemiluminescent reagent (e.g., acridinium ester compounds, luminol and derivatives thereof, or ruthenium derivatives), a fluorescent dye (e.g., fluorescein or fluorescent protein), a radionuclide, or a biotin.

In a fourteenth aspect, the present application further provides a kit comprising the single-domain antibody or antigen-binding fragment thereof of the first aspect, or the polypeptide construct of the second or third aspect, or the conjugate of the thirteenth aspect.

In some embodiments, the kit comprises a detection buffer.

In some embodiments, the kit comprises the conjugate of the thirteenth aspect.

In some embodiments, the kit comprises the single-domain antibody or antigen-binding fragment thereof of the first aspect, or the polypeptide construct of the second or third aspect, and a second antibody that specifically recognizes the single-domain antibody or antigen-binding fragment thereof or the polypeptide construct. Optionally, the second antibody further comprises a detectable label, such as an enzyme (e.g., horseradish peroxidase or alkaline phosphatase), a chemiluminescent reagent (e.g., acridinium ester compounds, luminol and derivatives thereof, or ruthenium derivatives), a fluorescent dye (e.g., fluorescein or fluorescent protein), a radionuclide, or a biotin.

In a fifteenth aspect, the present application further provides a method for detecting the presence or level of GHR in a sample, comprising using the single-domain antibody or antigen-binding fragment thereof of the first aspect, the polypeptide construct of the second or third aspect, or the conjugate of the thirteenth aspect.

In some embodiments, the method is used for therapeutic purposes, diagnostic purposes, or non-therapeutic and non-diagnostic purposes.

In some embodiments, the method is an immunoassay, such as Western blotting, enzyme immunoassay (e.g., ELISA), chemiluminescent immunoassay, fluorescent immunoassay, or radioimmunoassay.

In some embodiments, the method comprises using the conjugate of the thirteenth aspect.

In some embodiments, the method comprises using the single-domain antibody or antigen-binding fragment thereof of the first aspect or the polypeptide construct of the second or third aspect, and the method further comprises using a second antibody carrying a detectable label (e.g., an enzyme (e.g., horseradish peroxidase or alkaline phosphatase), a chemiluminescent reagent (e.g., acridinium ester compounds, luminol and derivatives thereof, or ruthenium derivatives), a fluorescent dye (e.g., fluorescein or fluorescent protein), a radionuclide, or a biotin) to detect the single-domain antibody or antigen-binding fragment thereof or the polypeptide construct.

In some embodiments, the method comprises: (1) contacting the sample with the single-domain antibody or antigen-binding fragment thereof, polypeptide construct, or conjugate of the present invention; and (2) detecting the formation of an antigen-antibody immune complex, or detecting the amount of the immune complex. The formation of the immune complex indicates the presence of GHR or GHR-expressing cells.

The present application further provides a method for diagnosing a GHR-related disease (e.g., a disease associated with abnormal GHR level or structure), comprising detecting the presence or level of GHR in a sample from a subject using the method of the fifteenth aspect. In some embodiments, the presence of GHR or an increased level of GHR relative to a reference level (e.g., relative to a healthy control) indicates that the subject has a GHR-related disease (e.g., a disease associated with abnormal GHR level or structure).

In some embodiments, the GHR-related disease is selected from Laron syndrome.

In some embodiments, the GHR is human GHR or murine (e.g., rat) GHR.

In some embodiments, the subject is a mammal (preferably a human or a murine).

In some embodiments, the sample is a tissue sample or cell sample from a subject (e.g., a mammal, preferably a human or a murine).

In a sixteenth aspect, the present application further provides the use of the single-domain antibody or antigen-binding fragment thereof of the first aspect, the polypeptide construct of the second or third aspect, or the conjugate of the thirteenth aspect in the manufacture of a detection reagent for detecting the presence or level of GHR in a sample and/or diagnosing a GHR-related disease (e.g., a disease associated with abnormal GHR level or structure).

In some embodiments, the detection reagent is used to detect the presence or level of GHR in a sample by the method of the fifteenth aspect.

In some embodiments, the detection reagent detects the presence or level of GHR in a sample by the method of the fifteenth aspect, thereby diagnosing a GHR-related disease (e.g., a disease associated with abnormal GHR level or structure). In some embodiments, the presence of GHR or an increased level of GHR relative to a reference level (e.g., relative to a healthy control) indicates that the subject has a GHR-related disease (e.g., a disease associated with abnormal GHR level or structure).

In some embodiments, the GHR-related disease is selected from Laron syndrome.

In some embodiments, the GHR is human GHR or murine (e.g., rat) GHR.

In some embodiments, the subject is a mammal (preferably a human or a murine).

In some embodiments, the sample is a tissue sample or cell sample from a subject (e.g., a mammal, preferably a human or a murine).

### Definitions of terms

In the present invention, unless otherwise specified, the scientific and technical terms used herein have the meanings commonly understood by a person of skill in the art. Furthermore, the virology, biochemistry, and immunology laboratory operation procedures used herein are all conventional procedures widely used in the corresponding fields. Moreover, in order to better understand the present invention, definitions and explanations of relevant terms are provided below.

When the terms "for example", "e.g.", "such as", "including", "comprising", or variations thereof are used herein, these terms will not be considered as restrictive terms, but will be interpreted to mean "but not limited to" or "not limited to".

The terms "a", "an", "the", and similar referents in the context of describing the present invention (especially in the context of the following claims) should be construed to cover both the singular and the plural referents, unless otherwise indicated herein or clearly contradicted by context.

As used herein, the term <single-domain antibody (sdAb)= has the meaning commonly understood by those skilled in the art, and refers to an antibody fragment consisting of a single monomeric variable antibody domain (e.g., a single heavy-chain variable region, VHH), typically derived from the variable region of a heavy-chain antibody (e.g., a camelid antibody or a cartilaginous fish antibody).

Single-domain antibodies are also referred to as nanobodies, and the two terms are used interchangeably. Typically, a nanobody is composed of 4 framework regions and 3 complementarity-determining regions, with the structure FR1-CDR1-FR2-CDR2-FR3-CDR3-FR4. A nanobody may be truncated at the N-terminus or C-terminus such that it comprises only part of FR1 and/or FR4, or lacks one or both of those framework regions, so long as it substantially retains antigen binding and specificity.

As used herein, the term <antigen-binding fragment= of a single-domain antibody refers to a polypeptide comprising a fragment of a single-domain antibody that retains the ability to specifically bind the same antigen bound by the single-domain antibody, and/or competes with the single-domain antibody for specific binding to the antigen, and is also referred to as an <antigen-binding portion=. Generally, see Fundamental Immunology, Ch. 7 (Paul, W., ed., 2nd ed., Raven Press, N.Y. (1989), which is incorporated herein by reference in its entirety for all purposes. Antigen-binding fragments of the nanobodies of the present invention may be produced by recombinant DNA techniques or by enzymatic or chemical cleavage of the nanobodies of the present invention. In some embodiments, the <antigen-binding fragment= of the single-domain antibody may be truncated at the N-terminus or C-terminus relative to the full-length single-domain antibody such that it comprises only part of FR1 and/or FR4, or lacks one or both of those framework regions, so long as it substantially retains antigen binding and specificity.

Antigen-binding fragments of a single-domain antibody may be obtained from a given single-domain antibody (e.g., a nanobody provided herein) using conventional techniques known to those skilled in the art (e.g., recombinant DNA techniques or enzymatic or chemical cleavage methods), and screened for specificity in the same manner as for intact nanobodies.

As used herein, unless the context clearly indicates otherwise, reference to the term <single-domain antibody= includes not only the intact single-domain antibody but also antigen-binding fragments of the single-domain antibody.

As used herein, the term "complementarity-determining region" or "CDR" refers to amino acid residues responsible for antigen binding in a variable region of an antibody. A nanobody contains three CDRs, designated CDR1, CDR2 and CDR3. The precise boundaries of these CDRs can be defined according to various numbering systems known in the art, such as the definitions in the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), or the IMGT numbering system (Lefranc et al., Dev. Comparat. Immunol. 27:55-77, 2003). For a given nanobody, a person of skill in the art will readily identify the CDRs defined by each numbering system. Furthermore, the corresponding relationship between different numbering systems is well known to a person of skill in the art (for example, see Lefranc et al., Dev. Comparat. Immunol. 27:55-77, 2003). In the present invention, the CDR comprised in the single-domain antibody or antigen-binding fragment thereof of the present invention can be determined according to various numbering systems known in the art. In some embodiments, the CDR comprised in the single-domain antibody or antigen-binding fragment thereof of the present invention is preferably determined by the Kabat, Chothia or IMGT numbering system.

As used herein, the term "framework region" or "FR" residues refer to those amino acid residues in variable regions of an antibody other than the CDR residues as defined above.

As used herein, the term <polypeptide construct= is employed in its broadest sense. In general, the polypeptide construct refers to a construct comprising one or more polypeptide or protein moieties, wherein each of the one or more polypeptide or protein moieties may independently be derived from different sources or possess different biological activities or functions, and are linked covalently and/or non-covalently (e.g., covalently linked via covalent bonds including peptide bonds, isopeptide bonds and/or disulfide bonds, and/or non-covalently linked via hydrogen bonds). The polypeptide construct of the present invention is not limited in the number of molecular chains (e.g., peptide chains). For example, the polypeptide construct of the present invention may comprise only one molecular chain (e.g., peptide chain), or two or more molecular chains (e.g., peptide chains), wherein the two or more molecular chains (e.g., peptide chains) are linked covalently and/or non-covalently (e.g., covalently linked via covalent bonds including peptide bonds, isopeptide bonds and/or disulfide bonds, and/or non-covalently linked via hydrogen bonds). Likewise, it will be readily understood by those skilled in the art that, in embodiments comprising multiple polypeptide or protein moieties, the multiple polypeptide or protein moieties comprised in the polypeptide construct of the present invention may be located, in whole or in part, in the same molecular chain (e.g., peptide chain), or may each be located in different molecular chains (e.g., peptide chains).

As used herein, the terms <Fc peptide,= <Fc,= <Fc fragment,= or <Fc region= refer to an antibody fragment formed from the second constant region (CH2) and the third constant region (CH3) of the immunoglobulin heavy chain. The Fc fragment of an antibody has a variety of different functions but is not involved in antigen binding. For example, the Fc peptide can mediate the binding of immunoglobulins to host tissues or factors, including various cells of the immune system (such as effector cells) and components of the complement system. In some embodiments, the Fc peptide comprises a hinge region located between the CH1 and CH2 regions. In some embodiments, the Fc peptide does not comprise a hinge region located between the CH1 and CH2 regions.

As used herein, the term "hinge peptide" or "hinge region" refers to a hinge peptide of the immunoglobulin heavy chain located at the C-terminus of the CH1 region of the immunoglobulin heavy chain, which is used to connect to the CH2 region of the immunoglobulin heavy chain. The hinge peptide typically contains cysteine, which can be used to form a disulfide bond between two heavy chains of an immunoglobulin containing the hinge peptide, thereby mediating the dimerization between two Fc peptides contained in the two heavy chains.

As used herein, the term <heavy chain antibody= (HCAb) or <heavy-chain-only antibody= refers to an antibody that lacks the light chain of a conventional antibody. In some embodiments, the heavy chain antibody includes, but is not limited to, a dimer antibody comprising a VHH antigen-binding domain and a CH2 and/or CH3 constant domain, as well as an optional hinge region, in the absence of a CH1 domain. In some embodiments, the heavy chain antibody comprises at least a portion of a VHH antigen-binding domain, a hinge region, and a CH2 and/or CH3 domain.

As used herein, the term <bispecific antibody= or <bispecific molecule= refers to an antibody with binding specificity for two different antigens (or epitopes). The term <multispecific antibody= or <multispecific molecule= refers to an antibody with binding specificity for at least two or more (e.g., three or four) different antigens (or epitopes). The bispecific antibody or the multispecific antibody comprises multiple antigen-binding domains having a binding specificity for different antigens (or epitopes), and thus is able to bind to at least two different binding sites and/or target molecules.

The term "antibody" as used herein refers to an immunoglobulin-derived molecule capable of specifically binding to a target antigen, and the immunoglobulin-derived molecule binds to the target antigen through at least one antigen-binding site located in a variable region thereof. An "intact antibody" typically consists of two pairs of polypeptide chains, each pair having one light chain (LC) and one heavy chain (HC). Light chains of the antibody can be classified as kappa (κ) and lambda (λ) light chains. Heavy chains can be classified as µ, δ, γ, α or ε, and the isotype of the antibody is respectively defined as IgM, IgD, IgG, IgA, and IgE. Within light and heavy chains, the variable and constant regions are joined by a "J" region of about 12 or more amino acids, and the heavy chain also comprises a "D" region of about 3 or more amino acids. Each heavy chain consists of a heavy chain variable region (VH) and a heavy chain constant region (CH). The heavy chain constant region consists of 3 domains (CH1, CH2, and CH3). Each light chain consists of a light chain variable region (VL) and a light chain constant region (CL). The light chain constant region consists of one domain CL. The constant domain is not directly involved in binding of an antibody to an antigen, but exhibits various effector functions, such as mediating the binding of immunoglobulins to host tissues or factors, including binding of various cells (e.g., effector cells) of the immune system and the first component (C1q) of the classical complement system. VH and VL regions can be further subdivided into regions with high variability (called complementarity-determining regions (CDRs)) interspersed with relatively conserved regions called framework regions (FRs). Each of the VH and VL consists of 3 CDRs and 4 FRs arranged from an amino terminal to a carboxyl terminal in the following order: FR1, CDR1, FR2, CDR2, FR3, CDR3, and FR4. The variable regions (VH and VL) of each heavy chain/light chain pair respectively form an antigen-binding site. The distribution of amino acids in each region or domain can be based on the definitions in Kabat, Sequences of Proteins of Immunological Interest (National Institutes of Health, Bethesda, Md. (1987 and 1991)), or Chothia & Lesk (1987) J. Mol. Biol. 196:901-917; Chothia et al. (1989) Nature 342:878-883.

As used herein, the term <humanized= refers to the modification of the amino acid sequence of a non-human antibody by genetic engineering to improve its homology to the sequence of a human antibody. Generally speaking, all or part of CDR regions of a humanized antibody are derived from a non-human antibody (donor antibody), and all or part of non-CDR regions (e.g., variable region FR and/or constant region) are derived from a human immunoglobulin (receptor antibody). In some embodiments, the CDR regions of a humanized antibody are derived from a non-human antibody (donor antibody), and all or part of non-CDR regions (e.g., variable region FR and/or constant region) are derived from a human immunoglobulin (receptor antibody). A humanized antibody generally retains the desired properties of a donor antibody, including but not limited to, antigen specificity, affinity, reactivity, etc. In the present application, the donor antibody may be a camelid antibody having desired properties (e.g., antigen specificity, affinity, reactivity). To prepare a humanized antibody, CDR regions of a donor antibody can be inserted into human framework sequences by a method known in the art. In some cases, the human framework sequence may comprise amino acid mutations that are substituted with corresponding non-human residues. In addition, a humanized antibody may also comprise residues that are not found in a variable region (e.g., light chain variable region or heavy chain variable region) of an initial donor antibody or in a human framework sequence to further improve or optimize the performance of the humanized antibody. In some embodiments, when humanizing a single-domain antibody, all or part of the CDR regions of the humanized antibody are derived from a non-human single-domain antibody (donor antibody), and all or part of the non-CDR regions (e.g., FRs of the variable domain and/or constant regions) are derived from the heavy chain of a human immunoglobulin (receptor antibody). In some embodiments, the CDR regions of a humanized antibody are derived from a non-human single-domain antibody (donor antibody), and all or part of non-CDR regions (e.g., variable region FR and/or constant region) are derived from a human immunoglobulin (receptor antibody).

As used herein, the term "identity" is used for referring to the matching of sequences between two polypeptides or between two nucleic acids. To determine the percent identity between two amino acid sequences or two nucleic acid sequences, the sequences are aligned for optimal comparison purposes (for example, gaps can be introduced in a first amino acid or nucleic acid sequence for optimal alignment with a second amino acid or nucleic acid sequence). Then, amino acid residues or nucleotides at corresponding amino acid positions or nucleotide positions are compared. When a position in the first sequence is occupied by the same amino acid residue or nucleotide as the corresponding position in the second sequence, molecules are identical at that position. The percent identity between two sequences is a function of the number of identical positions shared by the sequences (i.e., percent identity = number of identical overlapping positions/total number of positions x 100%). In some embodiments, two sequences have the same length.

The determination of the percent identity between two sequences can also be achieved by a mathematical algorithm. A non-limiting example of a mathematical algorithm for comparison of two sequences is improved in algorithms of Karlin and Altschul, 1990, Proc. Natl. Acad. Sci. U.S.A. 87:2264-2268, as in Karlin and Altschul, 1993, Proc. Natl. Acad. Sci. U.S.A. 90:5873-5877. Such an algorithm is integrated into NBLAST and XBLAST programs of Altschul et al., 1990, J. Mol. Biol. 215:403.

As used herein, the term "variant", in the context of polypeptides (including polypeptides), also refers to a polypeptide or peptide comprising an amino acid sequence that has been altered by the introduction of amino acid residue substitutions, deletions or insertions. In some cases, the term "variant" also refers to a polypeptide or peptide that has been modified (i.e., by covalent linkage of any type of molecule to the polypeptide or peptide). For example, but not limitation, the polypeptide can be modified, for example, by glycosylation, acetylation, pegylation, phosphorylation, amidation, derivatization by known protecting/blocking groups, proteolytic cleavage, linkage to cellular ligands or other proteins, etc. Derived polypeptides or peptides can be produced by chemical modification using technologies known to a person of skill in the art. The technologies include but not limited to specific chemical cleavage, acetylation, formylation, metabolic synthesis of tunicamycin, etc. Furthermore, the variant has similar, identical or improved functions as the polypeptide or peptide from which it is derived.

As used herein, the term "specific binding" refers to a non-random binding reaction between two molecules, such as the reaction between an antibody and a targeted antigen. The strength or affinity of a specific binding interaction can be expressed in terms of an equilibrium dissociation constant (K_{D}) for the interaction. In the present invention, the term "K_{D}" refers to a dissociation equilibrium constant for a specific antibody-antigen interaction, which is used for describing the binding affinity between an antibody and an antigen. The smaller the equilibrium dissociation constant, the tighter the antibody-antigen binding, and the higher the affinity between the antibody and the antigen.

The specific binding properties between two molecules can be determined by a method known in the art. One method involves measurement of the rates of formation and dissociation of antigen-binding sites/antigen complexes. Both the "association rate constant" (ka or kon) and the "dissociation rate constant" (kdis or koff) can be calculated by the concentration and the actual rates of association and dissociation (see Malmqvist M, Nature,1993, 361:186-187). The ratio of kdis/kon is equal to the dissociation constant K_{D} (see Davies et al., Annual Rev Biochem, 1990; 59:439-473). The values of the K_{D}, kon and kdis can be measured by any effective method. In some embodiments, the dissociation constant can be measured in Biacore by surface plasmon resonance (SPR). In addition, bioluminescence interferometry or Kinexa can be used for measuring the dissociation constant.

As used herein, the term "vector" refers to a nucleic acid delivery vehicle into which a polynucleotide can be inserted. When the vector can express the protein encoded by the inserted polynucleotide, the vector is called an expression vector. The vector can be introduced into the host cell by transformation, transduction or transfection, so that the genetic material elements carried by the vector are expressed in the host cell. Vectors are well known to those skilled in the art, and include but are not limited to: plasmids; bacteriophagemids; cosmids; artificial chromosomes, such as yeast artificial chromosomes (YACs), bacterial artificial chromosomes (BACs), or P1-derived artificial chromosomes (PACs); and bacteriophages such as λ bacteriophages or M13 bacteriophages and animal viruses. Animal viruses that can be used as vectors include, but are not limited to, retroviruses (including lentiviruses), adenoviruses, adeno-associated viruses, herpes viruses (e.g., herpes simplex viruses), poxviruses, baculoviruses, papillomaviruses, and papovaviruses (e.g., SV40). A vector may comprise a variety of elements for controlling expression, including, but not limited to, promoter sequences, transcription initiation sequences, enhancer sequences, selection elements, and reporter genes. In addition, the vector may also comprise a replication origin.

As used herein, the term "host cell" refers to a cell that can be used for introducing a vector, including but not limited to prokaryotic cells such as Escherichia coli or Bacillus subtilis, fungal cells such as yeast cells or Aspergillus, insect cells such as S2 Drosophila cells or Sf9, or animal cells such as fibroblasts, CHO cells, COS cells, NSO cells, HeLa cells, BHK cells, HEK 293 cells or human cells.

As used herein, the term "conservative substitution" means an amino acid substitution that does not adversely affect or alter the desired properties of the protein/polypeptide comprising the amino acid sequence. For example, conservative substitutions can be introduced by standard technologies known in the art, such as site-directed mutagenesis and PCR-mediated mutagenesis. Conservative amino acid substitutions include substitution of an amino acid residue with an amino acid residue having a similar side chain, such as a residue that is physically or functionally similar to the corresponding amino acid residue (e.g., having similar size, shape, charge and chemical properties, including the ability to form covalent bonds or hydrogen bonds). Families of amino acid residues having similar side chains have been defined in the art. These families include amino acids with basic side chains (e.g., lysine, arginine, and histidine), acidic side chains (e.g., aspartic acid and glutamic acid), uncharged polar side chains (e.g., glycine, asparagine, glutamine, serine, threonine, tyrosine, cysteine, and tryptophan), nonpolar side chains (e.g., alanine, valine, leucine, isoleucine, proline, phenylalanine, and methionine), β-branched side chains (e.g., threonine, valine, and isoleucine), and aromatic side chains (e.g., tyrosine, phenylalanine, tryptophan, and histidine). Thus, it is preferred to replace a corresponding amino acid residue with another amino acid residue from the same side chain family. Methods for identifying conservative amino acid substitutions are well known in the art (see, e.g. Brummell et al., Biochem. 32:1180-1187 (1993); Kobayashi et al. Protein Eng. 12(10):879-884 (1999); and Burks et al. Proc. Natl Acad. Set USA 94:412-417 (1997), which is incorporated herein by reference).

Twenty conventional amino acids mentioned herein are written in accordance with conventional usage. See, e.g. Immunology-A Synthesis (2nd Edition, E. S. Golub and D. R. Gren, Eds., Sinauer Associates , Sunderland, Mass. (1991)), which is incorporated herein by reference. In the present invention, the terms "polypeptide" and "protein" have the same meaning and are used interchangeably. Moreover, in the present invention, amino acids are generally represented by single-letter and three-letter abbreviations well known in the art. For example, alanine can be represented by A or Ala.

Unless otherwise specified herein or clearly contradicted by the context, <A, B and/or C= or similar expressions should be understood as <A, B, C or any combination thereof=, for example, it can be understood as any one of A, B, C, A and B, A and C, B and C, A and B and C.

As used herein, the term "pharmaceutically acceptable carrier and/or excipient" refers to a carrier and/or excipient that is pharmacologically and/or physiologically compatible with a subject and active ingredients, which is well known in the art (see, e.g., Remington's Pharmaceutical Sciences. Edited by Gennaro AR, 19th ed. Pennsylvania: Mack Publishing Company, 1995), and includes, but is not limited to: pH adjusting agents, surfactants, adjuvants, ionic strength enhancers, diluents, reagents for maintaining osmotic pressure, reagents for delaying absorption, preservatives, and stabilizers. For example, pH adjusting agents include, but are not limited to, phosphate buffers. Surfactants include, but are not limited to, cationic, anionic or nonionic surfactants, such as Tween-80. Ionic strength enhancers include, but are not limited to, sodium chloride. Reagents for maintaining osmotic pressure include, but are not limited to, sugars, NaCl, and analogs thereof. Reagents for delaying absorption include, but are not limited to, monostearates and gelatin. Diluents include, but are not limited to, water, aqueous buffers (e.g., buffered saline), alcohols, polyols (e.g., glycerol), etc. Preservatives include, but are not limited to, various antibacterial reagents and antifungal reagents such as thimerosal, 2-phenoxyethanol, parabens, chlorobutanol, phenol, sorbic acid, etc. Stabilizers have the meanings generally understood by a person of skill in the art, which are capable of stabilizing the desired activity of active ingredients in drugs, and include, but are not limited to, sodium glutamate, gelatin, SPGA, saccharides (e.g., sorbitol, mannitol, starch, sucrose, lactose, dextran, or glucose), amino acids (e.g., glutamic acid and glycine), proteins (e.g., dried whey, albumin or casein), or degradation products thereof (e.g., lactalbumin hydrolysate), etc. In some exemplary embodiments, the pharmaceutically acceptable carrier or excipient includes sterile injectable liquid (e.g., an aqueous or non-aqueous suspension or solution). In some exemplary embodiments, such sterile injectable liquid is selected from water for injection (WFI), bacteriostatic water for injection (BWFI), a sodium chloride solution (e.g., 0.9% NaCl), a glucose solution (e.g., 5% glucose), a solution comprising a surfactant (e.g., 0.01% polysorbate 20), a pH buffer (e.g., phosphate buffer), a Ringer's solution, and any combination thereof.

As used herein, the term "prevention" refers to a method implemented for preventing or delaying the occurrence of a disease or disorder or symptom in a subject. As used herein, the term "treatment" refers to a method implemented for obtaining beneficial or desired clinical results. For purposes of the present invention, beneficial or desired clinical results include, but are not limited to, alleviation of symptoms, reduction in the extent of diseases, stabilization (i.e., no longer worsening) of disease states, delay or slowing of disease progression, amelioration or palliation of disease states, and relief of symptoms (regardless partial or complete), regardless detectable or undetectable. In addition, the "treatment" may also mean prolonging the survival time as compared to the expected survival time (e.g., survival time without treatment).

As used herein, the term "subject" refers to a mammal, such as a human or a murine (e.g., a rat). In some embodiments, the subject (e.g., a human or a murine) has a disease associated with GHR signaling (e.g., a disease associated with deficiency of GH (growth hormone), IGF-1 (insulin-like growth factor-1) and/or GHRH (growth hormone-releasing hormone)), or is at risk of developing the above disease.

As used herein, the term "effective amount" refers to an amount sufficient to achieve or at least partially achieve desired effects. For example, an effective amount for preventing a disease (e.g., a disease associated with a deficiency of GH (growth hormone), IGF-1 (insulin-like growth factor-1), and/or GHRH (growth hormone-releasing hormone)) refers to an amount sufficient to prevent, inhibit, or delay the onset of the disease; an effective amount for treating a disease refers to an amount sufficient to cure, or at least partially arrest, the disease and its complications in a patient already suffering from the disease. Determination of such effective amounts is completely within the capability of those skilled in the art. For example, amounts effective for therapeutic use will depend on the severity of the disease to be treated, the overall state of the immune system of the patient, the general conditions of the patient such as age, weight and sex, the manner of administration of a drug, other therapies administered simultaneously, and the like.

### Beneficial effects of the present invention

Compared with existing GHR agonists (such as GH or modified variants thereof), the agonistic single-domain antibodies specifically binding GHR or polypeptide constructs comprising the same provided in the present application exhibit at least one or more of the following beneficial effects:
1) Existing GHR agonists engineered from GH itself (e.g., GH-Fc fusion protein-based GHR agonists) show unsatisfactory therapeutic effects in patients with GHR mutations (e.g., point mutations) or in the presence of GH-neutralizing antibodies, whereas the agonistic antibodies provided herein are theoretically effective in these two patient populations;
2) The agonistic antibodies provided herein do not interact with the prolactin receptor, and thus are free of breast-related adverse reactions;
3)The polypeptide constructs provided herein possess a long half-life comparable to that of general in vivo antibodies, which can greatly improve patient compliance compared with existing long-acting GHR agonist formulations;
4) The agonistic single-domain antibodies or polypeptide constructs provided herein induce a milder increase in IGF-1 levels than GH, thereby avoiding potential safety risks associated with high-dose administration;
5) The polypeptide constructs provided herein belong to heavy-chain antibodies and exhibit high stability and developability.

The embodiments of the present invention will be described in detail below in conjunction with the accompanying drawings and examples, but a person of skill in the art will understand that the following accompanying drawings and examples are only used for illustrating the present invention rather than limiting the scope of the present invention. Various purposes and advantages of the present invention will become apparent to a person of skill in the art according to the accompanying drawings and the following detailed descriptions of the preferred embodiments.

### BRIEF DESCRIPTION OF THE DRAWINGS

Fig. 1: ELISA binding activity assay results of P15164-1 molecule to hGHBP-mFc.
Fig. 2: Activity assay results of P15164-1 molecule in promoting the proliferation of Baf3-hGHR cells.
Fig. 3: Activity assay results of SZ0022 to SZ0030 in promoting the proliferation of Baf3-hGHR cells.
Fig. 4: Schematic diagram of the structure of an exemplary heavy chain antibody molecule of the present invention.
Fig. 5: Activity assay results of SZ0022 and SZ0035 to SZ0044 in promoting the proliferation of Baf3-hGHR cells.
Fig. 6A: Binding activity assay results of SZ20022 and SZ0051 to SZ0053 to HEK293-hGHR cells.
Fig. 6B: Binding activity assay results of SZ20022 and SZ0054 to SZ0056 to HEK293-hGHR cells.
Fig. 7: Activity assay results of SZ0022 and SZ0051 to SZ0056 in promoting the proliferation of Baf3-hGHR cells.
Fig. 8: Activity assay results of SZ0055 to SZ0056 in promoting the proliferation of Baf3-rGHR cells.
Fig. 9A: Binding activity assay results of SZ0055 to rat PRLR.
Fig. 9B: Binding activity assay results of SZ0055 to human PRLR.
Fig. 10: Binding activity assay results of GX-H9 to human PRLR and rat PRLR.
Fig. 11: Changes in body weight of animals in each group after administration.
Fig. 12: Changes in serum IGF-1 levels of animals in each group after administration.
Fig. 13: Body length of animals in each group after administration.
Fig. 14: Tibia length of animals in each group after administration.
Fig. 15: Results of the PK study in cynomolgus monkeys.

### Sequence information

The description of the sequences involved in the present application is provided in the following table.

**Table 1.1: Sequence information**

| **SEQ ID NO:** | **Sequence and description** |
|---|---|
| **52** | **hIgG1 Fc** |
| | APELLGGPSVFLFPPKPKDTLMISRTPEVTCVVVDVSHEDPEVKFNWY VDGVEVHNAKTKPREEQYNSTYRVVSVLTVLHQDWLNGKEYKCKVS NKALPAPIEKTISKAKGQPREPQVYTLPPSRDELTKNQVSLTCLVKGF YPSDIAVEWESNGQPENNYKTTPPVLDSDGSFFLYSKLTVDKSRWQQ GNVFSCSVMHEALHNHYTQKSLSLSPGK |
| **53** | **hGHBP-His (human GHBP (GH-binding protein)-Avi tag-His tag)** |
| | MDLWQLLLTLALAGSSDAFSGSEATAAILSRAPWSLQSVNPGLKTNSS KEPKFTKCRSPERETFSCHWTDEVHHGTKNLGPIQLFYTRRNTQEW TQEWKECPDYVSAGENSCYFNSSFTSIWIPYCIKLTSNGGTVDEKCFS VDEIVQPDPPIALNWTLLNVSLTGIHADIQVRWEAPRNADIQKGWMV LEYELQYKEVNETKWKMMDPILTTSVPVYSLKVDKEYEVRVRSKQR NSGNYGEFSEVLYVTLPQMSQDDDDKSGGGGSGLNDIFEAQKIEWHE HHHHHH |
| **54** | **rGHBP-His (rat GHBP (GH-binding protein)-Avi tag-His tag)** |
| | MDLWRVFLTLALAVSSDMFPGSGATPATLGKASPVLQRINPSLRESSS GKPRFTKCRSPELETFSCYWTEGDDHNLKVPGSIQLYYARRIAHEWT PEWKECPDYVSAGANSCYFNSSYTSIWIPYCIKLTTNGDLLDEKCFTV DEIVQPDPPIGLNWTLLNISLPGIRGDIQVSWQPPPSADVLKGWIILEY EIQYKEVNETKWKTMSPIWSTSVPLYSLRLDKEHEVRVRSRQRSFEK YSEFSEVLRVTFPQMDTDDDDKSGGGGSGLNDIFEAQKIEWHEHHHH HH |
| **55** | **cGHBP-His (cynomolgus GHBP (GH-binding protein)-Avi tag-His tag)** |
| | MDLWQLLLTLALAGSSDAFSGSEPTAAILSRASWSLQSVNPGLKTNSS KEPKFTKCRSPERETFSCHWTDAVHHGLKSLGPIQLFYTRRNIQEQT QEWKECPDYVSAGENSCYFNSSFTSVWIPYCIKLTSNGDTVDGKCFSV DEIVQPDPPIALNWTLLNVSLTGIHADIQVRWEAPPNADIQKGWMVL EYELQYKEVNETKWKMMDPILSTSVPVYSLKVDKEYEVRVRSKRRN SRNYGEFSEVLYVTLPQMNQDDDDKSGGGGSGLNDIFEAQKIEWHE HHHHHH |
| **56** | **hGHBP-mFc (human GHBP (GH-binding protein)-mIgG1Fc-Avi tag)** |
| | MDLWQLLLTLALAGSSDAFSGSEATAAILSRAPWSLQSVNPGLKTNSS KEPKFTKCRSPERETFSCHWTDEVHHGTKNLGPIQLFYTRRNTQEW TQEWKECPDYVSAGENSCYFNSSFTSIWIPYCIKLTSNGGTVDEKCFS VDEIVQPDPPIALNWTLLNVSLTGIHADIQVRWEAPRNADIQKGWMV LEYELQYKEVNETKWKMMDPILTTSVPVYSLKVDKEYEVRVRSKQR NSGNYGEFSEVLYVTLPQMSQDDDDKSGGGGSVPRDCGCKPCICTVP EVSSVFIFPPKPKDVLTITLTPKVTCVVVDISKDDPEVQFSWFVDDVEV HTAQTQPREEQFNSTFRSVSELPIMHQDWLNGKEFKCRVNSAAFPAPI EKTISKTKGRPKAPQVYTIPPPKEQMAKDKVSLTCMITDFFPEDITVE WQWNGQPAENYKNTQPIMDTDGSYFVYSKLNVQKSNWEAGNTFTC SVLHEGLHNHHTEKSLSHSPGKSGGGGSGLNDIFEAQKIEWHE |
| **57** | **rGHBP-mFc (rat GHBP (GH-binding protein)-mIgG1Fc-Avi tag)** |
| | MDLWRVFLTLALAVSSDMFPGSGATPATLGKASPVLQRINPSLRESSS GKPRFTKCRSPELETFSCYWTEGDDHNLKVPGSIQLYYARRIAHEWT PEWKECPDYVSAGANSCYFNSSYTSIWIPYCIKLTTNGDLLDEKCFTV DEIVQPDPPIGLNWTLLNISLPGIRGDIQVSWQPPPSADVLKGWIILEY EIQYKEVNETKWKTMSPIWSTSVPLYSLRLDKEHEVRVRSRQRSFEK YSEFSEVLRVTFPQMDTDDDDKSGGGGSVPRDCGCKPCICTVPEVSSV FIFPPKPKDVLTITLTPKVTCVVVDISKDDPEVQFSWFVDDVEVHTAQ TQPREEQFNSTFRSVSELPIMHQDWLNGKEFKCRVNSAAFPAPIEKTI SKTKGRPKAPQVYTIPPPKEQMAKDKVSLTCMITDFFPEDITVEWQW NGQPAENYKNTQPIMDTDGSYFVYSKLNVQKSNWEAGNTFTCSVLH EGLHNHHTEKSLSHSPGKSGGGGSGLNDIFEAQKIEWHE |
| **58** | **cGHBP-mFc (cynomolgus monkey GHBP (GH-binding protein)-mIgG1Fc-Avi tag)** |
| | MDLWQLLLTLALAGSSDAFSGSEPTAAILSRASWSLQSVNPGLKTNSS KEPKFTKCRSPERETFSCHWTDAVHHGLKSLGPIQLFYTRRNIQEQT QEWKECPDYVSAGENSCYFNSSFTSVWIPYCIKLTSNGDTVDGKCFSV DEIVQPDPPIALNWTLLNVSLTGIHADIQVRWEAPPNADIQKGWMVL EYELQYKEVNETKWKMMDPILSTSVPVYSLKVDKEYEVRVRSKRRN SRNYGEFSEVLYVTLPQMNQDDDDKSGGGGSVPRDCGCKPCICTVPE VSSVFIFPPKPKDVLTITLTPKVTCVVVDISKDDPEVQFSWFVDDVEVH TAQTQPREEQFNSTFRSVSELPIMHQDWLNGKEFKCRVNSAAFPAPIE KTISKTKGRPKAPQVYTIPPPKEQMAKDKVSLTCMITDFFPEDITVEW QWNGQPAENYKNTQPIMDTDGSYFVYSKLNVQKSNWEAGNTFTCSV LHEGLHNHHTEKSLSHSPGKSGGGGSGLNDIFEAQKIEWHE |
| **59** | **GX-H9 (GH-Fc)** |
| | FPTIPLSRLFDNAMLRAHRLHQLAFDTYQEFEEAYIPKEQKYSFLQNP QTSLCFSESIPTPSNREETQQKSNLELLRISLLLIQSWLEPVQFLRSVFA NSLVYGASDSNVYDLLKDLEEGIQTLMGRLEDGSPRTGQIFKQTYSK FDTNSHNDDALLKNYGLLYCFRKDMDKVETFLRIVQCRSVEGSCGFR NTGRGGEEKKKEKEKEEQEERETKTPECPSHTQPLGVFLFPPKPKDT LMISRTPEVTCVVVDVSQEDPEVQFNWYVDGVEVHNAKTKPREEQF NSTYRVVSVLTVLHQDWLNGKEYKCKVSNKGLPSSIEKTISKAKGQP REPQVYTLPPSQEEMTKNQVSLTCLVKGFYPSDIAVEWESNGQPENN YKTTPPVLDSDGSFFLYSRLTVDKSRWQEGNVFSCSVMHEALHNHYT QKSLSLSLGK |
| **60** | **GH (Human GH)** |
| | |
| **61** | **P15164 VHH** |
| | QLQLVESGGGVVQPGGSLTLSCAASGFTFINYAMGWYRQAPGRERE LVAAITSLGGHTTYADSVKGRFTISRDNAKSTVYLQMNSLKPEDTAV YYCHAAQYYREVLGGQYDYRGQGTQVTVSS |
| **62** | **hIgG2 Hinge region** |
| | **ERKCCVECPPCP** |
| **63** | **hIgG1 Hinge region** |
| | **EPKSCDKTHTCPPCP** |
| **64** | **hIgG4 Hinge region** |
| | **ESKYGPPCPSCP** |
| **65** | **hIgG1 Fc (T366Y, Δ447K) region** |
| | APELLGGPSVFLFPPKPKDTLMISRTPEVTCVVVDVSHEDPEVKFNWY VDGVEVHNAKTKPREEQYNSTYRVVSVLTVLHQDWLNGKEYKCKVS NKALPAPIEKTISKAKGQPREPQVYTLPPSRDELTKNQVSLYCLVKGF YPSDIAVEWESNGQPENNYKTTPPVLDSDGSFFLYSKLTVDKSRWQQ GNVFSCSVMHEALHNHYTQKSLSLSPG |
| **66** | **hIgG2 Hinge+hIgG1 Fc (T366Y, Δ447K)** |
| | ERKCCVECPPCPAPELLGGPSVFLFPPKPKDTLMISRTPEVTCVVVDV SHEDPEVKFNWYVDGVEVHNAKTKPREEQYNSTYRVVSVLTVLHQD WLNGKEYKCKVSNKALPAPIEKTISKAKGQPREPQVYTLPPSRDELT KNQVSLYCLVKGFYPSDIAVEWESNGQPENNYKTTPPVLDSDGSFFLY SKLTVDKSRWQQGNVFSCSVMHEALHNHYTQKSLSLSPG |
| **67** | **SZ0022 VHH** |
| | QLQLVESGGGVVQPGGSLRLSCAASGFTFINYAMGWYRQAPGRERE LVAAITSLGGHTTYADSVKGRFTISRDNAKSTVYLQMNSLRPEDTAVY YCHAAQYYREVLGGQYDYRGQGTLVTVSS |
| **68** | **SZ0023 VHH** |
| | EVQLVESGGGLVQPGGSLRLSCAASGFTFINYAMGWYRQAPGRERE LVAAITSLGGHTTYADSVKGRFTISRDNAKSTVYLQMNSLRPEDTAVY YCHAAQYYREVLGGQYDYRGQGTLVTVSS |
| **69** | **SZ0024 VHH** |
| | QLQLVESGGGVVQPGGSLRLSCAASGFTFINYAMGWFRQAPGRERE LVAAITSLGGHTTYADSVKGRFTISRDNAKSTVYLQMNSLRPEDTAVY YCHAAQYYREVLGGQYDYRGQGTLVTVSS |
| **70** | **SZ0025 VHH** |
| | QLQLVESGGGVVQPGGSLRLSCAASGFTFINYAMGWYRQAPGKERE LVAAITSLGGHTTYADSVKGRFTISRDNAKSTVYLQMNSLRPEDTAVY YCHAAQYYREVLGGQYDYRGQGTLVTVSS |
| **71** | **SZ0026 VHH** |
| | QLQLVESGGGVVQPGGSLRLSCAASGFTFINYAMGWYRQAPGRERE SVAAITSLGGHTTYADSVKGRFTISRDNAKSTVYLQMNSLRPEDTAVY YCHAAQYYREVLGGQYDYRGQGTLVTVSS |
| **72** | **SZ0027 VHH** |
| | QLQLVESGGGVVQPGGSLRLSCAASGFTFINYAMGWYRQAPGRERE LVAAITSLGGSTTYADSVKGRFTISRDNAKSTVYLQMNSLRPEDTAVY YCHAAQYYREVLGGQYDYRGQGTLVTVSS |
| **73** | **SZ0028 VHH** |
| | QLQLVESGGGVVQPGGSLRLSCAASGFTFINYAMGWYRQAPGRERE LVAAITSLGGHTYYADSVKGRFTISRDNAKSTVYLQMNSLRPEDTAV YYCHAAQYYREVLGGQYDYRGQGTLVTVSS |
| **74** | **SZ0029 VHH** |
| | QLQLVESGGGVVQPGGSLRLSCAASGFTFINYAMGWYRQAPGRERE LVAAITSLGGHTTYADSVKGRFTISRDNSKNTLYLQMNSLRPEDTAVY YCHAAQYYREVLGGQYDYRGQGTLVTVSS |
| **75** | **SZ0030 VHH** |
| | QLQLVESGGGVVQPGGSLRLSCAASGFTFINYAMGWYRQAPGRERE LVAAITSLGGHTTYADSVKGRFTISRDNAKSTVYLQMNSLRAEDTAV YYCHAAQYYREVLGGQYDYRGQGTLVTVSS |
| **76** | **IgG1 Fc (LALA) sequence** |
| | APEAAGGPSVFLFPPKPKDTLMISRTPEVTCVVVDVSHEDPEVKFNW YVDGVEVHNAKTKPREEQYNSTYRVVSVLTVLHQDWLNGKEYKCK VSNKALPAPIEKTISKAKGQPREPQVYTLPPSRDELTKNQVSLTCLVK GFYPSDIAVEWESNGQPENNYKTTPPVLDSDGSFFLYSKLTVDKSRW QQGNVFSCSVMHEALHNHYTQKSLSLSPGK |
| **77** | **hIgG2 Hinge+hIgG1 Fc (LALA)** |
| | ERKCCVECPPCPAPEAAGGPSVFLFPPKPKDTLMISRTPEVTCVVVDV SHEDPEVKFNWYVDGVEVHNAKTKPREEQYNSTYRVVSVLTVLHQD WLNGKEYKCKVSNKALPAPIEKTISKAKGQPREPQVYTLPPSRDELT KNQVSLTCLVKGFYPSDIAVEWESNGQPENNYKTTPPVLDSDGSFFLY SKLTVDKSRWQQGNVFSCSVMHEALHNHYTQKSLSLSPGK |
| **78** | **IgG4 hinge (S228P) + IgG4Fc (L235E)** |
| | ESKYGPPCPPCPAPEFEGGPSVFLFPPKPKDTLMISRTPEVTCVVVDVS QEDPEVQFNWYVDGVEVHNAKTKPREEQFNSTYRVVSVLTVLHQDW LNGKEYKCKVSNKGLPSSIEKTISKAKGQPREPQVYTLPPSQEEMTK NQVSLTCLVKGFYPSDIAVEWESNGQPENNYKTTPPVLDSDGSFFLYS RLTVDKSRWQEGNVFSCSVMHEALHNHYTQKSLSLSLGK |
| **79** | **IgG4Fc (L235E)** |
| | APEFEGGPSVFLFPPKPKDTLMISRTPEVTCVVVDVSQEDPEVQFNWY VDGVEVHNAKTKPREEQFNSTYRVVSVLTVLHQDWLNGKEYKCKVS NKGLPSSIEKTISKAKGQPREPQVYTLPPSQEEMTKNQVSLTCLVKGF YPSDIAVEWESNGQPENNYKTTPPVLDSDGSFFLYSRLTVDKSRWQE GNVFSCSVMHEALHNHYTQKSLSLSLGK |
| **80** | **Artificially designed hinge region sequence** |
| | **ERKSSVECPPCP** |
| **81** | **Artificially designed hinge region sequence** |
| | **ERKCPPCP** |
| **82** | **Artificially designed hinge region sequence** |
| | **EPKSSDKTHTCPPCP** |
| **83** | **Artificially designed hinge region sequence** |
| | **DKTHTCPPCP** |
| **84** | **Artificially designed hinge region sequence** |
| | **KTHTCPPCP** |
| **85** | **Artificially designed hinge region sequence** |
| | **THTCPPCP** |
| **86** | **Artificially designed hinge region sequence** |
| | **HTCPPCP** |
| **87** | **Artificially designed hinge region sequence** |
| | **TCPPCP** |
| **88** | **Artificially designed hinge region sequence** |
| | **CPPCP** |
| **89** | **Artificially designed hinge region sequence** |
| | **ESKYGPPCPPCP** |
| **90** | **P15164-1-12 VHH** |
| | QLQLVESGGGVVQPGGSLRLSCAASGFTFINYAMGWYRQAPGRERE LVAAITSLGGSTTYADSVKGRFTISRDNSKNTLYLQMNSLRAEDTAVY YCHAAQYYREVLGGQYDYRGQGTLVTVSS |
| **91** | **P15164-1-13 VHH** |
| | QLQLVESGGGVVQPGGSLRLSCAASGFTFINYAMGWYRQAPGRERE LVAAITSLGGHTYYADSVKGRFTISRDNSKNTLYLQMNSLRAEDTAV YYCHAAQYYREVLGGQYDYRGQGTLVTVSS |
| **92** | **P15164-1-14 VHH** |
| | QLQLVESGGGVVQPGGSLRLSCAASGFTFINYAMGWYRQAPGRERE LVAAITSLGGHTTYADSVKGRFTISRDNSKNTLYLQMNSLRAEDTAV YYCHAAQYYREVLGGQYDYRGQGTLVTVSS |
| **93** | **IgG1Fc(AA,YTE)** |
| | APEAAGGPSVFLFPPKPKDTLYITREPEVTCVVVDVSHEDPEVKFNWY VDGVEVHNAKTKPREEQYNSTYRVVSVLTVLHQDWLNGKEYKCKVS NKALPAPIEKTISKAKGQPREPQVYTLPPSRDELTKNQVSLTCLVKGF YPSDIAVEWESNGQPENNYKTTPPVLDSDGSFFLYSKLTVDKSRWQQ GNVFSCSVMHEALHNHYTQKSLSLSPGK |
| **94** | **SZ0055 (VHH-Hinge-IgG1Fc(AA))** |
| | QLQLVESGGGVVQPGGSLRLSCAASGFTFINYAMGWYRQAPGRERE LVAAITSLGGHTTYADSVKGRFTISRDNSKNTLYLQMNSLRAEDTAV YYCHAAQYYREVLGGQYDYRGQGTLVTVSSHTCPPCPAPEAAGGPS VFLFPPKPKDTLMISRTPEVTCVVVDVSHEDPEVKFNWYVDGVEVHN AKTKPREEQYNSTYRVVSVLTVLHQDWLNGKEYKCKVSNKALPAPI EKTISKAKGQPREPQVYTLPPSRDELTKNQVSLTCLVKGFYPSDIAVE WESNGQPENNYKTTPPVLDSDGSFFLYSKLTVDKSRWQQGNVFSCSV MHEALHNHYTQKSLSLSPGK |
| **95** | **SZ0056 (VHH-Hinge-IgG1Fc(AA))** |
| | QLQLVESGGGVVQPGGSLRLSCAASGFTFINYAMGWYRQAPGRERE LVAAITSLGGHTTYADSVKGRFTISRDNSKNTLYLQMNSLRAEDTAV YYCHAAQYYREVLGGQYDYRGQGTLVTVSSTCPPCPAPEAAGGPSVF LFPPKPKDTLMISRTPEVTCVVVDVSHEDPEVKFNWYVDGVEVHNAK TKPREEQYNSTYRVVSVLTVLHQDWLNGKEYKCKVSNKALPAPIEKT ISKAKGQPREPQVYTLPPSRDELTKNQVSLTCLVKGFYPSDIAVEWES NGQPENNYKTTPPVLDSDGSFFLYSKLTVDKSRWQQGNVFSCSVMHE ALHNHYTQKSLSLSPGK |
| **96** | **SZ0112 (SZ0055-YTE) (VHH-Hinge- IgG1Fc(AA,YTE)** |
| | QLQLVESGGGVVQPGGSLRLSCAASGFTFINYAMGWYRQAPGRERE LVAAITSLGGHTTYADSVKGRFTISRDNSKNTLYLQMNSLRAEDTAV YYCHAAQYYREVLGGQYDYRGQGTLVTVSSHTCPPCPAPEAAGGPS VFLFPPKPKDTLYITREPEVTCVVVDVSHEDPEVKFNWYVDGVEVHN AKTKPREEQYNSTYRVVSVLTVLHQDWLNGKEYKCKVSNKALPAPI EKTISKAKGQPREPQVYTLPPSRDELTKNQVSLTCLVKGFYPSDIAVE WESNGQPENNYKTTPPVLDSDGSFFLYSKLTVDKSRWQQGNVFSCSV MHEALHNHYTQKSLSLSPGK |
| **97** | **SZ0113 (SZ0056-YTE) (VHH-Hinge- IgG1Fc(AA,YTE)** |
| | QLQLVESGGGVVQPGGSLRLSCAASGFTFINYAMGWYRQAPGRERE LVAAITSLGGHTTYADSVKGRFTISRDNSKNTLYLQMNSLRAEDTAV YYCHAAQYYREVLGGQYDYRGQGTLVTVSSTCPPCPAPEAAGGPSVF LFPPKPKDTLYITREPEVTCVVVDVSHEDPEVKFNWYVDGVEVHNAK TKPREEQYNSTYRVVSVLTVLHQDWLNGKEYKCKVSNKALPAPIEKT ISKAKGQPREPQVYTLPPSRDELTKNQVSLTCLVKGFYPSDIAVEWES NGQPENNYKTTPPVLDSDGSFFLYSKLTVDKSRWQQGNVFSCSVMHE ALHNHYTQKSLSLSPGK |
| **98** | **IgG4 Hinge+Fc** |
| | ESKYGPPCPSCPAPEFLGGPSVFLFPPKPKDTLMISRTPEVTCVVVDVS QEDPEVQFNWYVDGVEVHNAKTKPREEQFNSTYRVVSVLTVLHQDW LNGKEYKCKVSNKGLPSSIEKTISKAKGQPREPQVYTLPPSQEEMTK NQVSLTCLVKGFYPSDIAVEWESNGQPENNYKTTPPVLDSDGSFFLYS RLTVDKSRWQEGNVFSCSVMHEALHNHYTQKSLSLSLGK |
| **99** | **IgG4 Fc** |
| | APEFLGGPSVFLFPPKPKDTLMISRTPEVTCVVVDVSQEDPEVQFNWY VDGVEVHNAKTKPREEQFNSTYRVVSVLTVLHQDWLNGKEYKCKVS NKGLPSSIEKTISKAKGQPREPQVYTLPPSQEEMTKNQVSLTCLVKGF YPSDIAVEWESNGQPENNYKTTPPVLDSDGSFFLYSRLTVDKSRWQE GNVFSCSVMHEALHNHYTQKSLSLSLGK |
| **100** | **Formula I** |
| | **Z-CPPCP** |
| **101** | **Formula II** |
| | **X₁X₂X₃X₄X₅X₆X₇X₈X₉X₁₀CPPCP** |
| **102** | **Formula III** |
| | **XᵢXᵢᵢXᵢᵢᵢXᵢᵥXᵥXᵥᵢXᵥᵢᵢCPPCP** |
| **103** | **P15164-1 (VHH-Hinge-IgG1Fc(T366Y, Δ447K))** |
| | QLQLVESGGGVVQPGGSLTLSCAASGFTFINYAMGWYRQAPGRERE LVAAITSLGGHTTYADSVKGRFTISRDNAKSTVYLQMNSLKPEDTAV YYCHAAQYYREVLGGQYDYRGQGTQVTVSSERKCCVECPPCPAPEL LGGPSVFLFPPKPKDTLMISRTPEVTCVVVDVSHEDPEVKFNWYVDG VEVHNAKTKPREEQYNSTYRVVSVLTVLHQDWLNGKEYKCKVSNKA LPAPIEKTISKAKGQPREPQVYTLPPSRDELTKNQVSLYCLVKGFYPS DIAVEWESNGQPENNYKTTPPVLDSDGSFFLYSKLTVDKSRWQQGNV FSCSVMHEALHNHYTQKSLSLSPG |
| **104** | **SZ0022 (VHH-Hinge-IgG1Fc(AA))** |
| | QLQLVESGGGVVQPGGSLRLSCAASGFTFINYAMGWYRQAPGRERE LVAAITSLGGHTTYADSVKGRFTISRDNAKSTVYLQMNSLRPEDTAVY YCHAAQYYREVLGGQYDYRGQGTLVTVSSERKCCVECPPCPAPEAA GGPSVFLFPPKPKDTLMISRTPEVTCVVVDVSHEDPEVKFNWYVDGV EVHNAKTKPREEQYNSTYRVVSVLTVLHQDWLNGKEYKCKVSNKAL PAPIEKTISKAKGQPREPQVYTLPPSRDELTKNQVSLTCLVKGFYPSDI AVEWESNGQPENNYKTTPPVLDSDGSFFLYSKLTVDKSRWQQGNVFS CSVMHEALHNHYTQKSLSLSPGK |
| **105** | **SZ0023 (VHH-Hinge-IgG1Fc(AA))** |
| | EVQLVESGGGLVQPGGSLRLSCAASGFTFINYAMGWYRQAPGRERE LVAAITSLGGHTTYADSVKGRFTISRDNAKSTVYLQMNSLRPEDTAVY YCHAAQYYREVLGGQYDYRGQGTLVTVSSERKCCVECPPCPAPEAA GGPSVFLFPPKPKDTLMISRTPEVTCVVVDVSHEDPEVKFNWYVDGV EVHNAKTKPREEQYNSTYRVVSVLTVLHQDWLNGKEYKCKVSNKAL PAPIEKTISKAKGQPREPQVYTLPPSRDELTKNQVSLTCLVKGFYPSDI AVEWESNGQPENNYKTTPPVLDSDGSFFLYSKLTVDKSRWQQGNVFS CSVMHEALHNHYTQKSLSLSPGK |
| **106** | **SZ0024 (VHH-Hinge-IgG1Fc(AA))** |
| | QLQLVESGGGVVQPGGSLRLSCAASGFTFINYAMGWFRQAPGRERE LVAAITSLGGHTTYADSVKGRFTISRDNAKSTVYLQMNSLRPEDTAVY YCHAAQYYREVLGGQYDYRGQGTLVTVSSERKCCVECPPCPAPEAA GGPSVFLFPPKPKDTLMISRTPEVTCVVVDVSHEDPEVKFNWYVDGV EVHNAKTKPREEQYNSTYRVVSVLTVLHQDWLNGKEYKCKVSNKAL PAPIEKTISKAKGQPREPQVYTLPPSRDELTKNQVSLTCLVKGFYPSDI AVEWESNGQPENNYKTTPPVLDSDGSFFLYSKLTVDKSRWQQGNVFS CSVMHEALHNHYTQKSLSLSPGK |
| **107** | **SZ0025 (VHH-Hinge-IgG1Fc(AA))** |
| | QLQLVESGGGVVQPGGSLRLSCAASGFTFINYAMGWYRQAPGKERE LVAAITSLGGHTTYADSVKGRFTISRDNAKSTVYLQMNSLRPEDTAVY YCHAAQYYREVLGGQYDYRGQGTLVTVSSERKCCVECPPCPAPEAA GGPSVFLFPPKPKDTLMISRTPEVTCVVVDVSHEDPEVKFNWYVDGV EVHNAKTKPREEQYNSTYRVVSVLTVLHQDWLNGKEYKCKVSNKAL PAPIEKTISKAKGQPREPQVYTLPPSRDELTKNQVSLTCLVKGFYPSDI AVEWESNGQPENNYKTTPPVLDSDGSFFLYSKLTVDKSRWQQGNVFS CSVMHEALHNHYTQKSLSLSPGK |
| **108** | **SZ0026 (VHH-Hinge-IgG1Fc(AA))** |
| | QLQLVESGGGVVQPGGSLRLSCAASGFTFINYAMGWYRQAPGRERE SVAAITSLGGHTTYADSVKGRFTISRDNAKSTVYLQMNSLRPEDTAVY YCHAAQYYREVLGGQYDYRGQGTLVTVSSERKCCVECPPCPAPEAA GGPSVFLFPPKPKDTLMISRTPEVTCVVVDVSHEDPEVKFNWYVDGV EVHNAKTKPREEQYNSTYRVVSVLTVLHQDWLNGKEYKCKVSNKAL PAPIEKTISKAKGQPREPQVYTLPPSRDELTKNQVSLTCLVKGFYPSDI AVEWESNGQPENNYKTTPPVLDSDGSFFLYSKLTVDKSRWQQGNVFS CSVMHEALHNHYTQKSLSLSPGK |
| **109** | **SZ0027 (VHH-Hinge-IgG1Fc(AA))** |
| | QLQLVESGGGVVQPGGSLRLSCAASGFTFINYAMGWYRQAPGRERE LVAAITSLGGSTTYADSVKGRFTISRDNAKSTVYLQMNSLRPEDTAVY YCHAAQYYREVLGGQYDYRGQGTLVTVSSERKCCVECPPCPAPEAA GGPSVFLFPPKPKDTLMISRTPEVTCVVVDVSHEDPEVKFNWYVDGV EVHNAKTKPREEQYNSTYRVVSVLTVLHQDWLNGKEYKCKVSNKAL PAPIEKTISKAKGQPREPQVYTLPPSRDELTKNQVSLTCLVKGFYPSDI AVEWESNGQPENNYKTTPPVLDSDGSFFLYSKLTVDKSRWQQGNVFS CSVMHEALHNHYTQKSLSLSPGK |
| **110** | **SZ0028 (VHH-Hinge-IgG1Fc(AA))** |
| | QLQLVESGGGVVQPGGSLRLSCAASGFTFINYAMGWYRQAPGRERE LVAAITSLGGHTYYADSVKGRFTISRDNAKSTVYLQMNSLRPEDTAV YYCHAAQYYREVLGGQYDYRGQGTLVTVSSERKCCVECPPCPAPEA AGGPSVFLFPPKPKDTLMISRTPEVTCVVVDVSHEDPEVKFNWYVDG VEVHNAKTKPREEQYNSTYRVVSVLTVLHQDWLNGKEYKCKVSNKA LPAPIEKTISKAKGQPREPQVYTLPPSRDELTKNQVSLTCLVKGFYPS DIAVEWESNGQPENNYKTTPPVLDSDGSFFLYSKLTVDKSRWQQGNV FSCSVMHEALHNHYTQKSLSLSPGK |
| **111** | **SZ0029 (VHH-Hinge-IgG1Fc(AA))** |
| | QLQLVESGGGVVQPGGSLRLSCAASGFTFINYAMGWYRQAPGRERE LVAAITSLGGHTTYADSVKGRFTISRDNSKNTLYLQMNSLRPEDTAVY YCHAAQYYREVLGGQYDYRGQGTLVTVSSERKCCVECPPCPAPEAA GGPSVFLFPPKPKDTLMISRTPEVTCVVVDVSHEDPEVKFNWYVDGV EVHNAKTKPREEQYNSTYRVVSVLTVLHQDWLNGKEYKCKVSNKAL PAPIEKTISKAKGQPREPQVYTLPPSRDELTKNQVSLTCLVKGFYPSDI AVEWESNGQPENNYKTTPPVLDSDGSFFLYSKLTVDKSRWQQGNVFS CSVMHEALHNHYTQKSLSLSPGK |
| **112** | **SZ0030 (VHH-Hinge-IgG1Fc(AA))** |
| | QLQLVESGGGVVQPGGSLRLSCAASGFTFINYAMGWYRQAPGRERE LVAAITSLGGHTTYADSVKGRFTISRDNAKSTVYLQMNSLRAEDTAV YYCHAAQYYREVLGGQYDYRGQGTLVTVSSERKCCVECPPCPAPEA AGGPSVFLFPPKPKDTLMISRTPEVTCVVVDVSHEDPEVKFNWYVDG VEVHNAKTKPREEQYNSTYRVVSVLTVLHQDWLNGKEYKCKVSNKA LPAPIEKTISKAKGQPREPQVYTLPPSRDELTKNQVSLTCLVKGFYPS DIAVEWESNGQPENNYKTTPPVLDSDGSFFLYSKLTVDKSRWQQGNV FSCSVMHEALHNHYTQKSLSLSPGK |
| **113** | **SZ0035 (VHH-Hinge-IgG1Fc(AA))** |
| | QLQLVESGGGVVQPGGSLRLSCAASGFTFINYAMGWYRQAPGRERE LVAAITSLGGHTTYADSVKGRFTISRDNAKSTVYLQMNSLRPEDTAVY YCHAAQYYREVLGGQYDYRGQGTLVTVSSERKSSVECPPCPAPEAAG GPSVFLFPPKPKDTLMISRTPEVTCVVVDVSHEDPEVKFNWYVDGVE VHNAKTKPREEQYNSTYRVVSVLTVLHQDWLNGKEYKCKVSNKALP APIEKTISKAKGQPREPQVYTLPPSRDELTKNQVSLTCLVKGFYPSDIA VEWESNGQPENNYKTTPPVLDSDGSFFLYSKLTVDKSRWQQGNVFSC SVMHEALHNHYTQKSLSLSPGK |
| **114** | **SZ0036 (VHH-Hinge-IgG1Fc(AA))** |
| | QLQLVESGGGVVQPGGSLRLSCAASGFTFINYAMGWYRQAPGRERE LVAAITSLGGHTTYADSVKGRFTISRDNAKSTVYLQMNSLRPEDTAVY YCHAAQYYREVLGGQYDYRGQGTLVTVSSERKCPPCPAPEAAGGPS VFLFPPKPKDTLMISRTPEVTCVVVDVSHEDPEVKFNWYVDGVEVHN AKTKPREEQYNSTYRVVSVLTVLHQDWLNGKEYKCKVSNKALPAPI EKTISKAKGQPREPQVYTLPPSRDELTKNQVSLTCLVKGFYPSDIAVE WESNGQPENNYKTTPPVLDSDGSFFLYSKLTVDKSRWQQGNVFSCSV MHEALHNHYTQKSLSLSPGK |
| **115** | **SZ0037 (VHH-Hinge-IgG1Fc(AA))** |
| | QLQLVESGGGVVQPGGSLRLSCAASGFTFINYAMGWYRQAPGRERE LVAAITSLGGHTTYADSVKGRFTISRDNAKSTVYLQMNSLRPEDTAVY YCHAAQYYREVLGGQYDYRGQGTLVTVSSEPKSSDKTHTCPPCPAPE AAGGPSVFLFPPKPKDTLMISRTPEVTCVVVDVSHEDPEVKFNWYVD GVEVHNAKTKPREEQYNSTYRVVSVLTVLHQDWLNGKEYKCKVSN KALPAPIEKTISKAKGQPREPQVYTLPPSRDELTKNQVSLTCLVKGFY PSDIAVEWESNGQPENNYKTTPPVLDSDGSFFLYSKLTVDKSRWQQG NVFSCSVMHEALHNHYTQKSLSLSPGK |
| **116** | **SZ0038 (VHH-Hinge-IgG1Fc(AA))** |
| | QLQLVESGGGVVQPGGSLRLSCAASGFTFINYAMGWYRQAPGRERE LVAAITSLGGHTTYADSVKGRFTISRDNAKSTVYLQMNSLRPEDTAVY YCHAAQYYREVLGGQYDYRGQGTLVTVSSDKTHTCPPCPAPEAAGG PSVFLFPPKPKDTLMISRTPEVTCVVVDVSHEDPEVKFNWYVDGVEV HNAKTKPREEQYNSTYRVVSVLTVLHQDWLNGKEYKCKVSNKALPA PIEKTISKAKGQPREPQVYTLPPSRDELTKNQVSLTCLVKGFYPSDIAV EWESNGQPENNYKTTPPVLDSDGSFFLYSKLTVDKSRWQQGNVFSCS VMHEALHNHYTQKSLSLSPGK |
| **117** | **SZ0039 (VHH-Hinge-IgG1Fc(AA))** |
| | QLQLVESGGGVVQPGGSLRLSCAASGFTFINYAMGWYRQAPGRERE LVAAITSLGGHTTYADSVKGRFTISRDNAKSTVYLQMNSLRPEDTAVY YCHAAQYYREVLGGQYDYRGQGTLVTVSSKTHTCPPCPAPEAAGGP SVFLFPPKPKDTLMISRTPEVTCVVVDVSHEDPEVKFNWYVDGVEVH NAKTKPREEQYNSTYRVVSVLTVLHQDWLNGKEYKCKVSNKALPAP IEKTISKAKGQPREPQVYTLPPSRDELTKNQVSLTCLVKGFYPSDIAVE WESNGQPENNYKTTPPVLDSDGSFFLYSKLTVDKSRWQQGNVFSCSV MHEALHNHYTQKSLSLSPGK |
| **118** | **SZ0040 (VHH-Hinge-IgG1Fc(AA))** |
| | QLQLVESGGGVVQPGGSLRLSCAASGFTFINYAMGWYRQAPGRERE LVAAITSLGGHTTYADSVKGRFTISRDNAKSTVYLQMNSLRPEDTAVY YCHAAQYYREVLGGQYDYRGQGTLVTVSSTHTCPPCPAPEAAGGPS VFLFPPKPKDTLMISRTPEVTCVVVDVSHEDPEVKFNWYVDGVEVHN AKTKPREEQYNSTYRVVSVLTVLHQDWLNGKEYKCKVSNKALPAPI EKTISKAKGQPREPQVYTLPPSRDELTKNQVSLTCLVKGFYPSDIAVE WESNGQPENNYKTTPPVLDSDGSFFLYSKLTVDKSRWQQGNVFSCSV MHEALHNHYTQKSLSLSPGK |
| **119** | **SZ0041 (VHH-Hinge-IgG1Fc(AA))** |
| | QLQLVESGGGVVQPGGSLRLSCAASGFTFINYAMGWYRQAPGRERE LVAAITSLGGHTTYADSVKGRFTISRDNAKSTVYLQMNSLRPEDTAVY YCHAAQYYREVLGGQYDYRGQGTLVTVSSHTCPPCPAPEAAGGPSV FLFPPKPKDTLMISRTPEVTCVVVDVSHEDPEVKFNWYVDGVEVHNA KTKPREEQYNSTYRVVSVLTVLHQDWLNGKEYKCKVSNKALPAPIE KTISKAKGQPREPQVYTLPPSRDELTKNQVSLTCLVKGFYPSDIAVEW ESNGQPENNYKTTPPVLDSDGSFFLYSKLTVDKSRWQQGNVFSCSVM HEALHNHYTQKSLSLSPGK |
| **120** | **SZ0042 (VHH-Hinge-IgG1Fc(AA))** |
| | QLQLVESGGGVVQPGGSLRLSCAASGFTFINYAMGWYRQAPGRERE LVAAITSLGGHTTYADSVKGRFTISRDNAKSTVYLQMNSLRPEDTAVY YCHAAQYYREVLGGQYDYRGQGTLVTVSSTCPPCPAPEAAGGPSVFL FPPKPKDTLMISRTPEVTCVVVDVSHEDPEVKFNWYVDGVEVHNAKT KPREEQYNSTYRVVSVLTVLHQDWLNGKEYKCKVSNKALPAPIEKTI SKAKGQPREPQVYTLPPSRDELTKNQVSLTCLVKGFYPSDIAVEWES NGQPENNYKTTPPVLDSDGSFFLYSKLTVDKSRWQQGNVFSCSVMHE ALHNHYTQKSLSLSPGK |
| **121** | **SZ0043 (VHH-Hinge-IgG1Fc(AA))** |
| | QLQLVESGGGVVQPGGSLRLSCAASGFTFINYAMGWYRQAPGRERE LVAAITSLGGHTTYADSVKGRFTISRDNAKSTVYLQMNSLRPEDTAVY YCHAAQYYREVLGGQYDYRGQGTLVTVSSCPPCPAPEAAGGPSVFLF PPKPKDTLMISRTPEVTCVVVDVSHEDPEVKFNWYVDGVEVHNAKT KPREEQYNSTYRVVSVLTVLHQDWLNGKEYKCKVSNKALPAPIEKTI SKAKGQPREPQVYTLPPSRDELTKNQVSLTCLVKGFYPSDIAVEWES NGQPENNYKTTPPVLDSDGSFFLYSKLTVDKSRWQQGNVFSCSVMHE ALHNHYTQKSLSLSPGK |
| **122** | **SZ0044 (VHH-Hinge-IgG4Fc(L235E))** |
| | QLQLVESGGGVVQPGGSLRLSCAASGFTFINYAMGWYRQAPGRERE LVAAITSLGGHTTYADSVKGRFTISRDNAKSTVYLQMNSLRPEDTAVY YCHAAQYYREVLGGQYDYRGQGTLVTVSSESKYGPPCPPCPAPEFEG GPSVFLFPPKPKDTLMISRTPEVTCVVVDVSQEDPEVQFNWYVDGVE VHNAKTKPREEQFNSTYRVVSVLTVLHQDWLNGKEYKCKVSNKGLP SSIEKTISKAKGQPREPQVYTLPPSQEEMTKNQVSLTCLVKGFYPSDIA VEWESNGQPENNYKTTPPVLDSDGSFFLYSRLTVDKSRWQEGNVFSC SVMHEALHNHYTQKSLSLSLGK |
| **123** | **SZ0051 (VHH-Hinge-IgG1Fc(AA))** |
| | QLQLVESGGGVVQPGGSLRLSCAASGFTFINYAMGWYRQAPGRERE LVAAITSLGGSTTYADSVKGRFTISRDNSKNTLYLQMNSLRAEDTAVY YCHAAQYYREVLGGQYDYRGQGTLVTVSSHTCPPCPAPEAAGGPSV FLFPPKPKDTLMISRTPEVTCVVVDVSHEDPEVKFNWYVDGVEVHNA KTKPREEQYNSTYRVVSVLTVLHQDWLNGKEYKCKVSNKALPAPIE KTISKAKGQPREPQVYTLPPSRDELTKNQVSLTCLVKGFYPSDIAVEW ESNGQPENNYKTTPPVLDSDGSFFLYSKLTVDKSRWQQGNVFSCSVM HEALHNHYTQKSLSLSPGK |
| **124** | **SZ0052 (VHH-Hinge-IgG1Fc(AA))** |
| | QLQLVESGGGVVQPGGSLRLSCAASGFTFINYAMGWYRQAPGRERE LVAAITSLGGSTTYADSVKGRFTISRDNSKNTLYLQMNSLRAEDTAVY YCHAAQYYREVLGGQYDYRGQGTLVTVSSTCPPCPAPEAAGGPSVFL FPPKPKDTLMISRTPEVTCVVVDVSHEDPEVKFNWYVDGVEVHNAKT KPREEQYNSTYRVVSVLTVLHQDWLNGKEYKCKVSNKALPAPIEKTI SKAKGQPREPQVYTLPPSRDELTKNQVSLTCLVKGFYPSDIAVEWES NGQPENNYKTTPPVLDSDGSFFLYSKLTVDKSRWQQGNVFSCSVMHE ALHNHYTQKSLSLSPGK |
| **125** | **SZ0053 (VHH-Hinge-IgG1Fc(AA))** |
| | QLQLVESGGGVVQPGGSLRLSCAASGFTFINYAMGWYRQAPGRERE LVAAITSLGGHTYYADSVKGRFTISRDNSKNTLYLQMNSLRAEDTAV YYCHAAQYYREVLGGQYDYRGQGTLVTVSSHTCPPCPAPEAAGGPS VFLFPPKPKDTLMISRTPEVTCVVVDVSHEDPEVKFNWYVDGVEVHN AKTKPREEQYNSTYRVVSVLTVLHQDWLNGKEYKCKVSNKALPAPI EKTISKAKGQPREPQVYTLPPSRDELTKNQVSLTCLVKGFYPSDIAVE WESNGQPENNYKTTPPVLDSDGSFFLYSKLTVDKSRWQQGNVFSCSV MHEALHNHYTQKSLSLSPGK |
| **126** | **SZ0054 (VHH-Hinge-IgG1Fc(AA))** |
| | QLQLVESGGGVVQPGGSLRLSCAASGFTFINYAMGWYRQAPGRERE LVAAITSLGGHTYYADSVKGRFTISRDNSKNTLYLQMNSLRAEDTAV YYCHAAQYYREVLGGQYDYRGQGTLVTVSSTCPPCPAPEAAGGPSVF LFPPKPKDTLMISRTPEVTCVVVDVSHEDPEVKFNWYVDGVEVHNAK TKPREEQYNSTYRVVSVLTVLHQDWLNGKEYKCKVSNKALPAPIEKT ISKAKGQPREPQVYTLPPSRDELTKNQVSLTCLVKGFYPSDIAVEWES NGQPENNYKTTPPVLDSDGSFFLYSKLTVDKSRWQQGNVFSCSVMHE ALHNHYTQKSLSLSPGK |

**Table 1.2: Sequence Information of CDRs and FRs of single-domain antibody/heavy-chain antibody molecules defined by various numbering systems**

| **Kabat** | FR1 | CDR1 | FR2 | CDR2 | FR3 | CDR3 | FR4 |
|---|---|---|---|---|---|---|---|
| P15164 | QLQLVESGGGVVQPG GSLTLSCAASGFTFI | NYAMG | WYRQAPGR ERELVA | AITSLGGHTT YADSVKG | RFTISRDNAKSTVYLQ MNSLKPEDTAVYYCH A | AQYYREVLGG QYDY | RGQGTQVTV SS |
| | SEQ ID NO: 1 | SEQ ID NO: 7 | SEQ ID NO: 10 | SEQ ID NO: 22 | SEQ ID NO: 29 | SEQ ID NO: 48 | SEQ ID NO: 50 |
| SZ0022 | QLQLVESGGGVVQPG GSLRLSCAASGFTFI | NYAMG | WYRQAPGR ERELVA | AITSLGGHTT YADSVKG | RFTISRDNAKSTVYLQ MNSLRPEDTAVYYCH A | AQYYREVLGG QYDY | RGQGTLVTV SS |
| | SEQ ID NO: 2 | SEQ ID NO: 7 | SEQ ID NO: 10 | SEQ ID NO: 22 | SEQ ID NO: 30 | SEQ ID NO: 48 | SEQ ID NO: 51 |
| SZ0023 | EVQLVESGGGLVQPG GSLRLSCAASGFTFI | NYAMG | WYRQAPGR ERELVA | AITSLGGHTT YADSVKG | RFTISRDNAKSTVYLQ MNSLRPEDTAVYYCH A | AQYYREVLGG QYDY | RGQGTLVTV SS |
| | SEQ ID NO: 6 | SEQ ID NO: 7 | SEQ ID NO: 10 | SEQ ID NO: 22 | SEQ ID NO: 30 | SEQ ID NO: 48 | SEQ ID NO: 51 |
| SZ0024 | QLQLVESGGGVVQPG GSLRLSCAASGFTFI | NYAMG | WFRQAPGRE RELVA | AITSLGGHTT YADSVKG | RFTISRDNAKSTVYLQ MNSLRPEDTAVYYCH A | AQYYREVLGG QYDY | RGQGTLVTV SS |
| | SEQ ID NO: 2 | SEQ ID NO: 7 | SEQ ID NO: 11 | SEQ ID NO: 22 | SEQ ID NO: 30 | SEQ ID NO: 48 | SEQ ID NO: 51 |
| SZ0025 | QLQLVESGGGVVQPG GSLRLSCAASGFTFI | NYAMG | WYRQAPGK ERELVA | AITSLGGHTT YADSVKG | RFTISRDNAKSTVYLQ MNSLRPEDTAVYYCH A | AQYYREVLGG QYDY | RGQGTLVTV SS |
| | SEQ ID NO: 2 | SEQ ID NO: 7 | SEQ ID NO: 12 | SEQ ID NO: 22 | SEQ ID NO: 30 | SEQ ID NO: 48 | SEQ ID NO: 51 |
| SZ0026 | QLQLVESGGGVVQPG GSLRLSCAASGFTFI | NYAMG | WYRQAPGR ERESVA | AITSLGGHTT YADSVKG | RFTISRDNAKSTVYLQ MNSLRPEDTAVYYCH A | AQYYREVLGG QYDY | RGQGTLVTV SS |
| | SEQ ID NO: 2 | SEQ ID NO: 7 | SEQ ID NO: 13 | SEQ ID NO: 22 | SEQ ID NO: 30 | SEQ ID NO: 48 | SEQ ID NO: 51 |
| SZ0027 | QLQLVESGGGVVQPG GSLRLSCAASGFTFI | NYAMG | WYRQAPGR ERELVA | AITSLGGSTTY ADSVKG | RFTISRDNAKSTVYLQ MNSLRPEDTAVYYCH A | AQYYREVLGG QYDY | RGQGTLVTV SS |
| | SEQ ID NO: 2 | SEQ ID NO: 7 | SEQ ID NO: 10 | SEQ ID NO: 23 | SEQ ID NO: 30 | SEQ ID NO: 48 | SEQ ID NO: 51 |
| SZ0028 | QLQLVESGGGVVQPG GSLRLSCAASGFTFI | NYAMG | WYRQAPGR ERELVA | AITSLGGHTY YADSVKG | RFTISRDNAKSTVYLQ MNSLRPEDTAVYYCH A | AQYYREVLGG QYDY | RGQGTLVTV SS |
| | SEQ ID NO: 2 | SEQ ID NO: 7 | SEQ ID NO: 10 | SEQ ID NO: 24 | SEQ ID NO: 30 | SEQ ID NO: 48 | SEQ ID NO: 51 |
| SZ0029 | QLQLVESGGGVVQPG GSLRLSCAASGFTFI | NYAMG | WYRQAPGR ERELVA | AITSLGGHTT YADSVKG | RFTISRDNSKNTLYLQ MNSLRPEDTAVYYCH A | AQYYREVLGG QYDY | RGQGTLVTV SS |
| | SEQ ID NO: 2 | SEQ ID NO: 7 | SEQ ID NO: 10 | SEQ ID NO: 22 | SEQ ID NO: 31 | SEQ ID NO: 48 | SEQ ID NO: 51 |
| SZ0030 | QLQLVESGGGVVQPG GSLRLSCAASGFTFI | NYAMG | WYRQAPGR ERELVA | AITSLGGHTT YADSVKG | RFTISRDNAKSTVYLQ MNSLRAEDTAVYYCH A | AQYYREVLGG QYDY | RGQGTLVTV SS |
| | SEQ ID NO: 2 | SEQ ID NO: 7 | SEQ ID NO: 10 | SEQ ID NO: 22 | SEQ ID NO: 32 | SEQ ID NO: 48 | SEQ ID NO: 51 |
| P15164-1-12 | QLQLVESGGGVVQPG GSLRLSCAASGFTFI | NYAMG | WYRQAPGR ERELVA | AITSLGGSTTY ADSVKG | RFTISRDNSKNTLYLQ MNSLRAEDTAVYYCH A | AQYYREVLGG QYDY | RGQGTLVTV SS |
| | SEQ ID NO: 2 | SEQ ID NO: 7 | SEQ ID NO: 10 | SEQ ID NO: 23 | SEQ ID NO: 33 | SEQ ID NO: 48 | SEQ ID NO: 51 |
| P15164-1-13 | QLQLVESGGGVVQPG GSLRLSCAASGFTFI | NYAMG | WYRQAPGR ERELVA | AITSLGGHTY YADSVKG | RFTISRDNSKNTLYLQ MNSLRAEDTAVYYCH A | AQYYREVLGG QYDY | RGQGTLVTV SS |
| | SEQ ID NO: 2 | SEQ ID NO: 7 | SEQ ID NO: 10 | SEQ ID NO: 24 | SEQ ID NO: 33 | SEQ ID NO: 48 | SEQ ID NO: 51 |
| P15164-1-14 | QLQLVESGGGVVQPG GSLRLSCAASGFTFI | NYAMG | WYRQAPGR ERELVA | AITSLGGHTT YADSVKG | RFTISRDNSKNTLYLQ MNSLRAEDTAVYYCH A | AQYYREVLGG QYDY | RGQGTLVTV SS |
| | SEQ ID NO: 2 | SEQ ID NO: 7 | SEQ ID NO: 10 | SEQ ID NO: 22 | SEQ ID NO: 33 | SEQ ID NO: 48 | SEQ ID NO: 51 |
| **Chothia** | FR1 | CDR1 | FR2 | CDR2 | FR3 | CDR3 | FR4 |
| P15164 | QLQLVESGGGVVQPG GSLTLSCAAS | GFTFINY | AMGWYRQA PGRERELVA AI | TSLGGH | TTYADSVKGRFTISRD NAKSTVYLQMNSLKPE DTAVYYCHA | AQYYREVLGG QYDY | RGQGTQVTV SS |
| | SEQ ID NO: 3 | SEQ ID NO: 8 | SEQ ID NO: 14 | SEQ ID NO: 25 | SEQ ID NO: 34 | SEQ ID NO: 48 | SEQ ID NO: 50 |
| SZ0022 | QLQLVESGGGVVQPG GSLRLSCAAS | GFTFINY | AMGWYRQA PGRERELVA AI | TSLGGH | TTYADSVKGRFTISRD NAKSTVYLQMNSLRPE DTAVYYCHA | AQYYREVLGG QYDY | RGQGTLVTV SS |
| | SEQ ID NO: 4 | SEQ ID NO: 8 | SEQ ID NO: 14 | SEQ ID NO: 25 | SEQ ID NO: 35 | SEQ ID NO: 48 | SEQ ID NO: 51 |
| SZ0023 | | GFTFINY | AMGWYRQA PGRERELVA AI | TSLGGH | TTYADSVKGRFTISRD NAKSTVYLQMNSLRPE DTAVYYCHA | AQYYREVLGG QYDY | RGQGTLVTV SS |
| | SEQ ID NO: 5 | SEQ ID NO: 8 | SEQ ID NO: 14 | SEQ ID NO: 25 | SEQ ID NO: 35 | SEQ ID NO: 48 | SEQ ID NO: 51 |
| SZ0024 | QLQLVESGGGVVQPG GSLRLSCAAS | GFTFINY | AMGWFRQA PGRERELVA AI | TSLGGH | TTYADSVKGRFTISRD NAKSTVYLQMNSLRPE DTAVYYCHA | AQYYREVLGG QYDY | RGQGTLVTV SS |
| | SEQ ID NO: 4 | SEQ ID NO: 8 | SEQ ID NO: 15 | SEQ ID NO: 25 | SEQ ID NO: 35 | SEQ ID NO: 48 | SEQ ID NO: 51 |
| SZ0025 | QLQLVESGGGVVQPG GSLRLSCAAS | GFTFINY | AMGWYRQA PGKERELVA AI | TSLGGH | TTYADSVKGRFTISRD NAKSTVYLQMNSLRPE DTAVYYCHA | AQYYREVLGG QYDY | RGQGTLVTV SS |
| | SEQ ID NO: 4 | SEQ ID NO: 8 | SEQ ID NO: 16 | SEQ ID NO: 25 | SEQ ID NO: 35 | SEQ ID NO: 48 | SEQ ID NO: 51 |
| SZ0026 | QLQLVESGGGVVQPG GSLRLSCAAS | GFTFINY | AMGWYRQA PGRERESVA AI | TSLGGH | TTYADSVKGRFTISRD NAKSTVYLQMNSLRPE DTAVYYCHA | AQYYREVLGG QYDY | RGQGTLVTV SS |
| | SEQ ID NO: 4 | SEQ ID NO: 8 | SEQ ID NO: 17 | SEQ ID NO: 25 | SEQ ID NO: 35 | SEQ ID NO: 48 | SEQ ID NO: 51 |
| SZ0027 | QLQLVESGGGVVQPG GSLRLSCAAS | GFTFINY | AMGWYRQA PGRERELVA AI | TSLGGS | TTYADSVKGRFTISRD NAKSTVYLQMNSLRPE DTAVYYCHA | AQYYREVLGG QYDY | RGQGTLVTV SS |
| | SEQ ID NO: 4 | SEQ ID NO: 8 | SEQ ID NO: 14 | SEQ ID NO: 26 | SEQ ID NO: 35 | SEQ ID NO: 48 | SEQ ID NO: 51 |
| SZ0028 | QLQLVESGGGVVQPG GSLRLSCAAS | GFTFINY | AMGWYRQA PGRERELVA AI | TSLGGH | TYYADSVKGRFTISRD NAKSTVYLQMNSLRPE DTAVYYCHA | AQYYREVLGG QYDY | RGQGTLVTV SS |
| | SEQ ID NO: 4 | SEQ ID NO: 8 | SEQ ID NO: 14 | SEQ ID NO: 25 | SEQ ID NO: 36 | SEQ ID NO: 48 | SEQ ID NO: 51 |
| SZ0029 | QLQLVESGGGVVQPG GSLRLSCAAS | GFTFINY | AMGWYRQA PGRERELVA AI | TSLGGH | TTYADSVKGRFTISRD NSKNTLYLQMNSLRPE DTAVYYCHA | AQYYREVLGG QYDY | RGQGTLVTV SS |
| | SEQ ID NO: 4 | SEQ ID NO: 8 | SEQ ID NO: 14 | SEQ ID NO: 25 | SEQ ID NO: 37 | SEQ ID NO: 48 | SEQ ID NO: 51 |
| SZ0030 | QLQLVESGGGVVQPG GSLRLSCAAS | GFTFINY | AMGWYRQA PGRERELVA AI | TSLGGH | TTYADSVKGRFTISRD NAKSTVYLQMNSLRA EDTAVYYCHA | AQYYREVLGG QYDY | RGQGTLVTV SS |
| | SEQ ID NO: 4 | SEQ ID NO: 8 | SEQ ID NO: 14 | SEQ ID NO: 25 | SEQ ID NO: 38 | SEQ ID NO: 48 | SEQ ID NO: 51 |
| P15164-1-12 | QLQLVESGGGVVQPG GSLRLSCAAS | GFTFINY | AMGWYRQA PGRERELVA AI | TSLGGS | TTYADSVKGRFTISRD NSKNTLYLQMNSLRAE DTAVYYCHA | AQYYREVLGG QYDY | RGQGTLVTV SS |
| | SEQ ID NO: 4 | SEQ ID NO: 8 | SEQ ID NO: 14 | SEQ ID NO: 26 | SEQ ID NO: 39 | SEQ ID NO: 48 | SEQ ID NO: 51 |
| P15164-1-13 | QLQLVESGGGVVQPG GSLRLSCAAS | GFTFINY | AMGWYRQA PGRERELVA AI | TSLGGH | TYYADSVKGRFTISRD NSKNTLYLQMNSLRAE DTAVYYCHA | AQYYREVLGG QYDY | RGQGTLVTV SS |
| | SEQ ID NO: 4 | SEQ ID NO: 8 | SEQ ID NO: 14 | SEQ ID NO: 25 | SEQ ID NO: 40 | SEQ ID NO: 48 | SEQ ID NO: 51 |
| P15164-1-14 | QLQLVESGGGVVQPG GSLRLSCAAS | GFTFINY | AMGWYRQA PGRERELVA AI | TSLGGH | TTYADSVKGRFTISRD NSKNTLYLQMNSLRAE DTAVYYCHA | AQYYREVLGG QYDY | RGQGTLVTV SS |
| | SEQ ID NO: 4 | SEQ ID NO: 8 | SEQ ID NO: 14 | SEQ ID NO: 25 | SEQ ID NO: 39 | SEQ ID NO: 48 | SEQ ID NO: 51 |

| **IMGT** | FR1 | CDR1 | FR2 | CDR2 | FR3 | CDR3 | FR4 |
|---|---|---|---|---|---|---|---|
| P15164 | QLQLVESGGGVVQPG GSLTLSCAAS | GFTFINYA | MGWYRQAP GRERELVAA | ITSLGGHT | TYADSVKGRFTISRDN AKSTVYLQMNSLKPED TAVYYC | HAAQYYREVL GGQYDY | RGQGTQVTV SS |
| | SEQ ID NO: 3 | SEQ ID NO: 9 | SEQ ID NO: 18 | SEQ ID NO: 27 | SEQ ID NO: 41 | SEQ ID NO: 49 | SEQ ID NO: 50 |
| SZ0022 | QLQLVESGGGVVQPG GSLRLSCAAS | GFTFINYA | MGWYRQAP GRERELVAA | ITSLGGHT | TYADSVKGRFTISRDN AKSTVYLQMNSLRPED TAVYYC | HAAQYYREVL GGQYDY | RGQGTLVTV SS |
| | SEQ ID NO: 4 | SEQ ID NO: 9 | SEQ ID NO: 18 | SEQ ID NO: 27 | SEQ ID NO: 42 | SEQ ID NO: 49 | SEQ ID NO: 51 |
| SZ0023 | | GFTFINYA | MGWYRQAP GRERELVAA | ITSLGGHT | TYADSVKGRFTISRDN AKSTVYLQMNSLRPED TAVYYC | HAAQYYREVL GGQYDY | RGQGTLVTV SS |
| | SEQ ID NO: 5 | SEQ ID NO: 9 | SEQ ID NO: 18 | SEQ ID NO: 27 | SEQ ID NO: 42 | SEQ ID NO: 49 | SEQ ID NO: 51 |
| SZ0024 | QLQLVESGGGVVQPG GSLRLSCAAS | GFTFINYA | MGWFRQAP GRERELVAA | ITSLGGHT | TYADSVKGRFTISRDN AKSTVYLQMNSLRPED TAVYYC | HAAQYYREVL GGQYDY | RGQGTLVTV SS |
| | SEQ ID NO: 4 | SEQ ID NO: 9 | SEQ ID NO: 19 | SEQ ID NO: 27 | SEQ ID NO: 42 | SEQ ID NO: 49 | SEQ ID NO: 51 |
| SZ0025 | QLQLVESGGGVVQPG GSLRLSCAAS | GFTFINYA | MGWYRQAP GKERELVAA | ITSLGGHT | TYADSVKGRFTISRDN AKSTVYLQMNSLRPED TAVYYC | HAAQYYREVL GGQYDY | RGQGTLVTV SS |
| | SEQ ID NO: 4 | SEQ ID NO: 9 | SEQ ID NO: 20 | SEQ ID NO: 27 | SEQ ID NO: 42 | SEQ ID NO: 49 | SEQ ID NO: 51 |
| SZ0026 | QLQLVESGGGVVQPG GSLRLSCAAS | GFTFINYA | MGWYRQAP GRERESVAA | ITSLGGHT | TYADSVKGRFTISRDN AKSTVYLQMNSLRPED TAVYYC | HAAQYYREVL GGQYDY | RGQGTLVTV SS |
| | SEQ ID NO: 4 | SEQ ID NO: 9 | SEQ ID NO: 21 | SEQ ID NO: 27 | SEQ ID NO: 42 | SEQ ID NO: 49 | SEQ ID NO: 51 |
| SZ0027 | QLQLVESGGGVVQPG GSLRLSCAAS | GFTFINYA | MGWYRQAP GRERELVAA | ITSLGGST | TYADSVKGRFTISRDN AKSTVYLQMNSLRPED TAVYYC | HAAQYYREVL GGQYDY | RGQGTLVTV SS |
| | SEQ ID NO: 4 | SEQ ID NO: 9 | SEQ ID NO: 18 | SEQ ID NO: 28 | SEQ ID NO: 42 | SEQ ID NO: 49 | SEQ ID NO: 51 |
| SZ0028 | QLQLVESGGGVVQPG GSLRLSCAAS | GFTFINYA | MGWYRQAP GRERELVAA | ITSLGGHT | YYADSVKGRFTISRDN AKSTVYLQMNSLRPED TAVYYC | HAAQYYREVL GGQYDY | RGQGTLVTV SS |
| | SEQ ID NO: 4 | SEQ ID NO: 9 | SEQ ID NO: 18 | SEQ ID NO: 27 | SEQ ID NO: 43 | SEQ ID NO: 49 | SEQ ID NO: 51 |
| SZ0029 | QLQLVESGGGVVQPG GSLRLSCAAS | GFTFINYA | MGWYRQAP GRERELVAA | ITSLGGHT | TYADSVKGRFTISRDN SKNTLYLQMNSLRPED TAVYYC | HAAQYYREVL GGQYDY | RGQGTLVTV SS |
| | SEQ ID NO: 4 | SEQ ID NO: 9 | SEQ ID NO: 18 | SEQ ID NO: 27 | SEQ ID NO: 44 | SEQ ID NO: 49 | SEQ ID NO: 51 |
| SZ0030 | QLQLVESGGGVVQPG GSLRLSCAAS | GFTFINYA | MGWYRQAP GRERELVAA | ITSLGGHT | TYADSVKGRFTISRDN AKSTVYLQMNSLRAE DTAVYYC | HAAQYYREVL GGQYDY | RGQGTLVTV SS |
| | SEQ ID NO: 4 | SEQ ID NO: 9 | SEQ ID NO: 18 | SEQ ID NO: 27 | SEQ ID NO: 45 | SEQ ID NO: 49 | SEQ ID NO: 51 |
| P15164-1-12 | QLQLVESGGGVVQPG GSLRLSCAAS | GFTFINYA | MGWYRQAP GRERELVAA | ITSLGGST | TYADSVKGRFTISRDN SKNTLYLQMNSLRAED TAVYYC | HAAQYYREVL GGQYDY | RGQGTLVTV SS |
| | SEQ ID NO: 4 | SEQ ID NO: 9 | SEQ ID NO: 18 | SEQ ID NO: 28 | SEQ ID NO: 46 | SEQ ID NO: 49 | SEQ ID NO: 51 |
| P15164-1-13 | QLQLVESGGGVVQPG GSLRLSCAAS | GFTFINYA | MGWYRQAP GRERELVAA | ITSLGGHT | YYADSVKGRFTISRDN SKNTLYLQMNSLRAED TAVYYC | HAAQYYREVL GGQYDY | RGQGTLVTV SS |
| | SEQ ID NO: 4 | SEQ ID NO: 9 | SEQ ID NO: 18 | SEQ ID NO: 27 | SEQ ID NO: 47 | SEQ ID NO: 49 | SEQ ID NO: 51 |
| P15164-1-14 | QLQLVESGGGVVQPG GSLRLSCAAS | GFTFINYA | MGWYRQAP GRERELVAA | ITSLGGHT | TYADSVKGRFTISRDN SKNTLYLQMNSLRAED TAVYYC | HAAQYYREVL GGQYDY | RGQGTLVTV SS |
| | SEQ ID NO: 4 | SEQ ID NO: 9 | SEQ ID NO: 18 | SEQ ID NO: 27 | SEQ ID NO: 46 | SEQ ID NO: 49 | SEQ ID NO: 51 |

### DETAILED DESCRIPTION OF THE INVENTION

The present invention will be described with reference to the following examples which are intended to illustrate the present invention but not to limit the present invention.

Unless otherwise specified, the molecular biology experimental methods and immunoassays used in the present invention are basically performed based on the methods described in J. Sambrook et al., Molecular Cloning: Laboratory Manual, 2nd edition, Cold Spring Harbor Laboratory Press, 1989, and F. M. Ausubel et al., Compiled Molecular Biology Experiment Guide, 3rd edition, John Wiley & Sons, Inc., 1995. A person of skill in the art will appreciate that the Examples describe the present invention by way of examples and are not intended to limit the scope of protection claimed for the present invention.

### Reagent preparation and experimental methods

### 1. Protein sample preparation

The amino acid sequences of the proteins to be prepared (SEQ ID NO: 53360) were reverse-translated into nucleotide sequences, followed by construction of eukaryotic expression vectors and transient transfection into CHO or 293 cells. The target protein was purified using affinity chromatography (protein A, protein G, or nickel column). The purified protein was verified qualified by SDS-PAGE, SEC-HPLC and endotoxin assay before being used in subsequent experiments. Herein, hGHBP refers to the extracellular domain of human GHR.

### 2. Cell line construction

The encoding genes of human GHR protein (Uniprot accession number: P10912), cynomolgus monkey GHR protein (Uniprot accession number: A0A7N9D090), and rat GHR protein (Uniprot accession number: P16310) were ligated into plasmids to construct lentiviral plasmids for overexpressing hGHR, cGHR, and rGHR proteins, respectively. Lentiviral packaging and concentration were then performed to obtain lentiviral solutions. Baf3 (CTCC) and HEK293 cells were infected with the lentiviral solutions separately. After infection, infected cells were subjected to selective screening with puromycin (Puro) to obtain resistant cell pools. The expression of GHR protein was detected by flow cytometry. The identified positive cell pools were subjected to monoclonalization by limiting dilution. After amplification, culture and identification, Baf3 and HEK293 cell lines stably overexpressing GHR protein were obtained and designated as Baf3-hGHR, Baf3-cGHR, Baf3-rGHR, HEK293-hGHR, HEK293-cGHR and HEK293-rGHR cell lines.

### 3. Cell proliferative activity assay

Cell proliferative activity of Baf3-hGHR cells was determined using a cell proliferation and cytotoxicity assay kit (Dongren Chemical). Baf3-hGHR test cells were washed twice with assay medium (RPMI-1640 + 3% fetal bovine serum + 50 µM mercaptoethanol), starved for 4 h, counted, and seeded at 40,000 cells/50µL/well. GH was diluted to 25 nM in assay medium, and test samples were starting at 75-300 nM with 4-fold serial dilutions across 12 concentration gradients, added at 50 µL/well in triplicate. 50 ¿L of cells were seeded, and 50 ¿L of the test sample was added, for a total volume of 100 ¿L. Coincubation was conducted for 18 h. CCK8 detection reagent (5-fold diluted) was added at 50 µL/well, and the OD450 value was measured with a microplate reader after 3 h.

The proliferative activity assay for Baf3-rGHR cells was performed similarly to that for Baf3-hGHR cells, except that Baf3-rGHR cells were used and co-incubated for 72 h. CellCountingLite 2.0 working solution was added at 100 µL/well, and luminescence values were detected using a microplate detection system.

### 4. Affinity assay

The affinity assay was performed using a Biacore molecular interaction analyzer (cytiva, Biacore 8K+). Anti-Human antibody was immobilized on a CM-5 chip (cytiva, 29149603) according to the instructions of the anti-Human capture kit, type 2 (cytiva, 29234600). The test samples were captured by the chip, and the analyte was flowed over the chip with an association time of 180 s and a dissociation time of 300 s. Kinetic analysis was performed using Biacore Insight Evaluation Software 3.0.12 based on the collected data. hIgG1 was used as the negative control.

### 5. Rat growth assay

Animals used were rats with rGHR (rat GHR) knockout and hGHR (human GHR) knock-in. In these rats, rGH can only partially activate hGHR, resulting in a GH deficiency phenotype. The animals are smaller in body size and lower in body weight compared with wild-type SD rats of the same week age. hGHR transgenic rats aged 7-9 weeks were randomly grouped by body weight, with 6-8 rats per group. Administration was performed via subcutaneous injection at the neck. The negative control group received an equal volume of vehicle subcutaneously. PEG-GH was prepared according to the method described in CN101385858B, diluted with vehicle to the desired dosage, and administered subcutaneously at an equal volume.

In the single-dose experiment, body weight was measured daily for 14-18 days after administration. Blood samples were collected from rats at selected time points for IGF-1 level detection. Body length and tibia length were measured at the experimental endpoint. Body length refers to the distance from the tip of the rat's nose to its anus.

Blood was collected from the jugular vein using syringes according to the blood sampling schedule, with 100-150 ¿L of whole blood collected per animal. Whole blood was placed in clot-promoting tubes, allowed to stand at 2-8°C for 2 h, and then centrifuged twice at 1500 g for 10 min at 4°C. After centrifugation, the collected serum was transferred to a new labeled centrifuge tube and stored below -70°C.

Serum IGF-1 levels were determined using a commercially available ELISA kit (R&D Systems; Cat. No.: SMG100). The assay is a sandwich ELISA employing a highly IGF-1-specific polyclonal antibody as the capture agent and a high-affinity polyclonal antibody conjugated to horseradish peroxidase as the detection agent.

### Example 1: Discovery of GHR agonistic antibody molecules

### 1. Immunization, library construction and screening

Alpacas were immunized with the hGH/hGHBP-His complex and hGHBP-His. The first 7 immunizations were performed with the hGH/hGHBP-His complex, and the 8th immunization was boosted with hGHBP-His alone. Peripheral blood was collected after the 8th immunization for the construction of an alpaca immune library.

50 ml of fresh peripheral blood with qualified immune titer was collected, from which PBMCs were isolated. Total RNA was extracted and reverse-transcribed to synthesize first-strand cDNA. Two rounds of PCR were performed using the reverse-transcribed product as template: The first round of PCR primers designed against the signal peptide and heavy-chain constant region, yielding 2 bands, and the 2 bands were around 1000 bp and 750 bp. The 750 bp band was gel-recovered and used as template for the second round of PCR. The second round of PCR used primers targeting FR1 and FR4 regions, with restriction sites for the phagemid vector introduced. After restriction digestion and agarose gel recovery, the second-round PCR product was ligated into the digested vector and electrotransformed into Escherichia coli TG1 to complete the construction of the phage display library.

After library construction and phage packaging, solution-phase panning was performed using biotinylated hGHBP-His as the antigen. The bacterial cultures after rounds 1-4 of panning were subjected to limiting dilution and plated, followed by overnight incubation. Single colonies were picked for soluble expression. The soluble expression supernatant was detected using ELISA with hGHBP-mFc as the antigen. Finally, 1406 positive clones were selected for sequencing. Finally, Based on sequence characteristics, 11 clones were selected from 226 distinct sequences for further soluble expression and a series of in vitro activity assays. The preferred clone, the P15164 single-domain antibody, was identified for subsequent research. The VHH and CDRs sequences of clone P15164 are shown in Table 1.1 and Table 1.2.

### 2. Construction of full-length vector

### 2.1 Molecular construction

The aforementioned P15164 single-domain antibody was selected for fusion protein design. The design from N-terminus to C-terminus was as follows: P15164 single-domain antibody VHH (SEQ ID NO: 61), hIgG2 hinge region (SEQ ID NO: 62), and hIgG1 Fc region (T366Y, Δ447K, i.e., mutation of amino acid T at position 366 to Y and deletion of amino acid K at position 447) (SEQ ID NO: 65). The resulting fusion protein was designated as P15164-1.

### 2.2 ELISA binding activity assay

The P15164-1 molecule was purified and its binding activity to hGHBP was detected by ELISA.

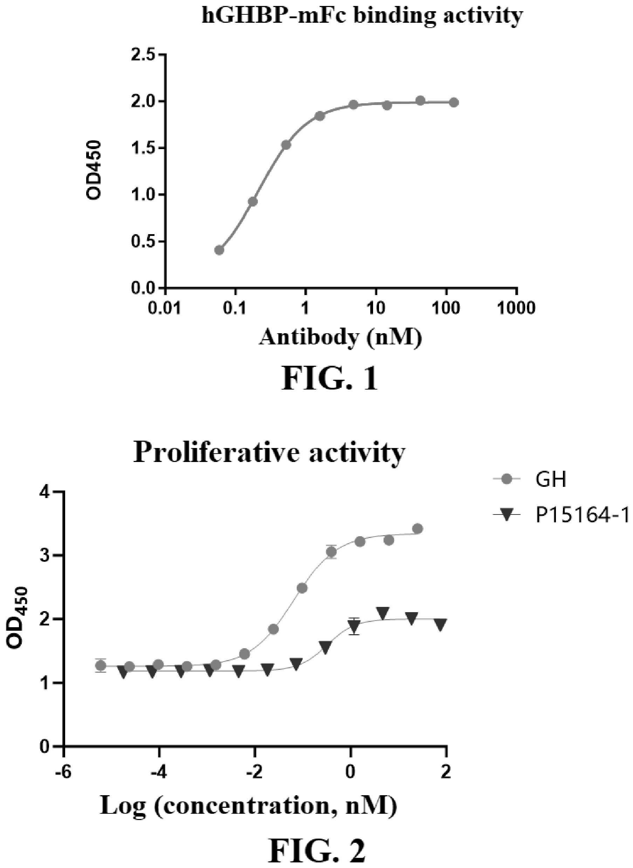

The hGHBP-mFc protein was coated onto an ELISA plate at 50 ¿l per well and incubated overnight at 4°C. After washing the plate 3 times with PBST, blocking was performed with 1% BSA at 250 ¿l per well, followed by incubation at room temperature for 2 h. After 3 washes with PBST, serially diluted P15164-1 samples were added separately at 50 ¿l per well, with an initial concentration of 10 µg/ml and eight 3-fold serial dilutions, then incubated at room temperature for 1 h. After washing the plate 6 times with PBST, a goat anti-human Fc secondary antibody diluted at a volume ratio of 1:5000 was added at 50 ¿l per well and incubated at room temperature for 1 h. The plate was washed 6 times with PBST, followed by the addition of TMB chromogenic solution at 50 ¿l per well, and color development was carried out in the dark at room temperature for 3-5 min. The reaction was terminated by adding 50 ¿l of stop solution per well. Immediately after termination, the microplate was placed in a microplate reader to measure the OD value at 450 nm, and the raw data were recorded and organized. The data was analyzed using software, and the results are shown in Fig. 1.

The results demonstrated that P15164-1 exhibited excellent binding activity to the hGHBP protein, with an EC50 value of 0.2135 nM.

### 2.3 Cell proliferative activity assay

The test cells were Baf3-hGHR cells, and the initial concentration of the test sample was 75 nM. The results are presented in Fig. 2 and Table 2.

**Table 2 Results of cell proliferative activity assay**

| **Sample name** | **EC50 (nM)** | **Maximum value** | **Minimum value** |
|---|---|---|---|
| **GH** | **0.06587** | **3.342** | **1.261** |
| **P15164-1** | **0.3225** | **2.003** | **1.188** |

The results showed that P15164-1 exhibited a certain degree of Baf3-hGHR cell proliferative activity.

### Example 2: Humanization

### Humanization design

Humanization was performed on the basis of P15164-1 by grafting the CDRs (defined by IMGT) into the human germline gene IgHV3-23. The importance of different amino acids in the CDR regions and FR regions was evaluated, and the corresponding amino acids were combined. The humanized variable region sequences are as set forth in SEQ ID NO: 67-75.

The humanized variable region was fused with the human IgG2 hinge region and the human IgG1 Fc region to construct heavy-chain antibodies. The human IgG1 Fc region was subjected to L234A/L235A (LALA) mutations to minimize Fc effector functions as much as possible. The sequences of the human IgG2 hinge region and the human IgG1 Fc (LALA) region are as set forth in SEQ ID NO: 62 and SEQ ID NO: 76, respectively. The constructed heavy chain antibody molecules were named SZ0022 to SZ0030.

### 2. Affinity assay

The experiment was carried out using a Biacore molecular interaction analyzer (cytiva, Biacore 8K+). The test samples were the SZ0022-SZ0030 series molecules (concentration: 0.5 µg/ml), and the analyte was hGHBP-His (concentration: 50 nM). The results are shown in Table 3.

**Table 3 Affinity assay results for SZ0022 to SZ0030**

| **Sample name** | **ka (1/Ms)** | **kd (1/s)** | **KD (M)** |
|---|---|---|---|
| **SZ0022** | **2.26E+05** | **2.67E-04** | **1.18E-09** |
| **SZ0023** | **2.33E+05** | **2.35E-04** | **1.01E-09** |
| **SZ0024** | **2.25E+05** | **2.95E-04** | **1.31E-09** |
| **SZ0025** | **2.28E+05** | **2.53E-04** | **1.11E-09** |
| **SZ0026** | **2.23E+05** | **2.99E-04** | **1.34E-09** |
| **SZ0027** | **2.82E+05** | **1.01E-03** | **3.57E-09** |
| **SZ0028** | **1.77E+05** | **1.04E-03** | **5.84E-09** |
| **SZ0029** | **2.24E+05** | **2.76E-04** | **1.23E-09** |
| **SZ0030** | **2.32E+05** | **2.54E-04** | **1.10E-09** |
| **hIgG 1** | **No binding** | | |

The results showed that the humanized molecules SZ0022 - SZ0030 all exhibited favorable binding affinity.

### 3. Cell proliferative activity assay

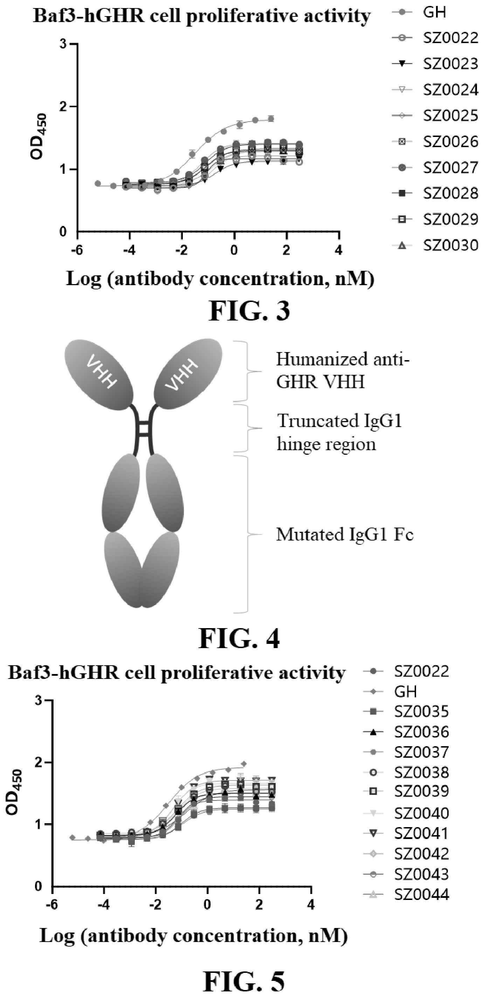

The cells used for detection were Baf3-hGHR cells, and the initial concentration of the test samples was 300 nM. The results are shown in Fig. 3 and Table 4.

**Table 4 Results of cell proliferative activity assay**

| **Sample name** | **Minimum value** | **Maximum value** | **EC₅₀ (nM)** |
|---|---|---|---|
| **GH** | **0.7225** | **1.79** | **0.032** |
| **SZ0022** | **0.7011** | **1.167** | **0.082** |
| **SZ0023** | **0.7292** | **1.13** | **0.180** |
| **SZ0024** | **0.7** | **1.217** | **0.128** |
| **SZ0025** | **0.7568** | **1.301** | **0.074** |
| **SZ0026** | **0.7231** | **1.286** | **0.106** |
| **SZ0027** | **0.7814** | **1.415** | **0.058** |
| **SZ0028** | **0.7815** | **1.401** | **0.074** |
| **SZ0029** | **0.759** | **1.327** | **0.085** |
| **SZ0030** | **0.74** | **1.304** | **0.133** |

The results showed that all humanized molecules SZ0022 - SZ0030 exhibited favorable cell proliferative activity on Baf3-hGHR cells.

### Example 3: Optimization of hinge region sequence

### 1. Sequence design

The hinge region of SZ0022 adopts the human IgG2 hinge region, in which 4 cysteines form interchain disulfide bonds with another heavy chain within the molecule. However, these disulfide bonds are unstable and tend to generate free thiol groups, which may form disulfide bonds with free thiols of other molecules, thereby leading to the formation of aggregates. In this example, the hinge region was optimized based on SZ0022. The optimization strategies include one or more of the following:
(1) mutating the two cysteines upstream of the IgG2 hinge region to serines; (2) adopting the hinge regions of IgG1 and IgG4; (3) mutating one cysteine upstream of the IgG1 hinge region to serine and/or gradually truncating the IgG1 hinge region to identify the optimal hinge region length; (4) optimizing the amino acid composition upstream of the hinge region disulfide bonds; (5) mutating residue S at position 228 (defined by the EU numbering system) in the IgG4 hinge region to P.

A series of hinge regions were designed, and the constructed heavy-chain antibody molecules were designated SZ0035 to SZ0044. The molecular information is shown in Table 5, and an exemplary structure of the constructs is shown in Fig. 4.

**Table 5 Information of constructed heavy chain antibody molecules**

| **Sample name** | **Variable region** | **Hinge region** | **Fc region** |
|---|---|---|---|
| **SZ0022** | **SEQ ID NO: 67** | **ERKCCVECPPCP (SEQ ID NO: 62)** | **IgG1Fc (LALA) (SEQ ID NO: 76)** |
| **SZ0035** | **SEQ ID NO: 67** | **ERKSSVECPPCP (SEQ ID NO: 80)** | **IgG1Fc (LALA) (SEQ ID NO: 76)** |
| **SZ0036** | **SEQ ID NO: 67** | **ERKCPPCP (SEQ ID NO: 81)** | **IgG1Fc (LALA) (SEQ ID NO: 76)** |
| **SZ0037** | **SEQ ID NO: 67** | **EPKSSDKTHTCPPCP (SEQ ID NO: 82)** | **IgG1Fc (LALA) (SEQ ID NO: 76)** |
| **SZ0038** | **SEQ ID NO: 67** | **DKTHTCPPCP (SEQ ID NO: 83)** | **IgG1Fc (LALA) (SEQ ID NO: 76)** |
| **SZ0039** | **SEQ ID NO: 67** | **KTHTCPPCP (SEQ ID NO: 84)** | **IgG1Fc (LALA) (SEQ ID NO: 76)** |
| **SZ0040** | **SEQ ID NO: 67** | **THTCPPCP (SEQ ID NO: 85)** | **IgG1Fc (LALA) (SEQ ID NO: 76)** |
| **SZ0041** | **SEQ ID NO: 67** | **HTCPPCP (SEQ ID NO: 86)** | **IgG1Fc (LALA) (SEQ ID NO: 76)** |
| **SZ0042** | **SEQ ID NO: 67** | **TCPPCP (SEQ ID NO: 87)** | **IgG1Fc (LALA) (SEQ ID NO: 76)** |
| **SZ0043** | **SEQ ID NO: 67** | **CPPCP (SEQ ID NO: 88)** | **IgG1Fc (LALA) (SEQ ID NO: 76)** |
| **SZ0044** | **SEQ ID NO: 67** | **ESKYGPPCPPCP (SEQ ID NO: 89)** | **IgG4Fc (L235E) (SEQ ID NO: 79)** |

### 2. Protein sample preparation

SZ0035 to SZ0044 were constructed via vector construction and expressed in the same batch as the parental molecule SZ0022. After one-step protein A affinity chromatography, the proteins were identified by SEC-HPLC. The overall expression, purification and identification results are shown in Table 6.

**Table 6 Results of protein expression, purification, and identification.**

| **Sample name** | **SEC-HPLC** | **Reducing SDS-PAGE** |
|---|---|---|
| **SZ0022** | **61.057%** | **>95%** |
| **SZ0035** | **99.331%** | **>95%** |
| **SZ0036** | **99.355%** | **>95%** |
| **SZ0037** | **99.542%** | **>85%** |
| **SZ0038** | **100.000%** | **>95%** |
| **SZ0039** | **99.362%** | **>95%** |
| **SZ0040** | **99.177%** | **>95%** |
| **SZ0041** | **99.622%** | **>95%** |
| **SZ0042** | **100.000%** | **>95%** |
| **SZ0043** | **99.110%** | **>95%** |
| **SZ0044** | **99.360%** | **>95%** |

The results showed that the monomer purity of SZ0022 was above 60%, while the SEC-HPLC purity of SZ0035 to SZ0044 all exceeded 99% after optimization of the hinge region sequence.

### 3. Affinity assay

The assay was performed using a Biacore molecular interaction analyzer (cytiva, Biacore 8K+). The test samples were the SZ0035 to SZ0044 series molecules (concentration: 0.5 µg/ml), and the analyte was hGHBP-His (concentration: 50 nM). The results are as shown in Table 7.

**Table 7 Protein affinity test results**

| **Sample** | **ka (1/Ms)** | **kd (1/s)** | **KD (M)** |
|---|---|---|---|
| **SZ0022** | **2.29E+05** | **3.46E-04** | **1.51E-09** |
| **SZ0035** | **2.31E+05** | **3.08E-04** | **1.33E-09** |
| **SZ0036** | **2.15E+05** | **2.64E-04** | **1.23E-09** |
| **SZ0037** | **2.29E+05** | **2.74E-04** | **1.20E-09** |
| **SZ0038** | **2.27E+05** | **2.67E-04** | **1.18E-09** |
| **SZ0039** | **1.97E+05** | **2.51E-04** | **1.27E-09** |
| **SZ0040** | **2.09E+05** | **2.54E-04** | **1.22E-09** |
| **SZ0041** | **2.21E+05** | **2.32E-04** | **1.05E-09** |
| **SZ0042** | **2.17E+05** | **2.94E-04** | **1.35E-09** |
| **SZ0043** | **2.26E+05** | **2.67E-04** | **1.18E-09** |
| **SZ0044** | **2.43E+05** | **3.06E-04** | **1.26E-09** |
| **hIgG1** | **No binding** | | |

The results showed that when the variable region was not changed and only the constant region was changed, the mutations in the hinge region did not affect the affinity of SZ0035 to SZ0044.

### 4. Cell proliferative activity identification

The test cells were Baf3-hGHR cells, and the initial concentration of the test sample was 300 nM. The results are shown in FIG. 5 and Table 8.

**Table 8 Results of cell proliferative activity assay**

| **Sample name** | **Minimum value** | **Maximum value** | **EC₅₀ (nM)** |
|---|---|---|---|
| **GH** | **0.7462** | **1.926** | **0.030** |
| **SZ0022** | **0.7828** | **1.398** | **0.074** |
| **SZ0035** | **0.7573** | **1.273** | **0.099** |
| **SZ0036** | **0.8212** | **1.507** | **0.059** |
| **SZ0037** | **0.7924** | **1.252** | **0.122** |
| **SZ0038** | **0.8542** | **1.585** | **0.069** |
| **SZ0039** | **0.817** | **1.637** | **0.061** |
| **SZ0040** | **0.8248** | **1.664** | **0.043** |
| **SZ0041** | **0.7788** | **1.715** | **0.044** |
| **SZ0042** | **0.8148** | **1.511** | **0.061** |
| **SZ0043** | **0.7762** | **1.45** | **0.073** |
| **SZ0044** | **0.8114** | **1.565** | **0.137** |

The results showed that all of SZ0035 to SZ0044 exhibited favorable cell proliferative activity in Baf3-hGHR cells. The cell proliferative activity gradually increased with the shortening of the hinge region upstream of the disulfide bond, and reached the highest activity (maximum value) when truncated to only two amino acids (SZ0041, namely hinge region HTCPPCP), while further shortening results in decreased activity.

### Example 4: Combined optimization of variable region and hinge region

In this example, combined optimization of the variable region and hinge region was further performed on the basis of Examples 2 and 3, aiming to combine the variable region sequence with the highest degree of humanization and the optimized hinge region sequence.

### 1. Molecular design

Based on previous humanization results, amino acids with little effect on activity were selected and combined to improve the degree of humanization, and 3 new humanized variable region sequences, P15164-1-12 to P15164-1-15, were designed, and the sequences are as set forth in SEQ ID NO: 90-92. The hinge region adopts the optimized hinge region, with the sequence HTCPPCP (SEQ ID NO: 86) and TCPPCP (SEQ ID NO: 87). The Fc region uses IgG1Fc (LALA) (SEQ ID NO: 76).

The constructed heavy chain antibody molecules were named SZ0051 to SZ0056, and their molecular information is shown in Table 9.

**Table 9 Information of constructed heavy chain antibody molecules**

| **Sample name** | **Variable region** | **Hinge region** | **Fc region** |
|---|---|---|---|
| **SZ0051** | **P15164-1-12 (SEQ ID NO : 90)** | **HTCPPCP (SEQ ID NO: 86)** | **IgG1Fc(LALA) (SEQ ID NO: 76)** |
| **SZ0052** | **P15164-1-12 (SEQ ID NO : 90)** | **TCPPCP (SEQ ID NO: 87)** | **IgG1Fc(LALA) (SEQ ID NO: 76)** |
| **SZ0053** | **P15164-1-13 (SEQ ID NO: 91)** | **HTCPPCP (SEQ ID NO: 86)** | **IgG1Fc(LALA) (SEQ ID NO: 76)** |
| **SZ0054** | **P15164-1-13 (SEQ ID NO: 91)** | **TCPPCP (SEQ ID NO: 87)** | **IgG1Fc(LALA) (SEQ ID NO: 76)** |
| **SZ0055** | **P15164-1-14 (SEQ ID NO: 92)** | **HTCPPCP (SEQ ID NO: 86)** | **IgG1Fc(LALA) (SEQ ID NO: 76)** |
| **SZ0056** | **P15164-1-14 (SEQ ID NO: 92)** | **TCPPCP (SEQ ID NO: 87)** | **IgG1Fc(LALA) (SEQ ID NO: 76)** |

### 2. Affinity assay

The assay was performed using a Biacore molecular interaction analyzer (cytiva, Biacore 8K+). The test samples were the SZ0051 to SZ0056 series molecules (concentration: 0.5 µg/ml), and the analyte was hGHBP-His (concentration: 50 nM). The results are as shown in Table 10.

**Table 10 Affinity assay esults**

| **Sample** | **ka (1/Ms)** | **kd (1/s)** | **KD (M)** |
|---|---|---|---|
| **SZ0022** | **2.09E+05** | **2.69E-04** | **1.29E-09** |
| **SZ0051** | **2.73E+05** | **1.33E-03** | **4.86E-09** |
| **SZ0052** | **2.67E+05** | **9.55E-04** | **3.57E-09** |
| **SZ0053** | **2.12E+05** | **1.31E-03** | **6.18E-09** |
| **SZ0054** | **1.96E+05** | **1.16E-03** | **5.94E-09** |
| **SZ0055** | **2.03E+05** | **2.73E-04** | **1.34E-09** |
| **SZ0056** | **2.05E+05** | **2.53E-04** | **1.23E-09** |
| **hIgG1** | **No binding** | | |

The results showed that SZ0051 to SZ0056 had good affinity.

### 3. Species cross-reactivity assay

The assay was performed using a Biacore molecular interaction analyzer (cytiva, Biacore 8K+). The test samples were the SZ0051 to SZ0056 series molecules (concentration: 2 µg/ml), and the analyte was cyno/rat GHBP-His (concentration: 200 nM). The results are as shown in Table 11.

**Table 11 Results of species cross-reactivity assay**

| **Sample name** | **cGHBP-His (200nM)** | | |
|---|---|---|---|
| | **ka (1/Ms)** | **kd (1/s)** | **KD (M)** |
| **SZ0022** | **7.99E+04** | **3.56E-02** | **4.46E-07** |
| **SZ0051** | **1.57E+05** | **8.06E-02** | **5.13E-07** |
| **SZ0052** | **4.30E+05** | **1.64E-01** | **3.82E-07** |
| **SZ0053** | **3.68E+05** | **1.38E-01** | **3.76E-07** |
| **SZ0054** | **4.43E+05** | **1.48E-01** | **3.35E-07** |
| **SZ0055** | **1.19E+05** | **4.80E-02** | **4.01E-07** |
| **SZ0056** | **7.37E+04** | **4.27E-02** | **5.80E-07** |

| **Sample name** | **rGHBP-His(200nM)** | | |
|---|---|---|---|
| | **ka (1/Ms)** | **kd (1/s)** | **KD (M)** |
| **SZ0022** | **9.67E+05** | **1.87E-02** | **1.93E-08** |
| **SZ0051** | **1.36E+07** | **1.54E-01** | **1.13E-08** |
| **SZ0052** | **6.40E+06** | **9.39E-02** | **1.47E-08** |
| **SZ0053** | **3.77E+05** | **2.30E-03** | **6.09E-09** |
| **SZ0054** | **3.37E+05** | **2.08E-03** | **6.16E-09** |
| **SZ0055** | **9.61E+05** | **1.05E-02** | **1.09E-08** |
| **SZ0056** | **8.33E+05** | **1.53E-02** | **1.84E-08** |

The results showed that SZ0051 to SZ0056 exhibited certain cross-reactivity with rat GHBP (rGHBP-His), while showing little to no cross-reactivity with cynomolgus monkey GHBP (cGHBP-His).

### 4. Cell binding activity assay

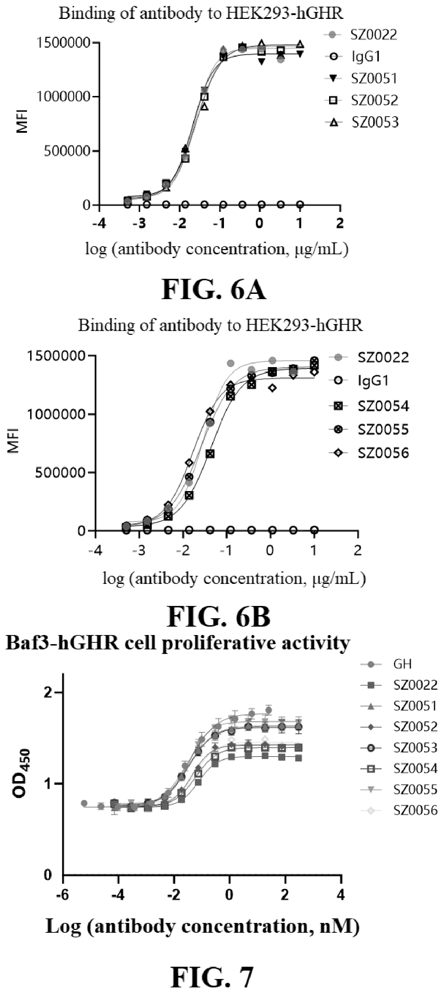

The binding activity of the test antibody to HEK293-hGHR was detected using the iQue3 system (Sartorius): 10 µg/mL, 3-fold serial dilution, for a total of 10 gradients, incubated at 4°C for 1 h; the secondary antibody for detection was R-Phycoerythrin AffiniPure Goat Anti-Human IgG, Fcγ fragment specific (Jackson). The results are shown in Figs. 6A-6B.

The results showed that SZ0051~SZ0056 exhibited favorable binding activity to HEK293-hGHR cells.

### 5. Cell proliferative activity assay

### 5.1 Baf3-hGHR cell proliferative activity

The test cells were Baf3-hGHR cells, and the initial concentration of the test sample was 300 nM. The results are presented in Fig. 7 and Table 12.

**Table 12 Results of cell proliferative activity assay**

| **Sample name** | **EC₅₀ (nM)** | **Minimum value** | **Maximum value** |
|---|---|---|---|
| **GH** | **0.035** | **0.7444** | **1.772** |
| **SZ0022** | **0.074** | **0.743** | **1.299** |
| **SZ0051** | **0.030** | **0.7372** | **1.635** |
| **SZ0052** | **0.043** | **0.7538** | **1.427** |
| **SZ0053** | **0.033** | **0.7624** | **1.619** |
| **SZ0054** | **0.058** | **0.746** | **1.397** |
| **SZ0055** | **0.034** | **0.7812** | **1.682** |
| **SZ0056** | **0.058** | **0.786** | **1.453** |

The results showed that SZ0051 to SZ0056 all exhibited good Baf3-hGHR cell proliferative activity.

### 5.2 Baf3-rGHR cell proliferative activity

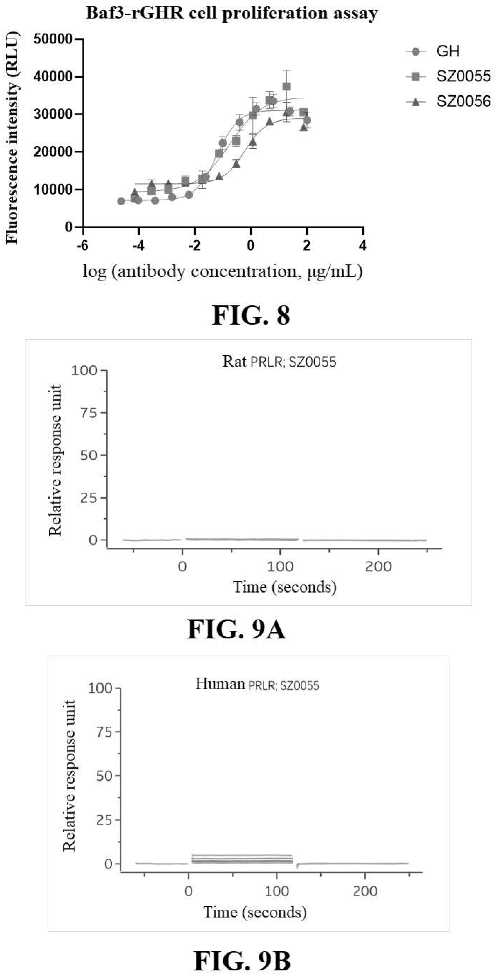

The test cells were Baf3-rGHR cells, and the initial concentration of the test sample was 300 nM. The results are presented in Fig. 8 and Table 13.

**Table 13 Results of cell proliferative activity assay**

| **Sample name** | **Minimum value** | **Maximum value** | **EC₅₀ (nM)** |
|---|---|---|---|
| **GH** | **7169** | **31098** | **0.06** |
| **SZ0055** | **9409** | **34715** | **0.17** |
| **SZ0056** | **11541** | **28963** | **0.60** |

The results showed that both SZ0055 and SZ0056 had Baf3-rGHR cell proliferative activity.

### 6. Verification of PRLR (prolactin receptor) binding activity

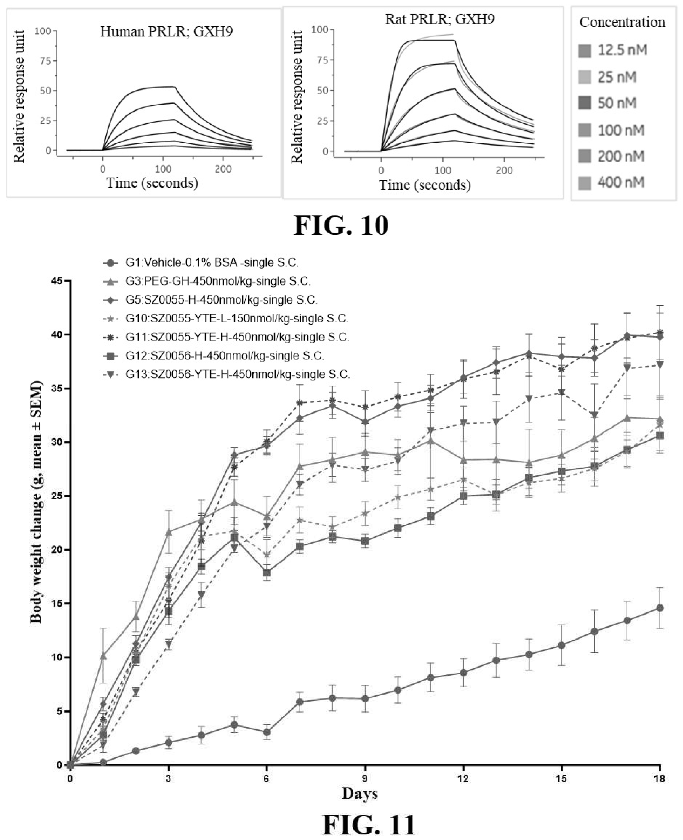

The assay was performed using a Biacore molecular interaction analyzer (cytiva, Biacore 8K+). The test samples were SZ0055 or GX-H9 molecules at a concentration of 2 µg/ml, and the analytes were human PRLR (hum PRLR) and rat PRLR (Sino Biological) at concentrations of 12.5, 25, 50, 100, 200, and 400 nM. The results are presented in Figs. 9A-9B, Fig. 10 and Table 14.

**Table 14 Results of PRLR binding activity assay**

| **Immobilized molecule** | **Analyte** | **ka (1/Ms)** | **kd (1/s)** | **KD (M)** |
|---|---|---|---|---|
| **SZ0055** | **hum PRLR** | **No binding** | | |
| **GX-H9** | | **1.00E+05** | **1.65E-02** | **1.65E-07** |
| **SZ0055** | **rat PRLR** | **No binding** | | |
| **GX-H9** | | **7.53E+05** | **4.70E-02** | **6.25E-08** |

The results showed that SZ0055 exhibited no binding to either human PRLR or rat PRLR, indicating that SZ0055 does not activate the prolactin receptor.

### 7. Rat growth assay

Male hGHR transgenic rats aged 7-9 weeks were randomly grouped according to body weight. The dosing information for each group is as follows:

**Table 15 Dosing information for each group**

| **Group** | **Dosing formulation** | **Number of animals** | **Dose (nmol/kg)** | **Dosing route** | **Dosing frequency** |
|---|---|---|---|---|---|
| **G1** | **Vehicle** | **8** | **/** | **Subcutaneous** | **1** |
| **G3** | **PEG-GH** | **8** | **450** | **Subcutaneous** | **1** |
| **G5** | **SZ0055** | **8** | **450** | **Subcutaneous** | **1** |
| **G10** | **SZ0055-YTE** | **8** | **150** | **Subcutaneous** | **1** |
| **G11** | **SZ0055-YTE** | **8** | **450** | **Subcutaneous** | **1** |
| **G12** | **SZ0056** | **8** | **450** | **Subcutaneous** | **1** |
| **G13** | **SZ0056-YTE** | **8** | **450** | **Subcutaneous** | **1** |

Among them, SZ0055-YTE (also designated SZ0112, SEQ ID NO: 96) is a construct obtained by replacing the Fc region of SZ0055 with IgG1 Fc(AA,YTE); similarly, SZ0056-YTE (also designated SZ0113, SEQ ID NO: 97) is a construct obtained by replacing the Fc region of SZ0056 with IgG1 Fc(AA,YTE). IgG1 Fc(AA,YTE) comprises the L234A/L235A and M252Y/S254T/T256E mutations, and its amino acid sequence is as set forth in SEQ ID NO: 93.

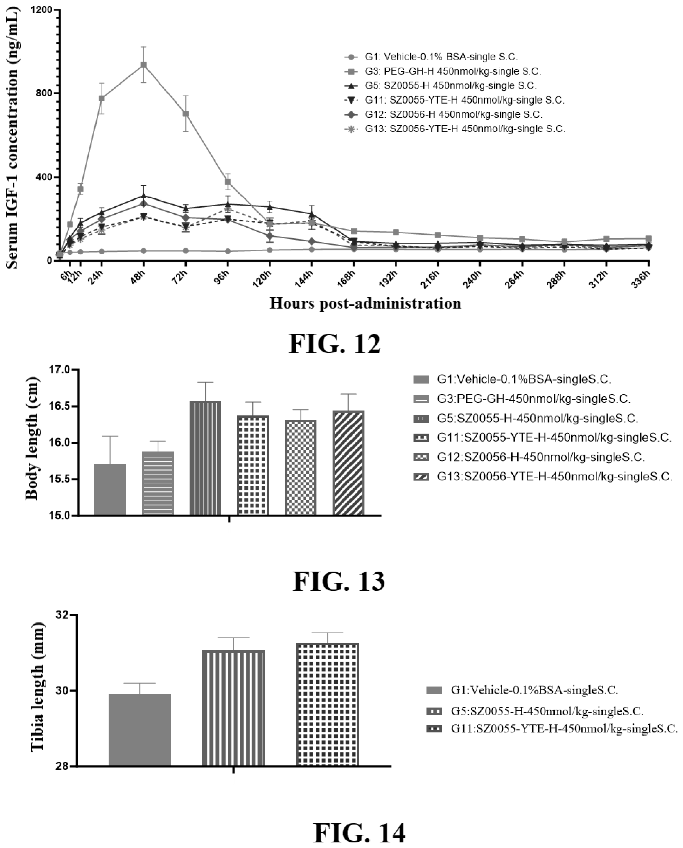

Body weight, body length, tibia length, and IGF-1 level were measured, and the results are shown in Figs. 11-14.

The results demonstrated that all treatment groups significantly increased body weight, body length, and tibia length in animals compared with the vehicle control group. Serum IGF-1 detection revealed that, relative to the vehicle control group, the test drug groups significantly elevated IGF-1 levels, which persisted until returning to vehicle control levels on day 7. In comparison with PEG-GH, whose level returned to vehicle control levels on day 3, the test drugs significantly prolonged the duration of elevated IGF-1 levels, while exhibiting a lower IGF-1 peak than PEG-GH.

### 8. PK in Cynomolgus monkeys (YTE selected)

Following single subcutaneous injection of SZ0055, SZ0056, SZ0055-YTE and SZ0056-YTE to male cynomolgus monkeys, serum samples were collected at various time points to investigate the changes in systemic exposure of SZ0055, SZ0056, SZ0055-YTE and SZ0056-YTE molecules in male cynomolgus monkeys. The pharmacokinetic profiles of the four molecules (SZ0055, SZ0056, SZ0055-YTE and SZ0056-YTE) were evaluated and compared.

### 8.1 Formulation preparation

The test drugs were liquid formulations for subcutaneous (S.C.) injection of SZ0055, SZ0056, SZ0055-YTE and SZ0056-YTE, provided by Changchun GeneScience Pharmaceuticals Co., Ltd. According to Table 17, the test drugs were diluted to the corresponding concentration (5 mg/mL) with formulation vehicle (vehicle, no drug) and then administered.

### 8.2 Experimental animals

The experimental animals were housed in the animal facility of Suzhou Xihua Pharmaceutical Development Co., Ltd. (Animal Use License No.: SYXK (Su) 2021-0018). he source and number of animals used in this experiment are shown in Table 16.

**Table 16 Source and number of experimental animals**

| **Species** | **Strain** | **Sex** | **Source** | **Body weight** | **Age** | **Number of animals** |
|---|---|---|---|---|---|---|
| **Monkey** | **Cynomolgus monkey** | **Male** | **Guangxi** | **About 3-6.5 kg** | **3.5-6 years** | **12 Animals** |

### 8.3 Experimental design:

### 8.3.1 Grouping of experimental animals

The dosing regimen design is shown in Table 17.

**Table 17 Animal grouping**

| **Group** | **Dosing formulation** | **Number of animals/Animal ID** | **Dose (mg/kg)** | **Volume (mL/kg)** | **Concentration (mg/mL)** | **Dosing route** | **Dosing frequency** |
|---|---|---|---|---|---|---|---|
| **A** | **SZ0055** | **3/C01 - C03** | **10** | **2** | **5** | **S.C** | **Single dose** |
| **B** | **SZ0056** | **3/C04 - C06** | **10** | **2** | **5** | **S.C** | **Single dose** |
| **C** | **SZ0055-YTE** | **3/C07 - C09** | **10** | **2** | **5** | **S.C** | **Single dose** |
| **D** | **SZ0056-YTE** | **3/C10 - C12** | **10** | **2** | **5** | **S.C** | **Single dose** |

### 8.3.2 Sample collection and processing

Samples were collected at 0 h (within 10 min before dosing); and at 1 h, 2 h, 4 h, 8 h, 12 h, 18 h, 24 h (D1), 36 h, 48 h (D2), 72 h (D3), 96 h (D4), 120 h (D5), 144 h (D6), 168 h (D7), 192 h (D8), 216 h (D9), 240 h (D10), 264 h (D11), 288 h (D12), 312 h (D13), 336 h (D14), 384 h (D16), 432 h (D18), 480 h (D20), 576 h (D24), and 672 h (D28) after administration.

### 8.4 Experimental data

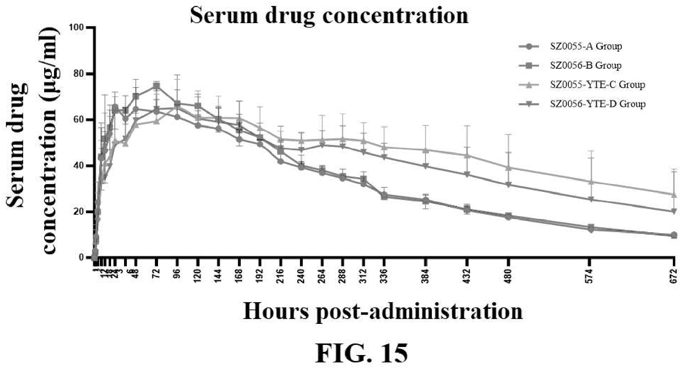

The experimental results are shown in FIG. 15. The results indicated that the YTE mutation in the Fc fragment significantly prolonged the PK of the SZ0055 and SZ0056 molecules.

Although the specific embodiments of the present invention have been described in detail, those skilled in the art will understand that various modifications and changes may be made to the details based on all the teachings published, and these changes are all within the scope of protection of the present invention. The entire scope of the present invention is given by the appended claims and any equivalents thereof.

## Claims

1. A single-domain antibody specifically binding to a growth hormone receptor (GHR) or an antigen-binding fragment thereof.

2. The single-domain antibody or antigen-binding fragment thereof of claim 1, comprising:
CDR1 or a variant thereof, CDR2 or a variant thereof, and CDR3 or a variant thereof contained in a heavy chain variable region (VHH) as set forth in any one of SEQ ID NO: 61, 67-75, 90-92;
wherein the variant has one or several amino acid substitutions, deletions or insertions (e.g., 1, 2 or 3 amino acid substitutions, deletions or insertions) compared with the sequence from which it is derived;
preferably, the substitutions are conservative substitutions;
preferably, the single-domain antibody or antigen-binding fragment thereof comprises the 3 CDRs contained in the heavy chain variable region (VHH) as set forth in any one of SEQ ID NO: 61, 67-75, 90-92.

3. The single-domain antibody or antigen-binding fragment thereof of claim 1 or 2, comprising:
(a) (i) CDR1 having a sequence as set forth in SEQ ID NO: 7, or a sequence having one or several amino acid substitutions, deletions or insertions (e.g., 1, 2 or 3 amino acid substitutions, deletions or insertions) compared with the sequence as set forth in SEQ ID NO: 7;
(ii) CDR2 having a sequence as set forth in any one of SEQ ID NO: 22-24, or a sequence having one or several amino acid substitutions, deletions or insertions (e.g., 1, 2 or 3 amino acid substitutions, deletions or insertions) compared with the sequence as set forth in any one of SEQ ID NO: 22-24; and
(iii) CDR3 having a sequence as set forth in SEQ ID NO: 48, or a sequence having one or several amino acid substitutions, deletions or insertions (e.g., 1, 2 or 3 amino acid substitutions, deletions or insertions) compared with the sequence as set forth in SEQ ID NO: 48;
wherein the CDRs are defined by the Kabat numbering system;
(b) (i) CDR1 having a sequence as set forth in SEQ ID NO: 9, or a sequence having one or several amino acid substitutions, deletions or insertions (e.g., 1, 2 or 3 amino acid substitutions, deletions or insertions) compared with the sequence as set forth in SEQ ID NO: 9;
(ii) CDR2 having a sequence as set forth in SEQ ID NO: 27 or 28, or a sequence having one or several amino acid substitutions, deletions or insertions (e.g., 1, 2 or 3 amino acid substitutions, deletions or insertions) compared with the sequence as set forth in SEQ ID NO: 27 or 28; and
(iii) CDR3 having a sequence as set forth in SEQ ID NO: 49, or a sequence having one or several amino acid substitutions, deletions or insertions (e.g., 1, 2 or 3 amino acid substitutions, deletions or insertions) compared with the sequence as set forth in SEQ ID NO: 49;
wherein the CDRs are defined by the IMGT numbering system;
or,
(c) (i) CDR1 having a sequence as set forth in SEQ ID NO: 8, or a sequence having one or several amino acid substitutions, deletions or insertions (e.g., 1, 2 or 3 amino acid substitutions, deletions or insertions) compared with the sequence as set forth in SEQ ID NO: 8;
(ii) CDR2 having a sequence as set forth in SEQ ID NO: 25 or 26, or a sequence having one or several amino acid substitutions, deletions or insertions (e.g., 1, 2 or 3 amino acid substitutions, deletions or insertions) compared with the sequence as set forth in SEQ ID NO: 25 or 26; and
(iii) CDR3 having a sequence as set forth in SEQ ID NO: 48, or a sequence having one or several amino acid substitutions, deletions or insertions (e.g., 1, 2 or 3 amino acid substitutions, deletions or insertions) compared with the sequence as set forth in SEQ ID NO: 48;
wherein the CDRs are defined by the Chothia numbering system;
preferably, the substitutions are conservative substitutions.

4. The single-domain antibody or antigen-binding fragment thereof of any one of claims 1 to 3, comprising:
(a) CDR1 as set forth in SEQ ID NO: 7, CDR2 as set forth in any one of SEQ ID NO: 22-24, and CDR3 as set forth in SEQ ID NO: 48; wherein the CDRs are defined by the Kabat numbering system;
(b) CDR1 as set forth in SEQ ID NO: 9, CDR2 as set forth in SEQ ID NO: 27 or 28, and CDR3 as set forth in SEQ ID NO: 49; wherein the CDRs are defined by the IMGT numbering system;
or,
(c) CDR1 as set forth in SEQ ID NO: 8, CDR2 as set forth in SEQ ID NO: 25 or 26, and CDR3 as set forth in SEQ ID NO: 48; wherein the CDRs are defined by the Chothia numbering system;
preferably, the single-domain antibody or antigen-binding fragment thereof comprises:
(i) CDR1 as set forth in SEQ ID NO: 7, CDR2 as set forth in SEQ ID NO: 22; and CDR3 as set forth in SEQ ID NO: 48; wherein the CDRs are defined by the Kabat numbering system;
(ii) CDR1 as set forth in SEQ ID NO: 7, CDR2 as set forth in SEQ ID NO: 23; and CDR3 as set forth in SEQ ID NO: 48; wherein the CDRs are defined by the Kabat numbering system;
(iii) CDR1 as set forth in SEQ ID NO: 7, CDR2 as set forth in SEQ ID NO: 24; and CDR3 as set forth in SEQ ID NO: 48; wherein the CDRs are defined by the Kabat numbering system;
(iv) CDR1 as set forth in SEQ ID NO: 9, CDR2 as set forth in SEQ ID NO: 27; and CDR3 as set forth in SEQ ID NO: 49; wherein the CDRs are defined by the IMGT numbering system;
(v) CDR1 as set forth in SEQ ID NO: 9, CDR2 as set forth in SEQ ID NO: 28; and CDR3 as set forth in SEQ ID NO: 49; wherein the CDRs are defined by the IMGT numbering system;
(vi) CDR1 as set forth in SEQ ID NO: 8, CDR2 as set forth in SEQ ID NO: 25; and CDR3 as set forth in SEQ ID NO: 48; wherein the CDRs are defined by the Chothia numbering system;
(vii) CDR1 as set forth in SEQ ID NO: 8, CDR2 as set forth in SEQ ID NO: 26; and CDR3 as set forth in SEQ ID NO: 48; wherein the CDRs are defined by the Chothia numbering system.

5. The single-domain antibody or antigen-binding fragment thereof of any one of claims 1 to 4, comprising an amino acid sequence selected from the group consisting of:
(i) a sequence as set forth in SEQ ID NO: 61;
(ii) a sequence having one or several amino acid substitutions, deletions or insertions (e.g., 1, 2, 3, 4 or 5 amino acid substitutions, deletions or insertions) compared with the sequence as set forth in SEQ ID NO: 61; or
(iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as set forth in SEQ ID NO: 61;
preferably, the substitutions are conservative substitutions.

6. The single-domain antibody or antigen-binding fragment thereof of any one of claims 1 to 5, wherein the single-domain antibody or antigen-binding fragment thereof is humanized;
preferably, the single-domain antibody or antigen-binding fragment thereof comprises the heavy chain framework regions of a human immunoglobulin;
preferably, the single-domain antibody or antigen-binding fragment thereof comprises the heavy chain framework regions in an amino acid sequence encoded by a human heavy chain germline antibody gene;
preferably, the single-domain antibody or antigen-binding fragment thereof comprises the heavy chain framework regions in an amino acid sequence encoded by a human IGHV gene, wherein the human IGHV gene is selected from IGHV3-23, IGHV3-30, IGHV3-34, IGHV3-7 and IGHV3-74;
preferably, the heavy chain framework regions optionally comprise one or more (e.g., 1, 2, 3, 4, 5, 6, 7, 8, 9 or 10) back mutations from human residues to camelid residues;
preferably, the single-domain antibody or antigen-binding fragment thereof comprises:
(a) FR1 as set forth in SEQ ID NO: 2 or 6, FR2 as set forth in any one of SEQ ID NO: 10-13, FR3 as set forth in any one of SEQ ID NO: 30-33, and/or FR4 as set forth in SEQ ID NO: 51; wherein the FRs are defined by the Kabat numbering system;
(b) FR1 as set forth in SEQ ID NO: 4 or 5, FR2 as set forth in any one of SEQ ID NO: 18-21, FR3 as set forth in any one of SEQ ID NO: 42-47, and/or FR4 as set forth in SEQ ID NO: 51; wherein the FRs are defined by the IMGT numbering system;
or
(c) FR1 as set forth in SEQ ID NO: 4 or 5, FR2 as set forth in any one of SEQ ID NO: 14-17, FR3 as set forth in any one of SEQ ID NO: 35-40, and/or FR4 as set forth in SEQ ID NO: 51; wherein the FRs are defined by the Chothia numbering system;
preferably, the single-domain antibody or antigen-binding fragment thereof comprises:
(i) a sequence as set forth in any one of SEQ ID NO: 67-75 and 90-92;
(ii) a sequence having one or several amino acid substitutions, deletions or insertions (e.g., 1, 2, 3, 4 or 5 amino acid substitutions, deletions or insertions) compared with the sequence as set forth in any one of SEQ ID NO: 67-75 and 90-92; or
(iii) a sequence having at least 80%, at least 85%, at least 90%, at least 91%, at least 92%, at least 93%, at least 94%, at least 95%, at least 96%, at least 97%, at least 98%, at least 99%, or 100% sequence identity to the sequence as set forth in any one of SEQ ID NO: 67-75 and 90-92;
preferably, the substitutions are conservative substitutions.

7. A polypeptide construct, comprising the single-domain antibody or antigen-binding fragment thereof of any one of claims 1 to 6, an Fc peptide derived from an immunoglobulin, and an optional hinge peptide;
preferably, the single-domain antibody or antigen-binding fragment thereof is covalently linked to the Fc peptide and the optional hinge peptide (e.g., forming a covalent linkage via covalent bonds including peptide bonds, isopeptide bonds and/or disulfide bonds);
preferably, the polypeptide construct comprises the single-domain antibody or antigen-binding fragment thereof of any one of claims 1 to 6, the Fc peptide and the hinge peptide, and the single-domain antibody or antigen-binding fragment thereof is covalently linked to the Fc peptide and the hinge peptide through covalent bonds including peptide bonds;
preferably, the polypeptide construct comprises a first peptide chain, wherein the first peptide chain comprises: a first single-domain antibody or an antigen-binding fragment thereof, a first hinge peptide, and a first Fc peptide, wherein the first single-domain antibody or antigen-binding fragment thereof is selected from the single-domain antibody or antigen-binding fragment thereof of any one of claims 1 to 6; preferably, the first peptide chain comprises, sequentially from the N-terminus to the C-terminus, the first single-domain antibody or antigen-binding fragment thereof, the first hinge peptide, and the first Fc peptide;
preferably, the polypeptide construct further comprises a second peptide chain, wherein the second peptide chain comprises a second hinge peptide and a second Fc peptide; preferably, the second peptide chain further comprises a second single-domain antibody or an antigen-binding fragment thereof; preferably, the second single-domain antibody is selected from single-domain antibodies specifically binding to GHR; preferably, the second single-domain antibody or antigen-binding fragment thereof is selected from the single-domain antibody or antigen-binding fragment thereof of any one of claims 1 to 6;
preferably, the second peptide chain comprises, sequentially from the N-terminus to the C-terminus: the second single-domain antibody or antigen-binding fragment thereof, the second hinge peptide, and the second Fc peptide;
preferably, the polypeptide construct has one or more features selected from the group consisting of:
(i) the first single-domain antibody or antigen-binding fragment thereof is the same as or different from the second single-domain antibody or antigen-binding fragment thereof;
(ii) the first hinge peptide and the second hinge peptide are derived from the same or different immunoglobulins;
(iii) the first Fc peptide and the second Fc peptide are derived from the same or different immunoglobulins;
(iv) the first hinge peptide and the second hinge peptide are the same or different;
(v) the first Fc peptide and the second Fc peptide are the same or different;
(vi) the first peptide chain and the second peptide chain are the same or different;
(vii) the first peptide chain and the second peptide chain are linked via disulfide bonds.

8. The polypeptide construct of claim 7, wherein the hinge peptide (e.g., the first hinge peptide and/or the second hinge peptide) is a hinge peptide derived from an immunoglobulin (e.g., IgG1, IgG2, IgG3 or IgG4);
preferably, the hinge peptide (e.g., the first hinge peptide and/or the second hinge peptide) is a hinge peptide derived from a human immunoglobulin (e.g., IgG1, IgG2, IgG3 or IgG4);
preferably, the hinge peptide (e.g., the first hinge peptide and/or the second hinge peptide) is selected from the hinge peptides of wild-type human immunoglobulins IgG1, IgG2 and IgG4, and artificially designed hinge peptides;
preferably, the artificially designed hinge peptide has a structure represented by Formula I from the N-terminus to the C-terminus:
Z-CPPCP (Formula I)
wherein Z is absent or is a short peptide consisting of 1, 2, 3, 4, 5, 6, 7, 8, 9 or 10 amino acid residues;
preferably, Z is linked to the short peptide CPPCP via a peptide bond;
preferably, Z does not comprise a cysteine residue.

9. The polypeptide construct of claim 7 or 8, wherein the hinge peptide (e.g., the first hinge peptide and/or the second hinge peptide) is selected from the hinge peptide of wild-type human immunoglobulin IgG1 and artificially designed hinge peptides;
preferably, the artificially designed hinge peptide has a structure represented by Formula II:
X₁X₂X₃X₄X₅X₆X₇X₈X₉X₁₀CPPCP (Formula II);
wherein X₁ is absent or selected from (i) amino acid residue E and (ii) an amino acid residue that is a conservative substitution relative to (i);
X₂ is absent or selected from (i) amino acid residue P and (ii) an amino acid residue that is a conservative substitution relative to (i);
X₃ is absent or selected from (i) amino acid residue K and (ii) an amino acid residue that is a conservative substitution relative to (i);
X₄ is absent or selected from (i) amino acid residue S and (ii) an amino acid residue that is a conservative substitution relative to (i);
X₅ is absent or selected from (i) amino acid residue S and (ii) an amino acid residue that is a conservative substitution relative to (i);
X₆ is absent or selected from (i) amino acid residue D and (ii) an amino acid residue that is a conservative substitution relative to (i);
X₇ is absent or selected from (i) amino acid residue K and (ii) an amino acid residue that is a conservative substitution relative to (i);
X₈ is absent or selected from (i) amino acid residue T and (ii) an amino acid residue that is a conservative substitution relative to (i);
X₉ is absent or selected from (i) amino acid residue H and (ii) an amino acid residue that is a conservative substitution relative to (i);
X₁₀ is absent or selected from (i) amino acid residue T and (ii) an amino acid residue that is a conservative substitution relative to (i);
preferably, the artificially designed hinge peptide has one or more features selected from the group consisting of: (1) X₁ is absent or is the amino acid residue E, (2) X₂ is absent or is the amino acid residue P, (3) X₃ is absent or is the amino acid residue K, (4) X₄ is absent or is the amino acid residue S, (5) X₅ is absent or is the amino acid residue S, (6) X₆ is absent or is the amino acid residue D, (7) X₇ is absent or is the amino acid residue K, (8) X₈ is absent or is the amino acid residue T, (9) X₉ is absent or is the amino acid residue H, (10) X₁₀ is absent or is the amino acid residue T;
preferably, X₁ is absent or is the amino acid residue E, X₂ is absent or is the amino acid residue P, X₃ is absent or is the amino acid residue K, X₄ is absent or is the amino acid residue S, X₅ is absent or is the amino acid residue S, X₆ is absent or is the amino acid residue D, X₇ is absent or is the amino acid residue K, X₈ is absent or is the amino acid residue T, X₉ is absent or is the amino acid residue H, and X₁₀ is absent or is the amino acid residue T;
preferably, any 1, 2, 3, 4, 5, 6, 7, 8, 9, or 10 amino acid residues of X₁ to X₁₀ are absent;
preferably, X₁ is absent, X₁ to X₂ are absent, X₁ to X₃ are absent, X₁ to X₄ are absent, X₁ to X₅ are absent, X₁ to X₆ are absent, X₁ to X₇ are absent, X₁ to X₈ are absent, X₁ to X₉ are absent, or X₁ to X₁₀ are absent;
preferably, X₁ to X₅ are absent, X₁ to X₆ are absent, X₁ to X₇ are absent, X₁ to X₈ are absent, X₁ to X₉ are absent, or X₁ to X₁₀ are absent; preferably, X₁ to X₇ are absent, or X₁ to X₈ are absent.

10. The polypeptide construct of claim 7 or 8, wherein the hinge peptide (e.g., the first hinge peptide and/or the second hinge peptide) is selected from the hinge peptide of wild-type human immunoglobulin IgG2 and artificially designed hinge peptides;
preferably, the artificially designed hinge peptide has a structure represented by Formula III:
XᵢXᵢᵢXᵢᵢᵢXᵢᵥXᵥXᵥᵢXᵥᵢᵢCPPCP (Formula III);
wherein Xᵢ is absent or selected from (i) amino acid residue E and (ii) an amino acid residue that is a conservative substitution relative to (i);
Xᵢᵢ is absent or selected from (i) amino acid residue R and (ii) an amino acid residue that is a conservative substitution relative to (i);
Xᵢᵢᵢ is absent or selected from (i) amino acid residue K and (ii) an amino acid residue that is a conservative substitution relative to (i);
Xᵢᵥ is absent or selected from non-cysteine residues; preferably, Xᵢᵥ is absent or selected from (i) amino acid residue S and (ii) an amino acid residue that is a conservative substitution relative to (i);
Xᵥ is absent or selected from non-cysteine residues; preferably, Xᵥ is absent or selected from (i) amino acid residue S and (ii) an amino acid residue that is a conservative substitution relative to (i);
Xᵥᵢ is absent or selected from (i) amino acid residue V and (ii) an amino acid residue that is a conservative substitution relative to (i);
Xᵥᵢᵢ is absent or selected from (i) amino acid residue E and (ii) an amino acid residue that is a conservative substitution relative to (i);
preferably, the artificially designed hinge peptide has one or more features selected from the group consisting of: (1) Xᵢ is absent or is the amino acid residue E, (2) Xᵢᵢ is absent or is the amino acid residue R, (3) Xᵢᵢᵢ is absent or is the amino acid residue K, (4) Xᵢᵥ is absent or is the amino acid residue S, (5) Xᵥ is absent or is the amino acid residue S, (6) Xᵥᵢ is absent or is the amino acid residue V, (7) Xᵥᵢᵢ is absent or is the amino acid residue E;
preferably, Xᵢ is absent or is the amino acid residue E, Xᵢᵢ is absent or is the amino acid residue R, Xᵢᵢᵢ is absent or is the amino acid residue K, Xᵢᵥ is absent or is the amino acid residue S, Xᵥ is absent or is the amino acid residue S, Xᵥᵢ is absent or is the amino acid residue V, and Xᵥᵢᵢ is absent or is the amino acid residue E;
preferably, any 1, 2, 3, 4, 5, 6, or 7 amino acid residues of Xᵢ to Xᵥᵢᵢ are absent; preferably, Xᵢᵥ to Xᵥᵢᵢ are absent.

11. The polypeptide construct of any one of claims 7 to 10, wherein the hinge peptide (e.g., the first hinge peptide and/or the second hinge peptide) has an amino acid sequence as set forth in any one of SEQ ID NO: 62-64 and 80-89.

12. The polypeptide construct of any one of claims 7 to 11, wherein the Fc peptide (e.g., the first Fc peptide and/or the second Fc peptide) is an Fc peptide derived from an immunoglobulin (e.g., IgG1, IgG2, IgG3 or IgG4);
preferably, the Fc peptide (e.g., the first Fc peptide and/or the second Fc peptide) is an Fc peptide derived from a human immunoglobulin (e.g., IgG1, IgG2, IgG3 or IgG4);
preferably, the Fc peptide (e.g., the first Fc peptide and/or the second Fc peptide) is an Fc peptide derived from human immunoglobulin IgG1 or IgG4.

13. The polypeptide construct of claim 12, wherein the Fc peptide (e.g., the first Fc peptide and/or the second Fc peptide) is an Fc peptide derived from human immunoglobulin IgG1;
preferably, the Fc peptide (e.g., the first Fc peptide and/or the second Fc peptide) is selected from the Fc peptide of wild-type human immunoglobulin IgG1 and variants thereof; wherein compared with the Fc peptide of wild-type human immunoglobulin IgG1, the Fc peptide variant has reduced Fc effector functions (e.g., reduced ADCC, CDC and/or ADCP activities) and/or an extended half-life;
preferably, compared with the Fc peptide of wild-type human immunoglobulin IgG1, the Fc peptide variant has reduced Fc³R binding activity, reduced serum complement molecule (C1q) binding activity, and/or enhanced FcRn binding activity;
preferably, compared with the Fc peptide of wild-type human immunoglobulin IgG1, the Fc peptide variant comprises mutations selected from L234A, L235A, M252Y, S254T, T256E, and any combination thereof (e.g., selected from mutations (i): L234A and L235A, (ii): M252Y, S254T and T256E, and (iii): a combination of (i) and (ii));
preferably, compared with the Fc peptide of wild-type human immunoglobulin IgG1, the Fc peptide variant comprises mutations L234A and L235A; preferably, the Fc peptide variant further comprises mutations M252Y, S254T, and T256E;
preferably, the Fc peptide (e.g., the first Fc peptide and/or the second Fc peptide) comprises a sequence as set forth in any one of SEQ ID NO: 52, 65, 76, 93.

14. The polypeptide construct of claim 12, wherein the Fc peptide (e.g., the first Fc peptide and/or the second Fc peptide) is an Fc peptide derived from human immunoglobulin IgG4;
preferably, the Fc peptide (e.g., the first Fc peptide and/or the second Fc peptide) is selected from the Fc peptide of wild-type human immunoglobulin IgG4 and variants thereof; wherein compared with the Fc peptide of wild-type human immunoglobulin IgG4, the Fc peptide variant has reduced Fc effector functions (e.g., reduced ADCC, CDC and/or ADCP activities) and/or an extended half-life;
preferably, compared with the Fc peptide of wild-type human immunoglobulin IgG4, the Fc peptide variant has reduced Fc³R binding activity, reduced serum complement molecule (C1q) binding activity, and/or enhanced FcRn binding activity;
preferably, compared with the Fc peptide of wild-type human immunoglobulin IgG4, the Fc peptide variant comprises mutation L235E;
preferably, the Fc peptide (e.g., the first Fc peptide and/or the second Fc peptide) comprises a sequence as set forth in SEQ ID NO: 79 or 99.

15. A polypeptide construct, comprising a single-domain antibody or an antigen-binding fragment thereof, a hinge peptide, and an Fc peptide derived from an immunoglobulin;
wherein the hinge peptide has a structure represented by Formula I from the N-terminus to the C-terminus:
Z-CPPCP (Formula I)
wherein Z is absent, or is a short peptide consisting of 1, 2, 3, 4, 5, 6, 7, 8, 9 or 10 non-cysteine amino acid residues;
preferably, Z is linked to the short peptide CPPCP via a peptide bond;
preferably, the single-domain antibody or antigen-binding fragment thereof is covalently linked to the Fc peptide and the hinge peptide (e.g., forming a covalent linkage via covalent bonds including peptide bonds, isopeptide bonds and/or disulfide bonds);
preferably, the single-domain antibody or antigen-binding fragment thereof is covalently linked to the Fc peptide and the hinge peptide via covalent bonds including peptide bonds;
preferably, the polypeptide construct comprises a first peptide chain, wherein the first peptide chain comprises: a first single-domain antibody or an antigen-binding fragment thereof, a first hinge peptide, and a first Fc peptide; preferably, the first peptide chain comprises, sequentially from the N-terminus to the C-terminus, the first single-domain antibody or antigen-binding fragment thereof, the first hinge peptide, and the first Fc peptide;
preferably, the polypeptide construct further comprises a second peptide chain, wherein the second peptide chain comprises a second hinge peptide and a second Fc peptide; preferably, the second peptide chain further comprises a second single-domain antibody or an antigen-binding fragment thereof;
preferably, the second peptide chain comprises, sequentially from the N-terminus to the C-terminus: the second single-domain antibody or antigen-binding fragment thereof, the second hinge peptide, and the second Fc peptide;
preferably, the polypeptide construct has one or more features selected from the group consisting of:
(i) the first single-domain antibody or antigen-binding fragment thereof is the same as or different from the second single-domain antibody or antigen-binding fragment thereof;
(ii) the first hinge peptide and the second hinge peptide are derived from the same or different immunoglobulins;
(iii) the first Fc peptide and the second Fc peptide are derived from the same or different immunoglobulins;
(iv) the first hinge peptide and the second hinge peptide are the same or different;
(v) the first Fc peptide and the second Fc peptide are the same or different;
(vi) the first peptide chain and the second peptide chain are the same or different;
(vii) the first peptide chain and the second peptide chain are linked via disulfide bonds.

16. The polypeptide construct of claim 15, wherein the first single-domain antibody is selected from single-domain antibodies specifically binding to a growth hormone receptor (GHR); preferably, the first single-domain antibody is selected from GHR agonistic antibodies; preferably, the first single-domain antibody or antigen-binding fragment thereof is selected from the single-domain antibody or antigen-binding fragment thereof of any one of claims 1 to 6;
preferably the second single-domain antibody is selected from single-domain antibodies specifically binding to a growth hormone receptor (GHR); preferably, the second single-domain antibody is selected from GHR agonistic antibodies; preferably, the second single-domain antibody or antigen-binding fragment thereof is selected from the single-domain antibody or antigen-binding fragment thereof of any one of claims 1 to 6.

17. The polypeptide construct of claim 15 or 16, wherein the hinge peptide (e.g., the first hinge peptide and/or the second hinge peptide) has a structure as shown in Formula II:
X₁X₂X₃X₄X₅X₆X₇X₈X₉X₁₀CPPCP (Formula II);
wherein X₁ is absent or selected from (i) amino acid residue E and (ii) an amino acid residue that is a conservative substitution relative to (i);
X₂ is absent or selected from (i) amino acid residue P and (ii) an amino acid residue that is a conservative substitution relative to (i);
X₃ is absent or selected from (i) amino acid residue K and (ii) an amino acid residue that is a conservative substitution relative to (i);
X₄ is absent or selected from (i) amino acid residue S and (ii) an amino acid residue that is a conservative substitution relative to (i);
X₅ is absent or selected from (i) amino acid residue S and (ii) an amino acid residue that is a conservative substitution relative to (i);
X₆ is absent or selected from (i) amino acid residue D and (ii) an amino acid residue that is a conservative substitution relative to (i);
X₇ is absent or selected from (i) amino acid residue K and (ii) an amino acid residue that is a conservative substitution relative to (i);
X₈ is absent or selected from (i) amino acid residue T and (ii) an amino acid residue that is a conservative substitution relative to (i);
X₉ is absent or selected from (i) amino acid residue H and (ii) an amino acid residue that is a conservative substitution relative to (i);
X₁₀ is absent or selected from (i) amino acid residue T and (ii) an amino acid residue that is a conservative substitution relative to (i);
preferably, the hinge peptide has one or more features selected from the group consisting of: (1) X₁ is absent or is the amino acid residue E, (2) X₂ is absent or is the amino acid residue P, (3) X₃ is absent or is the amino acid residue K, (4) X₄ is absent or is the amino acid residue S, (5) X₅ is absent or is the amino acid residue S, (6) X₆ is absent or is the amino acid residue D, (7) X₇ is absent or is the amino acid residue K, (8) X₈ is absent or is the amino acid residue T, (9) X₉ is absent or is the amino acid residue H, (10) X₁₀ is absent or is the amino acid residue T;
preferably, X₁ is absent or is the amino acid residue E, X₂ is absent or is the amino acid residue P, X₃ is absent or is the amino acid residue K, X₄ is absent or is the amino acid residue S, X₅ is absent or is the amino acid residue S, X₆ is absent or is the amino acid residue D, X₇ is absent or is the amino acid residue K, X₈ is absent or is the amino acid residue T, X₉ is absent or is the amino acid residue H, and X₁₀ is absent or is the amino acid residue T;
preferably, any 1, 2, 3, 4, 5, 6, 7, 8, 9, or 10 amino acid residues of X₁ to X₁₀ are absent;
preferably, X₁ is absent, X₁ to X₂ are absent, X₁ to X₃ are absent, X₁ to X₄ are absent, X₁ to X₅ are absent, X₁ to X₆ are absent, X₁ to X₇ are absent, X₁ to X₈ are absent, X₁ to X₉ are absent, or X₁ to X₁₀ are absent;
preferably, X₁ to X₅ are absent, X₁ to X₆ are absent, X₁ to X₇ are absent, X₁ to X₈ are absent, X₁ to X₉ are absent, or X₁ to X₁₀ are absent; preferably, X₁ to X₇ are absent, or X₁ to X₈ are absent.

18. The polypeptide construct of claim 15 or 16, wherein the hinge peptide (e.g., the first hinge peptide and/or the second hinge peptide) has a structure as shown in Formula III:
XᵢXᵢᵢXᵢᵢᵢXᵢᵥXᵥXᵥᵢXᵥᵢᵢCPPCP (Formula III);
wherein Xᵢ is absent or selected from (i) amino acid residue E and (ii) an amino acid residue that is a conservative substitution relative to (i);
Xᵢᵢ is absent or selected from (i) amino acid residue R and (ii) an amino acid residue that is a conservative substitution relative to (i);
Xᵢᵢᵢ is absent or selected from (i) amino acid residue K and (ii) an amino acid residue that is a conservative substitution relative to (i);
Xᵢᵥ is absent or selected from non-cysteine residues; preferably, Xᵢᵥ is absent or selected from (i) amino acid residue S and (ii) an amino acid residue that is a conservative substitution relative to (i);
Xᵥ is absent or selected from non-cysteine residues; preferably, Xᵥ is absent or selected from (i) amino acid residue S and (ii) an amino acid residue that is a conservative substitution relative to (i);
Xᵥᵢ is absent or selected from (i) amino acid residue V and (ii) an amino acid residue that is a conservative substitution relative to (i);
Xᵥᵢᵢ is absent or selected from (i) amino acid residue E and (ii) an amino acid residue that is a conservative substitution relative to (i);
preferably, the hinge peptide has one or more features selected from the group consisting of: (1) Xᵢ is absent or is the amino acid residue E, (2) Xᵢᵢ is absent or is the amino acid residue R, (3) Xᵢᵢᵢ is absent or is the amino acid residue K, (4) Xᵢᵥ is absent or is the amino acid residue S, (5) Xᵥ is absent or is the amino acid residue S, (6) Xᵥᵢ is absent or is the amino acid residue V, (7) Xᵥᵢᵢ is absent or is the amino acid residue E;
preferably, Xᵢ is absent or is the amino acid residue E, Xᵢᵢ is absent or is the amino acid residue R, Xᵢᵢᵢ is absent or is the amino acid residue K, Xᵢᵥ is absent or is the amino acid residue S, Xᵥ is absent or is the amino acid residue S, Xᵥᵢ is absent or is the amino acid residue V, and Xᵥᵢᵢ is absent or is the amino acid residue E;
preferably, any 1, 2, 3, 4, 5, 6, or 7 amino acid residues of Xᵢ to Xᵥᵢᵢ are absent; preferably, Xᵢᵥ to Xᵥᵢᵢ are absent.

19. The polypeptide construct of any one of claims 15 to 18, wherein the hinge peptide (e.g., the first hinge peptide and/or the second hinge peptide) has an amino acid sequence as set forth in any one of SEQ ID NO: 80-89.

20. The polypeptide construct of any one of claims 15 to 19, wherein the Fc peptide (e.g., the first Fc peptide and/or the second Fc peptide) is an Fc peptide derived from an immunoglobulin (e.g., IgG1, IgG2, IgG3 or IgG4);
preferably, the Fc peptide (e.g., the first Fc peptide and/or the second Fc peptide) is an Fc peptide derived from a human immunoglobulin (e.g., IgG1, IgG2, IgG3 or IgG4);
preferably, the Fc peptide (e.g., the first Fc peptide and/or the second Fc peptide) is an Fc peptide derived from human immunoglobulin IgG1 or IgG4.

21. The polypeptide construct of claim 20, wherein the Fc peptide (e.g., the first Fc peptide and/or the second Fc peptide) is an Fc peptide derived from human immunoglobulin IgG1;
preferably, the Fc peptide (e.g., the first Fc peptide and/or the second Fc peptide) is selected from the Fc peptide of wild-type human immunoglobulin IgG1 and variants thereof; wherein compared with the Fc peptide of wild-type human immunoglobulin IgG1, the Fc peptide variant has reduced Fc effector functions (e.g., reduced ADCC, CDC and/or ADCP activities) and/or an extended half-life;
preferably, compared with the Fc peptide of wild-type human immunoglobulin IgG1, the Fc peptide variant has reduced Fc³R binding activity, reduced serum complement molecule (C1q) binding activity, and/or enhanced FcRn binding activity;
preferably, compared with the Fc peptide of wild-type human immunoglobulin IgG1, the Fc peptide variant comprises mutations selected from L234A, L235A, M252Y, S254T, T256E, and any combination thereof (e.g., selected from mutations (i): L234A and L235A, (ii): M252Y, S254T and T256E, and (iii): a combination of (i) and (ii));
preferably, compared with the Fc peptide of wild-type human immunoglobulin IgG1, the Fc peptide variant comprises mutations L234A and L235A; preferably, the Fc peptide variant further comprises mutations M252Y, S254T, and T256E;
preferably, the Fc peptide (e.g., the first Fc peptide and/or the second Fc peptide) comprises a sequence as set forth in any one of SEQ ID NO: 52, 65, 76, 93.

22. The polypeptide construct of claim 20, wherein the Fc peptide (e.g., the first Fc peptide and/or the second Fc peptide) is an Fc peptide derived from human immunoglobulin IgG4;
preferably, the Fc peptide (e.g., the first Fc peptide and/or the second Fc peptide) is selected from the Fc peptide of wild-type human immunoglobulin IgG4 and variants thereof; wherein compared with the Fc peptide of wild-type human immunoglobulin IgG4, the Fc peptide variant has reduced Fc effector functions (e.g., reduced ADCC, CDC and/or ADCP activities) and/or an extended half-life;
preferably, compared with the Fc peptide of wild-type human immunoglobulin IgG4, the Fc peptide variant has reduced Fc³R binding activity, reduced serum complement molecule (C1q) binding activity, and/or enhanced FcRn binding activity;
preferably, compared with the Fc peptide of wild-type human immunoglobulin IgG4, the Fc peptide variant comprises mutation L235E;
preferably, the Fc peptide (e.g., the first Fc peptide and/or the second Fc peptide) comprises a sequence as set forth in SEQ ID NO: 79 or 99.

23. The polypeptide construct of any one of claims 7 to 22, comprising a sequence as set forth in any one of SEQ ID NO: 94-97, 103-126;
preferably, the polypeptide construct comprises the first peptide chain and optionally the second peptide chain; wherein the first peptide chain comprises a sequence as set forth in any one of SEQ ID NO: 94-97 and 103-126, and/or the second peptide chain comprises a sequence as set forth in any one of SEQ ID NO: 94-97 and 103-126;
preferably, the polypeptide construct comprises the first peptide chain and optionally the second peptide chain; wherein:
(1) the first peptide chain and the second peptide chain each independently comprise a sequence as set forth in SEQ ID NO: 94;
(2) the first peptide chain and the second peptide chain each independently comprise a sequence as set forth in SEQ ID NO: 95;
(3) the first peptide chain and the second peptide chain each independently comprise a sequence as set forth in SEQ ID NO: 96;
(4) the first peptide chain and the second peptide chain each independently comprise a sequence as set forth in SEQ ID NO: 97;
(5) the first peptide chain and the second peptide chain each independently comprise a sequence as set forth in SEQ ID NO: 103;
(6) the first peptide chain and the second peptide chain each independently comprise a sequence as set forth in SEQ ID NO: 104;
(7) the first peptide chain and the second peptide chain each independently comprise a sequence as set forth in SEQ ID NO: 105;
(8) the first peptide chain and the second peptide chain each independently comprise a sequence as set forth in SEQ ID NO: 106;
(9) the first peptide chain and the second peptide chain each independently comprise a sequence as set forth in SEQ ID NO: 107;
(10) the first peptide chain and the second peptide chain each independently comprise a sequence as set forth in SEQ ID NO: 108;
(11) the first peptide chain and the second peptide chain each independently comprise a sequence as set forth in SEQ ID NO: 109;
(12) the first peptide chain and the second peptide chain each independently comprise a sequence as set forth in SEQ ID NO: 110;
(13) the first peptide chain and the second peptide chain each independently comprise a sequence as set forth in SEQ ID NO: 111;
(14) the first peptide chain and the second peptide chain each independently comprise a sequence as set forth in SEQ ID NO: 112;
(15) the first peptide chain and the second peptide chain each independently comprise a sequence as set forth in SEQ ID NO: 113;
(16) the first peptide chain and the second peptide chain each independently comprise a sequence as set forth in SEQ ID NO: 114;
(17) the first peptide chain and the second peptide chain each independently comprise a sequence as set forth in SEQ ID NO: 115;
(18) the first peptide chain and the second peptide chain each independently comprise a sequence as set forth in SEQ ID NO: 116;
(19) the first peptide chain and the second peptide chain each independently comprise a sequence as set forth in SEQ ID NO: 117;
(20) the first peptide chain and the second peptide chain each independently comprise a sequence as set forth in SEQ ID NO: 118;
(21) the first peptide chain and the second peptide chain each independently comprise a sequence as set forth in SEQ ID NO: 119;
(22) the first peptide chain and the second peptide chain each independently comprise a sequence as set forth in SEQ ID NO: 120;
(23) the first peptide chain and the second peptide chain each independently comprise a sequence as set forth in SEQ ID NO: 121;
(24) the first peptide chain and the second peptide chain each independently comprise a sequence as set forth in SEQ ID NO: 122;
(25) the first peptide chain and the second peptide chain each independently comprise a sequence as set forth in SEQ ID NO: 123;
(26) the first peptide chain and the second peptide chain each independently comprise a sequence as set forth in SEQ ID NO: 124;
(27) the first peptide chain and the second peptide chain each independently comprise a sequence as set forth in SEQ ID NO: 125;
(28) the first peptide chain and the second peptide chain each independently comprise a sequence as set forth in SEQ ID NO: 126.

24. An isolated nucleic acid molecule, encoding the single-domain antibody or antigen-binding fragment thereof of any one of claims 1 to 6 or the polypeptide construct of any one of claims 7 to 23.

25. A vector, comprising the isolated nucleic acid molecule of claim 24, wherein preferably, the vector is a cloning vector or an expression vector.

26. A host cell, comprising the isolated nucleic acid molecule of claim 24 or the vector of claim 25.

27. A method for preparing the single-domain antibody or antigen-binding fragment thereof of any one of claims 1 to 6 or the polypeptide construct of any one of claims 7 to 23, comprising culturing the host cell of claim 26 under conditions permitting expression of the single-domain antibody or antigen-binding fragment thereof or the polypeptide construct, and recovering the single-domain antibody or antigen-binding fragment thereof or the polypeptide construct from the cultured host cell culture.

28. A bispecific or multispecific molecule, comprising the single-domain antibody or antigen-binding fragment thereof of any one of claims 1 to 6 or the polypeptide construct of any one of claims 7 to 23;
preferably, the bispecific or multispecific molecule specifically binds to GHR, and additionally specifically binds to one or more other targets;
preferably, the bispecific or multispecific molecule further comprises at least one molecule with a second binding specificity for a second target (e.g., a second antibody or an antigen-binding fragment thereof).

29. An immunoconjugate, comprising the single-domain antibody or antigen-binding fragment thereof of any one of claims 1 to 6 or the polypeptide construct of any one of claims 7 to 23, and a therapeutic agent linked to the single-domain antibody or antigen-binding fragment thereof or the polypeptide construct;
preferably, the immunoconjugate is an antibody-drug conjugate (ADC).

30. A pharmaceutical composition, comprising the single-domain antibody or antigen-binding fragment thereof of any one of claims 1 to 6, the polypeptide construct of any one of claims 7 to 23, the isolated nucleic acid molecule of claim 24, the vector of claim 25, the host cell of claim 26, the bispecific or multispecific molecule of claim 28 or the immunoconjugate of claim 29, and a pharmaceutically acceptable carrier and/or excipient;
preferably, the pharmaceutical composition further comprises an additional pharmaceutically active agent.

31. Use of the single-domain antibody or antigen-binding fragment thereof of any one of claims 1 to 6, the polypeptide construct of any one of claims 7 to 23, the isolated nucleic acid molecule of claim 24, the vector of claim 25, the host cell of claim 26, the bispecific or multispecific molecule of claim 28, the immunoconjugate of claim 29 or the pharmaceutical composition of claim 30 in the manufacture of a drug for preventing and/or treating a disease associated with GHR signaling in a subject;
preferably, the prevention and/or treatment of the disease associated with GHR signaling benefit from agonism of GHR signaling;
preferably, the disease associated with GHR signaling is selected from diseases associated with deficiency of GH (growth hormone), IGF-1 (insulin-like growth factor-1) and/or GHRH (growth hormone-releasing hormone);
preferably, the disease associated with GHR signaling is selected from: growth hormone deficiency (GHD), chronic kidney disease (CKD), Turner syndrome, Prader-Willi syndrome, small for gestational age (SGA), idiopathic short stature (ISS), SHOX gene deficiency, Noonan syndrome, childhood short stature, severe burns, adult growth hormone deficiency, adult short bowel syndrome, HIV wasting syndrome, aging, and reproductive disorders;
preferably, the growth hormone deficiency (GHD) is that caused by hypothalamic-pituitary diseases; the childhood short stature is that resulting from achondroplasia (ACH); and/or the reproductive disorder is a reproductive disorder associated with the growth hormone-growth hormone receptor signaling pathway (e.g., gonadal dysgenesis caused by growth disorders due to Turner syndrome);
preferably, the subject is a mammal, such as a human or murine;
preferably, the single-domain antibody or antigen-binding fragment thereof, polypeptide construct, isolated nucleic acid molecule, vector, host cell or pharmaceutical composition is used alone or administered in combination with an additional pharmaceutically active agent (e.g., administered simultaneously or sequentially).

32. A method for preventing and/or treating a disease associated with GHR signaling in a subject, comprising: administering to a subject in need thereof an effective amount of the single-domain antibody or antigen-binding fragment thereof of any one of claims 1 to 6, the polypeptide construct of any one of claims 7 to 23, the isolated nucleic acid molecule of claim 24, the vector of claim 25, the host cell of claim 26, the bispecific or multispecific molecule of claim 28, the immunoconjugate of claim 29 or the pharmaceutical composition of claim 30;
preferably, the prevention and/or treatment of the disease associated with GHR signaling benefit from agonism of GHR signaling;
preferably, the disease associated with GHR signaling is selected from diseases associated with deficiency of GH (growth hormone), IGF-1 (insulin-like growth factor-1) and/or GHRH (growth hormone-releasing hormone);
preferably, the disease associated with GHR signaling is selected from: growth hormone deficiency (GHD), chronic kidney disease (CKD), Turner syndrome, Prader-Willi syndrome, small for gestational age (SGA), idiopathic short stature (ISS), SHOX gene deficiency, Noonan syndrome, childhood short stature, severe burns, adult growth hormone deficiency, adult short bowel syndrome, HIV wasting syndrome, aging, and reproductive disorders;
preferably, the growth hormone deficiency (GHD) is that caused by hypothalamic-pituitary diseases; the childhood short stature is that resulting from achondroplasia (ACH); and/or the reproductive disorder is a reproductive disorder associated with the growth hormone-growth hormone receptor signaling pathway (e.g., gonadal dysgenesis caused by growth disorders due to Turner syndrome);
preferably, the subject is a mammal, such as a human or murine;
preferably, the single-domain antibody or antigen-binding fragment thereof, polypeptide construct, isolated nucleic acid molecule, vector, host cell or pharmaceutical composition is used alone or administered in combination with an additional pharmaceutically active agent (e.g., administered simultaneously or sequentially).

33. A conjugate, comprising the single-domain antibody or antigen-binding fragment thereof of any one of claims 1 to 6 or the polypeptide construct of any one of claims 7 to 23, and a detectable label linked to the single-domain antibody or antigen-binding fragment thereof or the polypeptide construct;
preferably, the detectable label is selected from an enzyme (e.g., horseradish peroxidase or alkaline phosphatase), a chemiluminescent reagent (e.g., acridinium ester compounds, luminol and derivatives thereof, or ruthenium derivatives), a fluorescent dye (e.g., fluorescein or fluorescent protein), a radionuclide, or a biotin.

34. A kit, comprising the single-domain antibody or antigen-binding fragment thereof of any one of claims 1 to 6, the polypeptide construct of any one of claims 7 to 23, or the conjugate of claim 33;
preferably, the kit comprises the conjugate of claim 33;
preferably, the kit comprises the single-domain antibody or antigen-binding fragment thereof of any one of claims 1 to 6 or the polypeptide construct of any one of claims 7 to 23, and a second antibody that specifically recognizes the single-domain antibody or antigen-binding fragment thereof or the polypeptide construct. Optionally, the second antibody further comprises a detectable label, such as an enzyme (e.g., horseradish peroxidase or alkaline phosphatase), a chemiluminescent reagent (e.g., acridinium ester compounds, luminol and derivatives thereof, or ruthenium derivatives), a fluorescent dye (e.g., fluorescein or fluorescent protein), a radionuclide, or a biotin.

35. A method for detecting the presence or level of GHR in a sample, comprising using the single-domain antibody or antigen-binding fragment thereof of any one of claims 1 to 6, the polypeptide construct of any one of claims 7 to 23, or the conjugate of claim 33;
preferably, the method is an immunoassay, such as Western blotting, enzyme immunoassay (e.g., ELISA), chemiluminescent immunoassay, fluorescent immunoassay, or radioimmunoassay;
preferably, the method comprises using the conjugate of claim 33;
preferably, the method comprises using the single-domain antibody or antigen-binding fragment thereof of any one of claims 1 to 6 or the polypeptide construct of any one of claims 7 to 23, and the method further comprises using a second antibody carrying a detectable label (e.g., an enzyme (e.g., horseradish peroxidase or alkaline phosphatase), a chemiluminescent reagent (e.g., acridinium ester compounds, luminol and derivatives thereof, or ruthenium derivatives), a fluorescent dye (e.g., fluorescein or fluorescent protein), a radionuclide, or a biotin) to detect the single-domain antibody or antigen-binding fragment thereof or the polypeptide construct;
preferably, the method comprises: (1) contacting the sample with the single-domain antibody or antigen-binding fragment thereof, the polypeptide construct, or the conjugate; and (2) detecting the formation of an antigen-antibody immune complex, or detecting the amount of the immune complex.

36. Use of the single-domain antibody or antigen-binding fragment thereof of any one of claims 1 to 6, the polypeptide construct of any one of claims 7 to 23, or the conjugate of claim 33 in the manufacture of a detection reagent, wherein the detection reagent is used for detecting the presence or level of GHR in a sample and/or diagnosing a GHR-related disease.
